# EUROPEAN PATENT SPECIFICATION

(11) **EP 4 081 513 B1**
(45) Date of publication and mention of the grant of the patent: **20.11.2024**
(21) Application number: 20845303.5
(22) Date of filing: 22.12.2020
(51) Int. Cl.: C07D 401/04, A61K 31/4709, A61P 35/00

(54) **SUBSTITUTED QUINOLINONYL PIPERAZINE COMPOUNDS USEFUL AS T CELL ACTIVATORS**
SUBSTITUIERTE QUINOLINONYL-PIPERAZINDERIVATE ALS T-ZELLAKTIVATOREN
DERIVES DE QUINOLINONYL-PIPERAZINE COMME DES ACTIVATEURS DES T-CELLULES

(30) Priority: 23.12.2019 IN 201911053557
(43) Date of publication of application: 02.11.2022
(73) Proprietor: Bristol-Myers Squibb Company, Princeton, NJ 08543 (US)
(72) Inventor: CHUPAK, Louis, S., Cambridge, Massachusetts 02142 (US); OLSON, Richard, E., Cambridge, MA 02139 (US); VELAPARTHI, Upender, Princeton, New Jersey 08543 (US); ZHENG, Xiaofan, Cambridge, Massachusetts 02142 (US); GENTLES, Robert, G., Cambridge, Massachusetts 02142 (US); DING, Min, Cambridge, Massachusetts 02142 (US); WARRIER, Jayakumar, Sankara, Bangalore, Karnataka 560 099 (IN)
(74) Representative: Dehns
(86) International application number: PCT/US2020/066503
(87) International publication number: WO 2021/133748

(56) References cited:
- WO-A1-2020/006018
- WO-A2-2004/074218
- TOMINAGA M ET AL: "Studies on positive inotropic agents. I. Synthesis of 3,4-dihydro-6-[4-(3,4-dimethoxybenzoyl)-1-piperazinyl]-2(1H)- quinolinone and related compounds", CHEM PHARM BULL (TOKYO),, vol. 32, no. 6, 1 June 1984 (1984-06-01), pages 2100 - 2110, XP009525856, DOI: 10.1248/CPB.32.2100

## Description

The present invention generally relates to substituted quinolinonyl compounds that activate T cells, promote T cell proliferation, and/or exhibit antitumor activity. Provided herein are substituted quinolinonyl compounds, compositions comprising such compounds, and methods of their use. The invention further pertains to pharmaceutical compositions comprising at least one compound according to the invention that are useful for the treatment of proliferative disorders, such as cancer, and viral infections.

### BACKGROUND OF THE INVENTION

Human cancers harbor numerous genetic and epigenetic alterations, generating neoantigens potentially recognizable by the immune system (Sjoblom et al. (2006) Science 314:268-74). The adaptive immune system, comprised of T and B lymphocytes, has powerful anti-cancer potential, with a broad capacity and exquisite specificity to respond to diverse tumor antigens. Further, the immune system demonstrates considerable plasticity and a memory component. The successful harnessing of all these attributes of the adaptive immune system would make immunotherapy unique among all cancer treatment modalities. However, although an endogenous immune response to cancer is observed in preclinical models and patients, this response is ineffective, and established cancers are viewed as "self' and tolerated by the immune system. Contributing to this state of tolerance, tumors may exploit several distinct mechanisms to actively subvert anti-tumor immunity. These mechanisms include dysfunctional T-cell signaling (Mizoguchi et al., (1992) Science 258:1795-98), suppressive regulatory cells (Facciabene et al., (2012) Cancer Res. 72:2162-71), and the co-opting of endogenous "immune checkpoints", which serve to down-modulate the intensity of adaptive immune responses and protect normal tissues from collateral damage, by tumors to evade immune destruction (Topalian et al., (2012) Curr. Opin. Immunol. 24:1-6; Mellman et al. (2011) Nature 480:480-489).

Diacylglycerol kinases (DGKs) are lipid kinases that mediate the conversion of diacylglycerol to phosphatidic acid thereby terminating T cell functions propagated through the TCR signaling pathway. Thus, DGKs serve as intracellular checkpoints and inhibition of DGKs are expected to enhance T cell signaling pathways and T cell activation. Supporting evidence include knock-out mouse models of either DGKα or DGKζ which show a hyper-responsive T cell phenotype and improved anti-tumor immune activity (Riese M.J. et al., Journal of Biological Chemistry, (2011) 7: 5254-5265; Zha Y et al., Nature Immunology, (2006) 12:1343; Olenchock B.A. et al., (2006) 11: 1174-81). Furthermore tumor infiltrating lymphocytes isolated from human renal cell carcinoma patients were observed to overexpress DGKα which resulted in inhibited T cell function (Prinz, P.U. et al., J Immunology (2012) 12:5990-6000). Thus, DGKα and DGKζ are viewed as targets for cancer immunotherapy (Riese M.J. et al., Front Cell Dev Biol. (2016) 4: 108; Chen, S.S. et al., Front Cell Dev Biol. (2016) 4: 130; Avila-Flores, A. et al., Immunology and Cell Biology (2017) 95: 549-563; Noessner, E., Front Cell Dev Biol. (2017) 5: 16; Krishna, S., et al., Front Immunology (2013) 4:178; Jing, W. et al., Cancer Research (2017) 77: 5676-5686.

There remains a need for compounds useful as inhibitors of one or both of DGKα and DGKζ. Additionally, there remains a need for compounds useful as inhibitors of one or both of DGKα and DGKζ that have selectivity over other diacylglycerol kinases, protein kinases, and/or other lipid kinases.

Accordingly, an agent that is safe and effective in restoring T cell activation, lowering antigen threshold, enhancing antitumor functionality, and/or overcoming the suppressive effects of one or more endogenous immune checkpoints, such as PD-1, LAG-3 and TGFβ, would be an important addition for the treatment of patients with proliferative disorders, such as cancer, as well as viral infections.

### SUMMARY OF THE INVENTION

Applicants have found compounds that have activity as inhibitors of one or both of DGKα and DGKζ. Further, applicants have found compounds that have activity as inhibitors of one or both of DGKα and DGKζ and have selectivity over other diacylglycerol kinases, protein kinases, and/or other lipid kinases. These compounds are provided to be useful as pharmaceuticals with desirable stability, bioavailability, therapeutic index, and toxicity values that are important to their drugability.

The present invention provides substituted quinolinonyl compounds of Formula (I), which are useful as inhibitors of DGKα, DGKζ, or both DGKα and DGKζ, including salts thereof.

The present invention also provides pharmaceutical compositions comprising a compound of Formula (I) and/or a pharmaceutically acceptable salt thereof; and a pharmaceutically acceptable carrier.

The present invention also provides compounds for use in a method of treating a disease or disorder associated with the activity of DGKα, DGKζ, or both DGKα and DGKζ, the method comprising administering to a mammalian patient a compound of Formula (I) and/or a pharmaceutically acceptable salt thereof.

Also described (but not claimed) are processes and intermediates for making the compounds of Formula (I) and/or salts thereof.

The present invention also provides a compound of Formula (I) and/or a pharmaceutically acceptable salt thereof, for use in therapy.

The present invention also provides the use of the compounds of Formula (I) and/or pharmaceutically acceptable salts thereof, for the manufacture of a medicament for the treatment of proliferative disorders, such as cancer and viral infections.

The compounds of Formula (I) and compositions comprising the compounds of Formula (I) may be used in treating, preventing, or curing viral infections and various proliferative disorders, such as cancer. Pharmaceutical compositions comprising these compounds are useful in treating, preventing, or slowing the progression of diseases or disorders in a variety of therapeutic areas, such as viral infections and cancer.

These and other features of the invention will be set forth in expanded form as the disclosure continues.

Any references in the description to methods of treatment refer to the compounds, pharmaceutical compositions and medicaments of the invention for use in a method for treatment of the human (or animal) body by therapy (or for diagnosis).

### DETAILED DESCRIPTION

The first aspect of the present invention provides at least one compound of Formula (I): 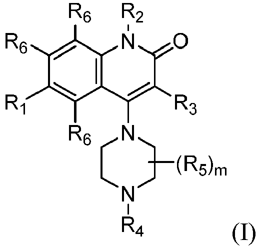 or a salt thereof, wherein:
R₁ is F, Cl, Br, -CN, C₁₋₃ alkyl substituted with zero to 4 R₁ₐ, C₃₋₄ cycloalkyl substituted with zero to 4 R₁ₐ, C₁₋₃ alkoxy substituted with zero to 4 R₁ₐ, -C(O)NRₐRₐ, -NRₐRₐ, -S(O)ₙRₑ, or -P(O)RₑRₑ;
each R₁ₐ is independently F, Cl, -CN, -OH, -OCH₃, or -NRₐRₐ;
each Rₐ is independently H or C₁₋₃ alkyl;
each Rₑ is independently C₃₋₄ cycloalkyl or C₁₋₃ alkyl substituted with zero to 4 R₁ₐ;
R₂ is H, C₁₋₃ alkyl substituted with zero to 4 R₂ₐ, C₂₋₃ alkenyl substituted with zero to 4 R₂ₐ, or C₃₋₄ cycloalkyl substituted with zero to 4 R₂ₐ;
each R₂ₐ is independently F, Cl, -CN, -OH, -O(C₁₋₂ alkyl), C₃₋₄ cycloalkyl, C₃₋₄ alkenyl, or C₃₋₄ alkynyl;
R₃ is H, F, Cl, Br, -CN, C₁₋₃ alkyl, C₁₋₂ fluoroalkyl, C₃₋₄ cycloalkyl, C₃₋₄ fluorocycloalkyl, -NO₂, or pyridinyl substituted with zero to 2 R₃ₐ;
each R₃ₐ is halo, -CN, C₁₋₃ alkyl, or C₁₋₃ alkoxy;
R₄ is -CH₂R₄ₐ, -CH₂CH₂R₄ₐ, -CH₂CHR₄ₐR_{4d}, -CHR₄ₐR_{4b}, or -CR₄ₐR_{4b}R_{4c};
R₄ₐ and R_{4b} are independently:
   (i) C₁₋₆ alkyl substituted with zero to 4 substituents independently selected from F, Cl, -CN, -OH, -OCH₃, -SCH₃, C₁₋₃ fluoroalkoxy, -NRₐRₐ, -S(O)₂Rₑ, or -NRₐS(O)₂Rₑ;
   (ii) C₃₋₆ cycloalkyl, heterocyclyl, phenyl, or heteroaryl, each substituted with zero to 4 substituents independently selected from F, Cl, Br, -CN, -OH, C₁₋₆ alkyl, C₁₋₃ fluoroalkyl, C₁₋₄ hydroxyalkyl, -(CH₂)₁₋₂O(C₁₋₃ alkyl), C₁₋₄ alkoxy, -O(C₁₋₄ hydroxyalkyl), -O(CH)₁₋₃O(C₁₋₃ alkyl), C₁₋₃ fluoroalkoxy, -O(CH)₁₋₃NR_{c}R_{c}, -OCH₂CH=CH₂, OCH₂C≡CH, -C(O)(C₁₋₄ alkyl), -C(O)OH, -C(O)O(C₁₋₄ alkyl), -NR_{c}R_{c}, -NRₐS(O)₂(C₁₋₃ alkyl), -NRₐC(O)(C₁₋₃ alkyl), -NRₐC(O)O(C₁₋₄ alkyl), -P(O)(C₁₋₃ alkyl)₂, -S(O)₂(C₁₋₃ alkyl), -O(CH₂)₁₋₂(C₃₋₆ cycloalkyl), -O(CH₂)₁₋₂(morpholinyl), cyclopropyl, cyanocyclopropyl, methylazetidinyl, acetylazetidinyl, (tert-butoxycarbonyl)azetidinyl, triazolyl, tetrahydropyranyl, morpholinyl, thiophenyl, methylpiperidinyl, and R_{d}; or
   (iii) C₁₋₄ alkyl substituted with one cyclic group selected from C₃₋₆ cycloalkyl, heterocyclyl, aryl, and heteroaryl, said cyclic group substituted with zero to 3 substituents independently selected from F, Cl, Br, -OH, -CN, C₁₋₆ alkyl, C₁₋₃ fluoroalkyl, C₁₋₃ alkoxy, C₁₋₃ fluoroalkoxy, -OCH₂CH=CH₂, -OCHzC=CH, -NR_{c}R_{c}, -NRₐS(O)₂(C₁₋₃ alkyl), -NRₐC(O)(C₁₋₃ alkyl), -NRₐC(O)O(C₁₋₄ alkyl), and C₃₋₆ cycloalkyl;
or R₄ₐ and R_{4b} together with the carbon atom to which they are attached form a C₃₋₆ cycloalkyl or a 3- to 6-membered heterocyclyl, each substituted with zero to 3 R_{f};
each R_{f} is independently F, Cl, Br, -OH, -CN, C₁₋₆ alkyl, C₁₋₃ fluoroalkyl, C₁₋₃ alkoxy, C₁₋₃ fluoroalkoxy, -OCH₂CH=CH₂, -OCHzC=CH, -NR_{c}R_{c}, or a cyclic group selected from C₃₋₆ cycloalkyl, 3- to 6-membered heterocyclyl, phenyl, monocyclic heteroaryl, and bicyclic heteroaryl, each cyclic group substituted with zero to 3 substituents independently selected from F, Cl, Br, -OH, -CN, C₁₋₆ alkyl, C₁₋₃ fluoroalkyl, C₁₋₃ alkoxy, C₁₋₃ fluoroalkoxy, and -NR_{c}R_{c};
R_{4c} is C₁₋₆ alkyl or C₃₋₆ cycloalkyl, each substituted with zero to 4 substituents independently selected from F, Cl, -OH, C₁₋₂ alkoxy, C₁₋₂ fluoroalkoxy, and -CN;
R_{4d} is -OCH₃;
each R_{c} is independently H or C₁₋₂ alkyl;
R_{d} is phenyl substituted with zero to 1 substituent selected from F, Cl, -CN, -CH₃, and -OCH₃;
each R₅ is independently F, Cl, -CN, -OH, C₁₋₆ alkyl substituted with zero to 4 R_{g}, C₁₋₃ alkoxy substituted with zero to 4 R_{g}, C₂₋₄ alkenyl substituted with zero to 4 R_{g}, C₂₋₄ alkynyl substituted with zero to 4 R_{g}, C₃₋₄ cycloalkyl substituted with zero to 4 R_{g}, phenyl substituted with zero to 4 R_{g}, oxadiazolyl substituted with zero to 3 R_{g}, pyridinyl substituted with zero to 4 R_{g}, -(CH₂)₁₋₂(heterocyclyl substituted with zero to 4 R_{g}), -(CH₂)₁₋₂NR_{c}C(O)(C₁₋₄ alkyl), -(CH₂)₁₋₂NR_{c}C(O)O(C₁₋₄ alkyl), -(CH₂)₁₋₂NR_{c}S(O)₂(C₁₋₄ alkyl), -C(O)(C₁₋₄ alkyl), -C(O)OH, -C(O)O(C₁₋₄ alkyl), -C(O)O(C₃₋₄ cycloalkyl), -C(O)NRₐRₐ, or -C(O)NRₐ(C₃₋₄ cycloalkyl), or two R₅ attached to the same carbon atom form =O;
each R_{g} is independently F, Cl, -CN, -OH, C₁₋₃ alkoxy, C₁₋₃ fluoroalkoxy, -O(CH₂)₁₋₂O(C₁₋₂ alkyl), C₃₋₅ cycloalkyl, or -NR_{c}R_{c};
each R₆ is H, F, Cl, -CN, -CH₃, -CH₂F, -CHF₂, -CF₃, or -OCH₃;
m is zero, 1, 2, or 3; and
n is zero, 1, or 2.

In one embodiment, a compound of Formula (I) or a salt thereof is provided wherein: R₁ is F, Cl, Br, -CN, C₁₋₃ alkyl substituted with zero to 4 R₁ₐ, cyclopropyl substituted with zero to 3 R₁ₐ, C₁₋₃ alkoxy substituted with zero to 3 R₁ₐ, -C(O)NRₐRₐ, -NRₐRₐ, -S(O)ₙCH₃, or -P(O)(CH₃)₂; each R₁ₐ is independently F, Cl, or -CN; each Rₐ is independently H or C₁₋₃ alkyl; R₂ is H, C₁₋₂ alkyl substituted with zero to 2 R₂ₐ, or C₂₋₃ alkenyl substituted with zero to 2 R₂ₐ; each R₂ₐ is independently F, Cl, -CN, -OH, -O(C₁₋₂ alkyl), cyclopropyl, C₃₋₄ alkenyl, or C₃₋₄ alkynyl; R₃ is H, F, Cl, Br, -CN, C₁₋₂ alkyl, C₁₋₂ fluoroalkyl, C₃₋₄ cycloalkyl, -NOz, or pyridinyl substituted with zero to 2 R₃ₐ; R₄ₐ and R_{4b} are independently: (i) C₁₋₄ alkyl substituted with zero to 4 substituents independently selected from F, Cl, -CN, -OH, -OCH₃, -SCH₃, C₁₋₃ fluoroalkoxy, and -NRₐRₐ; (ii) C₃₋₆ cycloalkyl, heterocyclyl, phenyl, or heteroaryl, each substituted with zero to 4 substituents independently selected from F, Cl, Br, -CN, -OH, C₁₋₆ alkyl, C₁₋₃ fluoroalkyl, -CH₂OH, -(CH₂)₁₋₂O(C₁₋₂ alkyl), C₁₋₄ alkoxy, -O(C₁₋₄ hydroxyalkyl), -O(CH)₁₋₂O(C₁₋₂ alkyl), C₁₋₃ fluoroalkoxy, -O(CH)₁₋₂NR_{c}R_{c}, -OCH₂CH=CH₂, -OCH₂C≡CH, -C(O)(C₁₋₄ alkyl), -C(O)OH, -C(O)O(C₁₋₄ alkyl), -NR_{c}R_{c}, -NRₐS(O)₂(C₁₋₃ alkyl), -NRₐC(O)(C₁₋₃ alkyl), -NRₐC(O)O(C₁₋₄ alkyl), -P(O)(C₁₋₂ alkyl)₂, -S(O)₂(C₁₋₃ alkyl), -O(CH₂)₁₋₂(C₃₋₄ cycloalkyl), -O(CH₂)₁₋₂(morpholinyl), cyclopropyl, cyanocyclopropyl, methylazetidinyl, acetylazetidinyl, (*tert-*butoxycarbonyl)azetidinyl, triazolyl, tetrahydropyranyl, morpholinyl, thiophenyl, methylpiperidinyl, and R_{d}; or (iii) C₁₋₃ alkyl substituted with one cyclic group selected from C₃₋₆ cycloalkyl, heterocyclyl, phenyl, and heteroaryl, said cyclic group substituted with zero to 3 substituents independently selected from F, Cl, Br, -OH, -CN, C₁₋₃ alkyl, C₁₋₂ fluoroalkyl, C₁₋₃ alkoxy, C₁₋₂ fluoroalkoxy, -OCH₂CH=CH₂, -OCHzC=CH, -NR_{c}R_{c}, -NRₐS(O)₂(C₁₋₃ alkyl), -NRₐC(O)(C₁₋₃ alkyl), -NRₐC(O)O(C₁₋₄ alkyl), and C₃₋₄ cycloalkyl; or R₄ₐ and R_{4b} together with the carbon atom to which they are attached, form a C₃₋₆ cycloalkyl or a 3- to 6-membered heterocyclyl, each substituted with zero to 3 R_{f}; each R_{f} is independently F, Cl, Br, -OH, -CN, C₁₋₄ alkyl, C₁₋₂ fluoroalkyl, C₁₋₃ alkoxy, C₁₋₂ fluoroalkoxy, -OCH₂CH=CH₂, -OCHzC=CH, -NR_{c}R_{c}, or a cyclic group selected from C₃₋₆ cycloalkyl, 3- to 6-membered heterocyclyl, phenyl, monocyclic heteroaryl, and bicyclic heteroaryl, each cyclic group substituted with zero to 3 substituents independently selected from F, Cl, Br, -OH, -CN, C₁₋₄ alkyl, C₁₋₂ fluoroalkyl, C₁₋₃ alkoxy, C₁₋₂ fluoroalkoxy, and -NR_{c}R_{c}; R_{4c} is C₁₋₄ alkyl or C₃₋₆ cycloalkyl, each substituted with zero to 4 substituents independently selected from F, Cl, -OH, C₁₋₂ alkoxy, C₁₋₂ fluoroalkoxy, and -CN; each R₅ is independently F, -CN, -OH, C₁₋₅ alkyl substituted with zero to 4 R_{g}, C₁₋₂ alkoxy substituted with zero to 3 R_{g}, C₂₋₃ alkenyl substituted with zero to 4 R_{g}, C₂₋₃ alkynyl substituted with zero to 4 R_{g}, C₃₋₄ cycloalkyl substituted with zero to 4 R_{g}, phenyl substituted with zero to 3 R_{g}, oxadiazolyl substituted with zero to 3 R_{g}, pyridinyl substituted with zero to 3 R_{g}, -(CH₂)₁₋₂(heterocyclyl substituted with zero to 4 R_{g}), -(CH₂)₁₋₂NR_{c}C(O)(C₁₋₄ alkyl), -(CH₂)₁₋₂NR_{c}C(O)O(C₁₋₄ alkyl), -(CH₂)₁₋₂NR_{c}S(O)₂(C₁₋₄ alkyl), -C(O)(C₁₋₄ alkyl), -C(O)OH, -C(O)O(C₁₋₄ alkyl), -C(O)O(C₃₋₄ cycloalkyl), -C(O)NRₐRₐ, or -C(O)NRₐ(C₃₋₄ cycloalkyl), or two R₅ attached to the same carbon atom form =O; each R₆ is H, F, or -CH₃; and m is zero, 1, 2, or 3.

In one embodiment, a compound of Formula (I) or a salt thereof is provided wherein: R₁ is F, -CN, or -OCH₃; R₂ is H, -CH₃, -CH₂CN, -CH₂CH₂F, or -CH₂CH=CH₂; R₃ is H, -CN, -CHzOH, -C(O)OCH₂CH₃, -NOz, or pyridinyl; R₄ is -CH₂R₄ₐ or -CHR₄ₐR_{4b}; R₄ₐ is phenyl, naphthalenyl, or indolyl, each substituted with zero to 2 substituents independently selected from F, -CH₃, -CH₂CH₃, and -OCH₃; R_{4b} is phenyl or fluorophenyl; each R₅ is -CH₃, or two R₅ attached to the same carbon atom form =O; and m is zero, 1, or 2.

In one embodiment, a compound of Formula (I) or a salt thereof is provided wherein R₁ is F, Cl, Br, -CN, C₁₋₃ alkyl substituted with zero to 4 R₁ₐ, cyclopropyl substituted with zero to 3 R₁ₐ, C₁₋₃ alkoxy substituted with zero to 3 R₁ₐ, -C(O)NRₐRₐ, -NRₐRₐ, -S(O)ₙCH₃, or -P(O)(CH₃)₂. Included in this embodiment are compounds in which R₁ is F, Cl, Br, -CN, -CH₃, -CHF₂, -CF₃, cyclopropyl, or -OCH₃. Also included in this embodiment are compounds in which R₁ is F, -CN, or -OCH₃.

In one embodiment, a compound of Formula (I) or a salt thereof is provided wherein R₂ is H, C₁₋₂ alkyl substituted with zero to 2 R₂ₐ, or C₂₋₃ alkenyl substituted with zero to 2 R₂ₐ. Included in this embodiment are compounds in which R₂ is H, -CH₃, -CH₂CN, -CH₂CH₂F, or -CH₂CH=CH₂. Also included in this embodiment are compounds in which R₂ is H, -CH₃, -CH₂CN, or -CH₂CH₂F.

In one embodiment, a compound of Formula (I) or a salt thereof is provided wherein R₃ is H, F, Cl, Br, -CN, C₁₋₂ alkyl, C₁₋₂ fluoroalkyl, C₃₋₄ cycloalkyl, -NO₂, or pyridinyl substituted with zero to 2 R₃ₐ. Included in this embodiment are compounds in which R₃ is H, -CN, -CH₂OH, -C(O)OCH₂CH₃, -NO₂, or pyridinyl. Also included in this embodiment are compounds in which R₃ is H, -CN, -CH₂OH, or -NO₂.

In one embodiment, a compound of Formula (I) or a salt thereof is provided wherein R₄ is -CH₂R₄ₐ or -CH₂CH₂R₄ₐ. Included in this embodiment are compounds in which R₄ is -CH₂R₄ₐ or -CD₂R₄ₐ.

In one embodiment, a compound of Formula (I) or a salt thereof is provided wherein R₄ is -CH₂R₄ₐ. Included in this embodiment are compounds in which R₄ is -CD₂R₄ₐ.

In one embodiment, a compound of Formula (I) or a salt thereof is provided wherein R₄ is -CHR₄ₐR_{4b} or -CR₄ₐR_{4b}R_{4c}.

In one embodiment, a compound of Formula (I) or a salt thereof is provided wherein R₄ is -CHR₄ₐR_{4b}.

In one embodiment, a compound of Formula (I) or a salt thereof is provided wherein R₄ is -CH₂R₄ₐ or -CHR₄ₐR_{4b}.

In one embodiment, a compound of Formula (I) or a salt thereof is provided wherein m is 1, 2, or 3; and each R₅ is independently F, -CN, -OH, C₁₋₅ alkyl substituted with zero to 4 R_{g}, C₁₋₂ alkoxy substituted with zero to 3 R_{g}, C₂₋₃ alkenyl substituted with zero to 4 R_{g}, C₂₋₃ alkynyl substituted with zero to 4 R_{g}, C₃₋₄ cycloalkyl substituted with zero to 4 R_{g}, phenyl substituted with zero to 3 R_{g}, oxadiazolyl substituted with zero to 3 R_{g}, pyridinyl substituted with zero to 3 R_{g}, -(CH₂)₁₋₂(heterocyclyl substituted with zero to 4 R_{g}), -(CH₂)₁₋₂NR_{c}C(O)(C₁₋₄ alkyl), -(CH₂)₁₋₂NR_{c}C(O)O(C₁₋₄ alkyl), -(CH₂)₁₋₂NR_{c}S(O)₂(C₁₋₄ alkyl), -C(O)(C₁₋₄ alkyl), -C(O)OH, -C(O)O(C₁₋₄ alkyl), -C(O)O(C₃₋₄ cycloalkyl), -C(O)NRₐRₐ, or -C(O)NRₐ(C₃₋₄ cycloalkyl), or two R₅ attached to the same carbon atom form =O. Included in this embodiment are compounds in which each R₅ is independently F, -CN, -OH, C₁₋₂ alkyl substituted with zero to 3 R_{g}, C₁₋₂ alkoxy substituted with zero to 3 R_{g}, C₃₋₄ cycloalkyl substituted with zero to 2 R_{g}, -C(O)(C₁₋₄ alkyl), -C(O)OH, -C(O)O(C₁₋₂ alkyl), or -C(O)NRₐRₐ, or two R₅ attached to the same carbon atom form =O. Also included in this embodiment are compounds in which each R₅ is -CH₃, or two R₅ attached to the same carbon atom form =O.

In one embodiment, a compound of Formula (I) or a salt thereof is provided wherein m is 2 and the two R₅ are attached to the same carbon atom to form =O.

In one embodiment, a compound of Formula (I) or a salt thereof is provided wherein m is zero.

In one embodiment, a compound of Formula (I) or a salt thereof is provided wherein m is 1, 2, or 3. Included in this embodiment are compounds in which m is 1 or 2. Also included in this embodiment are compounds in which m is 1.

In one embodiment, a compound of Formula (I) or a salt thereof is provided wherein m is 2 or 3. Included in this embodiment are compounds in which m is 2.

In one embodiment, a compound of Formula (I) or a salt thereof is provided wherein m is 3.

In one embodiment, a compound of Formula (I) or a salt thereof is provided having the structure of Formula (III):

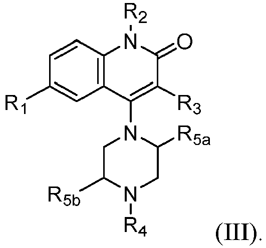

In one embodiment, a compound of Formula (III) or a salt thereof is provided wherein R₅ₐ is -CH₃ and R_{5c} is -CH₃.

In one embodiment, a compound of Formula (III) or a salt thereof is provided wherein R₅ₐ is -CH₃ and R_{5c} is -CH₂CH₃.

In one embodiment, a compound of Formula (III) or a salt thereof is provided wherein R₅ₐ is -CH₂CH₃ and R_{5c} is -CH₃.

In one embodiment, a compound of Formula (III) or a salt thereof is provided wherein R₅ₐ is -CH₂CH₃ and R_{5c} is -CH₂CH₃.

In one embodiment, a compound of Formula (III) or a salt thereof is provided wherein R₅ₐ is -CH₃ and R_{5c} is -CH₂OH.

In one embodiment, a compound of Formula (III) or a salt thereof is provided wherein R₅ₐ is -CH₃ and R_{5c} is -CH₂OCH₃.

In one embodiment, a compound of Formula (I) or a compound of Formula (III) or a salt thereof is provided having the structure: 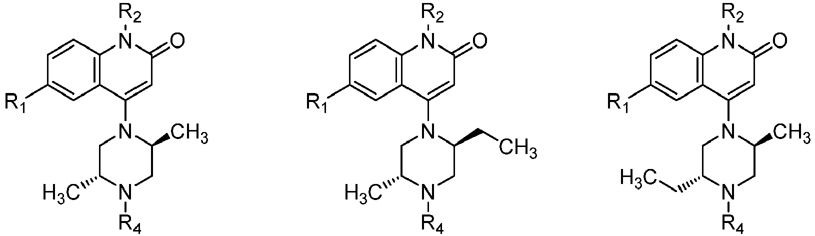 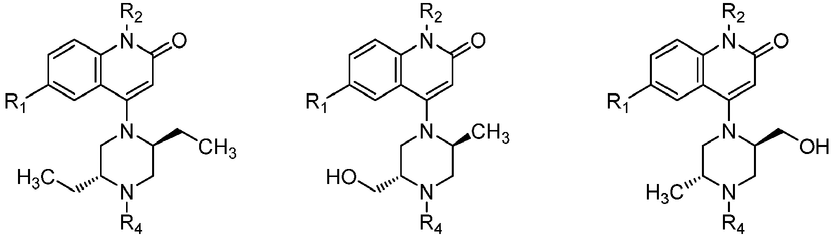 or

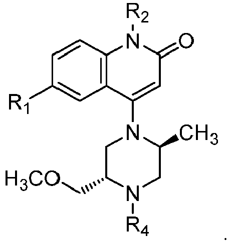

In one embodiment, a compound of Formula (I) or a salt thereof is provided wherein R₄ is:
(i) 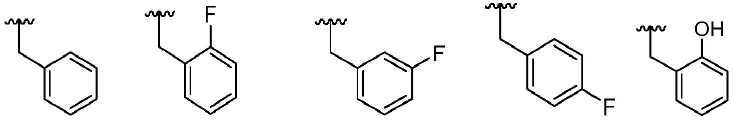 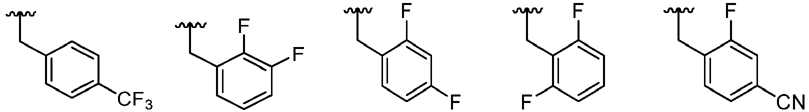 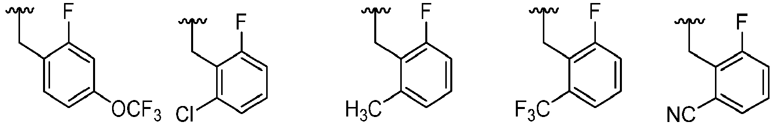 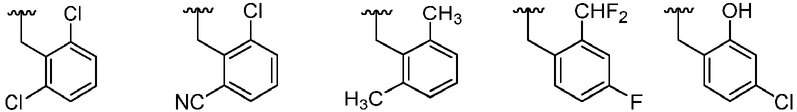 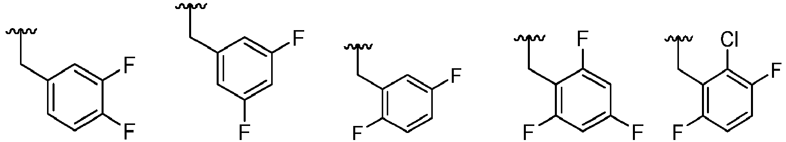 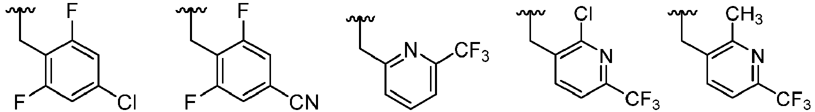 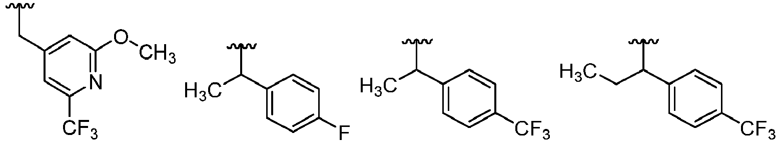 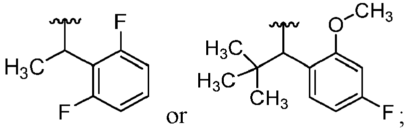
(ii) 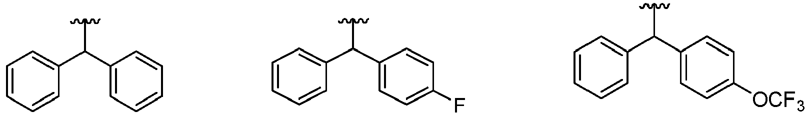 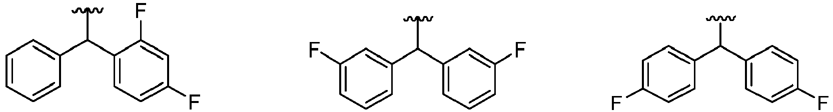 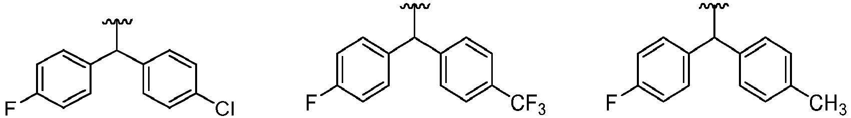 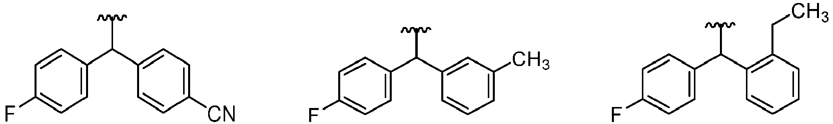 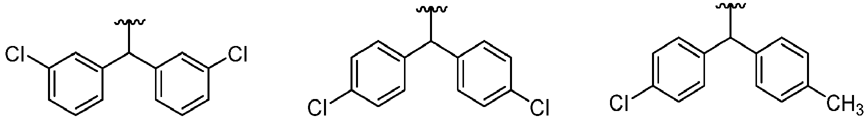 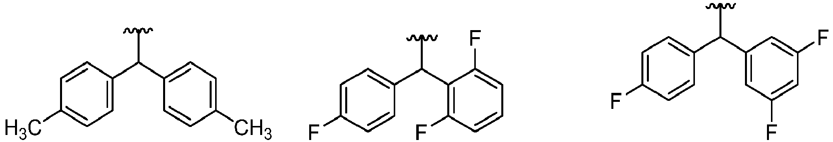 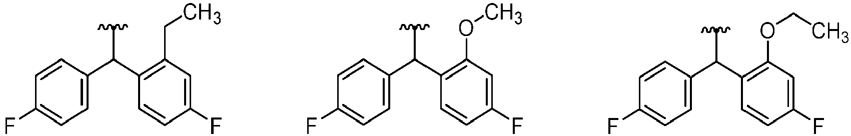 or 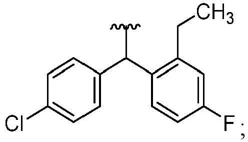 or
(iii) 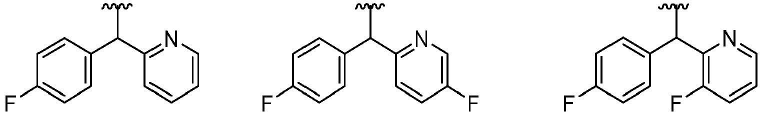 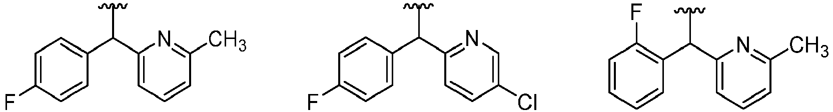 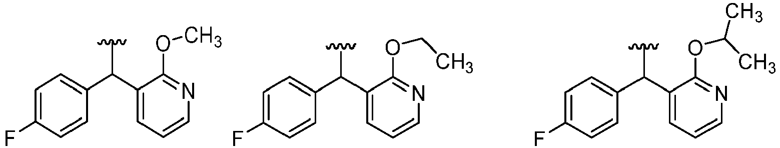 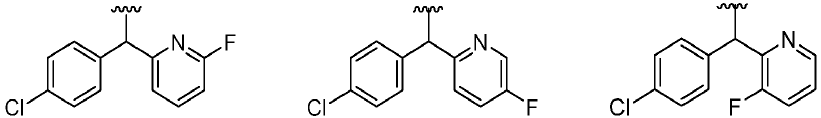 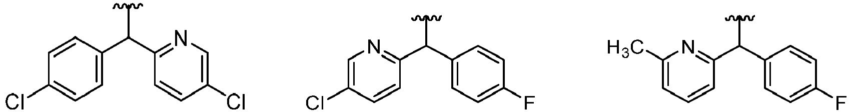 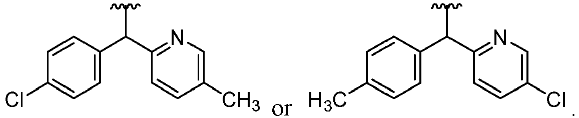 Included in this embodiment are compounds in which R₁ is Br, -CN, or -OCH₃; and R₂ is -CH₃.

In one embodiment, a compound of Formula (I) or a salt thereof is provided wherein R₄ is: 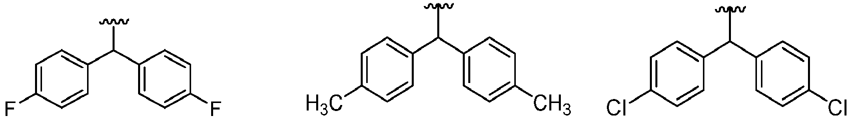 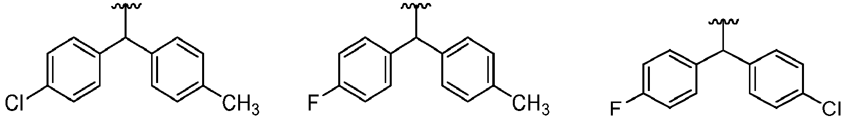 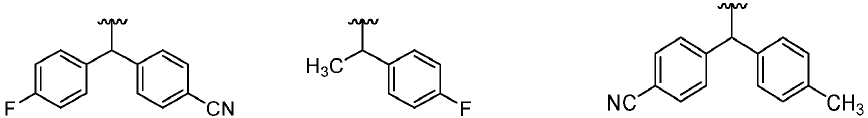 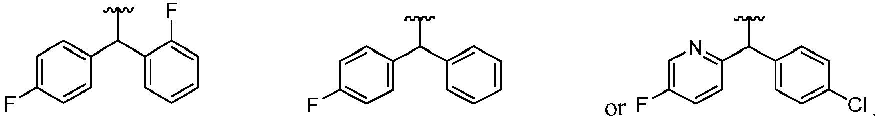 Included in this embodiment are compounds in which R₁ is Br, -CN, or -OCH₃; and R₂ is -CH₃. Also included in this embodiment are compounds in which R₁ is -CN; and R₂ is -CH₃.

In one embodiment, a compound of Formula (I) or a salt thereof is provided wherein said compound is:
6-fluoro-4-{4-[(3 -fluorophenyl)methyl]piperazin-1-yl} -1 -methyl-3 -nitro-1,2-dihydroquinolin-2-one (1);
6-fluoro-4-{4-[(3-fluoro-5-methylphenyl)methyl]piperazin-1-yl}-1-methyl-3-(pyridin-4-yl)-1,2-dihydroquinolin-2-one (2);
4-(4-((1H-indol-4-yl)methyl)piperazin-1-yl)-6-fluoro-1-methyl-3-nitroquinolin-2(1H)-one (3);
4-{4-[(1-ethyl-1H-indol-4-yl)methyl]piperazin-1-yl}-6-fluoro-1-methyl-3-nitro-1,2-dihydroquinolin-2-one (4);
4-(4-benzyl-3-oxopiperazin-1-yl)-6-fluoro-1-methyl-3-nitro-1,2-dihydroquinolin-2-one (5);
4-[(2 S, SR)-4-[bis(4-fluorophenyl)methyl]-2, 5-dimethylpiperazin-1-yl]-1-methyl-2-oxo-1,2-dihydroquinoline-6-carbonitrile (7);
6-methoxy-1-methyl-4-{4-[(naphthalen-1-yl)methyl]piperazin-1-yl}-3-nitro-1,2-dihydroquinolin-2-one (8).

### DEFINITIONS

The features and advantages of the invention may be more readily understood by those of ordinary skill in the art upon reading the following detailed description. It is to be appreciated that certain features of the invention that are, for clarity reasons, described above and below in the context of separate embodiments, may also be combined to form a single embodiment. Conversely, various features of the invention that are, for brevity reasons, described in the context of a single embodiment, may also be combined so as to form sub-combinations thereof. Embodiments identified herein as exemplary or preferred are intended to be illustrative and not limiting.

Unless specifically stated otherwise herein, references made in the singular may also include the plural. For example, "a" and "an" may refer to either one, or one or more.

As used herein, the phrase "compounds and/or salts thereof' refers to at least one compound, at least one salt of the compounds, or a combination thereof. For example, compounds of Formula (I) and/or salts thereof includes a compound of Formula (I); two compounds of Formula (I); a salt of a compound of Formula (I); a compound of Formula (I) and one or more salts of the compound of Formula (I); and two or more salts of a compound of Formula (I).

Unless otherwise indicated, any atom with unsatisfied valences is assumed to have hydrogen atoms sufficient to satisfy the valences.

Listed below are definitions of various terms used to describe the present invention. These definitions apply to the terms as they are used throughout the specification (unless they are otherwise limited in specific instances) either individually or as part of a larger group.

Throughout the specification, groups and substituents thereof may be chosen by one skilled in the field to provide stable moieties and compounds.

In accordance with a convention used in the art, 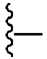 is used in structural formulas herein to depict the bond that is the point of attachment of the moiety or substituent to the core or backbone structure.

The terms "halo" and "halogen," as used herein, refer to F, Cl, Br, and I.

The term "cyano" refers to the group -CN.

The term "amino" refers to the group -NH₂.

The term "oxo" refers to the group =O.

The term "alkyl" as used herein, refers to both branched and straight-chain saturated aliphatic hydrocarbon groups containing, for example, from 1 to 12 carbon atoms, from 1 to 6 carbon atoms, and from 1 to 4 carbon atoms. Examples of alkyl groups include, but are not limited to, methyl (Me), ethyl (Et), propyl (*e.g*., n-propyl and i-propyl), butyl (*e.g*., n-butyl, i-butyl, sec-butyl, and *t*-butyl), and pentyl *(e.g.,* n-pentyl, isopentyl, neopentyl), n-hexyl, 2-methylpentyl, 2-ethylbutyl, 3-methylpentyl, and 4-methylpentyl. When numbers appear in a subscript after the symbol "C", the subscript defines with more specificity the number of carbon atoms that a particular group may contain. For example, "C₁₋₄ alkyl" denotes straight and branched chain alkyl groups with one to four carbon atoms.

The term "fluoroalkyl" as used herein is intended to include both branched and straight-chain saturated aliphatic hydrocarbon groups substituted with one or more fluorine atoms. For example, "C₁₋₄ fluoroalkyl" is intended to include C₁, C₂, C₃, and C₄ alkyl groups substituted with one or more fluorine atoms. Representative examples of fluoroalkyl groups include, but are not limited to, -CF₃ and -CH₂CF₃.

The term "hydroxyalkyl" includes both branched and straight-chain saturated alkyl groups substituted with one or more hydroxyl groups. For example, "hydroxyalkyl" includes -CH₂OH, -CH₂CH₂OH, and C₁₋₄ hydroxyalkyl.

The term "alkenyl" refers to a straight or branched chain hydrocarbon radical containing from 2 to 12 carbon atoms and at least one carbon-carbon double bond. Exemplary such groups include ethenyl or allyl. For example, "C₂₋₆ alkenyl" denotes straight and branched chain alkenyl groups with two to six carbon atoms.

The term "alkynyl" refers to a straight or branched chain hydrocarbon radical containing from 2 to 12 carbon atoms and at least one carbon to carbon triple bond. Exemplary such groups include ethynyl. For example, "C₂₋₆ alkynyl" denotes straight and branched chain alkynyl groups with two to six carbon atoms.

The term "cycloalkyl," as used herein, refers to a group derived from a nonaromatic monocyclic or polycyclic hydrocarbon molecule by removal of one hydrogen atom from a saturated ring carbon atom. Representative examples of cycloalkyl groups include, but are not limited to, cyclopropyl, cyclopentyl, and cyclohexyl. When numbers appear in a subscript after the symbol "C", the subscript defines with more specificity the number of carbon atoms that a particular cycloalkyl group may contain. For example, "C₃₋₆ cycloalkyl" denotes cycloalkyl groups with three to six carbon atoms.

The term "fluorocycloalkyl" as used herein is intended to include a cycloalkyl group substituted with one or more fluorine atoms.

The term "alkoxy," as used herein, refers to an alkyl group attached to the parent molecular moiety through an oxygen atom, for example, methoxy group (-OCH₃). For example, "C₁₋₃ alkoxy" denotes alkoxy groups with one to three carbon atoms.

The terms "fluoroalkoxy" and "-O(fluoroalkyl)" represent a fluoroalkyl group as defined above attached through an oxygen linkage (-O-). For example, "C₁₋₄ fluoroalkoxy" is intended to include C₁, C₂, C₃, and C₄ fluoroalkoxy groups.

The terms "carbocyclo", "carbocyclic" or "carbocyclyl" may be used interchangeably and refer to cyclic groups having at least one saturated or partially saturated non-aromatic ring wherein all atoms of all rings are carbon. The carbocyclyl ring may be unsubstituted or may contain one or more substituents as valence allows. Thus, the term includes nonaromatic rings such as for example, cycloalkyl, cycloalkenyl, and cycloalkynyl rings. Exemplary bicyclic carbocyclyl groups include, indanyl, indenyl, dihydronaphthalenyl, tetrahydronaphthenyl, hexahydronaphthalenyl, octahydronaphthalenyl, decahydronaphthalenyl, bicycloheptanyl, bicyclooctanyl, and bicyclononanyl.

The term "aryl" as used herein, refers to a group of atoms derived from a molecule containing aromatic ring(s) by removing one hydrogen that is bonded to the aromatic ring(s). Representative examples of aryl groups include, but are not limited to, phenyl and naphthyl. The aryl ring may be unsubstituted or may contain one or more substituents as valence allows.

The term "benzyl," as used herein, refers to a methyl group in which one of the hydrogen atoms is replaced by a phenyl group. The phenyl ring may be unsubstituted or may contain one or more substituents as valence allows.

The term "heteroatom" refers to oxygen (O), sulfur (S), and nitrogen (N).

The terms "heterocyclo", "heterocyclic", or "heterocyclyl" may be used interchangeably and refer to cyclic groups having at least one saturated or partially saturated non-aromatic ring and wherein one or more of the rings have at least one heteroatom (O, S or N), said heteroatom containing ring preferably having 1 to 3 heteroatoms independently selected from O, S, and/or N. The ring of such a group containing a heteroatom can contain one or two oxygen or sulfur atoms and/or from one to four nitrogen atoms provided that the total number of heteroatoms in each ring is four or less, and further provided that the ring contains at least one carbon atom. The nitrogen and sulfur atoms may optionally be oxidized and the nitrogen atoms may optionally be quaternized. The heterocyclo group may be attached at any available nitrogen or carbon atom. The heterocyclo ring may be unsubstituted or may contain one or more substituents as valence allows.

Exemplary monocyclic heterocyclyl groups include pyrrolidinyl, imidazolinyl, oxazolidinyl, isoxazolinyl, thiazolidinyl, isothiazolidinyl, tetrahydrofuranyl, piperidinyl, piperazinyl, 2-oxopiperazinyl, 2-oxopiperidinyl, 2-oxopyrrolodinyl, 2-oxoazepinyl, azepinyl, 4-piperidonyl, tetrahydropyranyl, morpholinyl, thiamorpholinyl, thiamorpholinyl sulfoxide, thiamorpholinyl sulfone, 1,3-dioxolane, tetrahydro-1,1-dioxothienyl, dihydroisoindolyl, and tetrahydroquinolinyl
The term "heteroaryl" refers to substituted and unsubstituted aromatic 5- or 6-membered monocyclic groups and 9- or 10-membered bicyclic groups that have at least one heteroatom (O, S or N) in at least one of the rings, said heteroatom-containing ring preferably having 1, 2, or 3 heteroatoms independently selected from O, S, and/or N. Each ring of the heteroaryl group containing a heteroatom can contain one or two oxygen or sulfur atoms and/or from one to four nitrogen atoms provided that the total number of heteroatoms in each ring is four or less and each ring has at least one carbon atom. The fused rings completing the bicyclic group are aromatic and may contain only carbon atoms. The nitrogen and sulfur atoms may optionally be oxidized and the nitrogen atoms may optionally be quaternized. Bicyclic heteroaryl groups must include only aromatic rings. The heteroaryl group may be attached at any available nitrogen or carbon atom of any ring. The heteroaryl ring system may be unsubstituted or may contain one or more substituents.

Exemplary monocyclic heteroaryl groups include pyrrolyl, pyrazolyl, pyrazolinyl, imidazolyl, oxazolyl, isoxazolyl, thiazolyl, thiadiazolyl, isothiazolyl, furanyl, thiophenyl, oxadiazolyl, pyridinyl, pyrazinyl, pyrimidinyl, pyridazinyl, and triazinyl.

Exemplary bicyclic heteroaryl groups include indolyl, benzothiazolyl, benzodioxolyl, benzoxazolyl, benzothienyl, quinolinyl, tetrahydroisoquinolinyl, isoquinolinyl, benzimidazolyl, benzopyranyl, indolizinyl, benzofuranyl, chromonyl, coumarinyl, benzopyranyl, cinnolinyl, quinoxalinyl, indazolyl, and pyrrolopyridyl.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The compounds of Formula (I) can form salts which are also within the scope of this invention. Unless otherwise indicated, reference to an inventive compound is understood to include reference to one or more salts thereof. The term "salt(s)" denotes acidic and/or basic salts formed with inorganic and/or organic acids and bases. In addition, the term "salt(s) may include zwitterions (inner salts), *e.g*., when a compound of Formula (I) contains both a basic moiety, such as an amine or a pyridine or imidazole ring, and an acidic moiety, such as a carboxylic acid. Pharmaceutically acceptable (*i.e.,* non-toxic, physiologically acceptable) salts are preferred, such as, for example, acceptable metal and amine salts in which the cation does not contribute significantly to the toxicity or biological activity of the salt. However, other salts may be useful, *e.g*., in isolation or purification steps which may be employed during preparation, and thus, are contemplated within the scope of the invention. Salts of the compounds of the formula (I) may be formed, for example, by reacting a compound of the Formula (I) with an amount of acid or base, such as an equivalent amount, in a medium such as one in which the salt precipitates or in an aqueous medium followed by lyophilization.

Exemplary acid addition salts include acetates (such as those formed with acetic acid or trihaloacetic acid, for example, trifluoroacetic acid), adipates, alginates, ascorbates, aspartates, benzoates, benzenesulfonates, bisulfates, borates, butyrates, citrates, camphorates, camphorsulfonates, cyclopentanepropionates, digluconates, dodecyl sulfates, ethanesulfonates, fumarates, glucoheptanoates, glycerophosphates, hemisulfates, heptanoates, hexanoates, hydrochlorides (formed with hydrochloric acid), hydrobromides (formed with hydrogen bromide), hydroiodides, maleates (formed with maleic acid), 2-hydroxyethanesulfonates, lactates, methanesulfonates (formed with methanesulfonic acid), 2-naphthalenesulfonates, nicotinates, nitrates, oxalates, pectinates, persulfates, 3-phenylpropionates, phosphates, picrates, pivalates, propionates, salicylates, succinates, sulfates (such as those formed with sulfuric acid), sulfonates (such as those mentioned herein), tartrates, thiocyanates, toluenesulfonates such as tosylates, undecanoates, and the like.

Exemplary basic salts include ammonium salts, alkali metal salts such as sodium, lithium, and potassium salts; alkaline earth metal salts such as calcium and magnesium salts; barium, zinc, and aluminum salts; salts with organic bases (for example, organic amines) such as trialkylamines such as triethylamine, procaine, dibenzylamine, N-benzyl-β-phenethylamine, 1-ephenamine, N,N'-dibenzylethylene-diamine, dehydroabietylamine, N-ethylpiperidine, benzylamine, dicyclohexylamine or similar pharmaceutically acceptable amines and salts with amino acids such as arginine, lysine and the like. Basic nitrogen-containing groups may be quaternized with agents such as lower alkyl halides (*e.g*., methyl, ethyl, propyl, and butyl chlorides, bromides and iodides), dialkyl sulfates (*e.g.,* dimethyl, diethyl, dibutyl, and diamyl sulfates), long chain halides (*e.g.,* decyl, lauryl, myristyl and stearyl chlorides, bromides and iodides), aralkyl halides (*e.g*., benzyl and phenethyl bromides), and others. Preferred salts include monohydrochloride, hydrogensulfate, methanesulfonate, phosphate or nitrate salts.

The compounds of Formula (I) can be provided as amorphous solids or crystalline solids. Lyophilization can be employed to provide the compounds of Formula (I) as a solid.

It should further be understood that solvates (e.g., hydrates) of the Compounds of Formula (I) are also within the scope of the present invention. The term "solvate" means a physical association of a compound of Formula (I) with one or more solvent molecules, whether organic or inorganic. This physical association includes hydrogen bonding. In certain instances the solvate will be capable of isolation, for example when one or more solvent molecules are incorporated in the crystal lattice of the crystalline solid. "Solvate" encompasses both solution-phase and isolable solvates. Exemplary solvates include hydrates, ethanolates, methanolates, isopropanolates, acetonitrile solvates, and ethyl acetate solvates. Methods of solvation are known in the art.

In addition, compounds of Formula (I), subsequent to their preparation, can be isolated and purified to obtain a composition containing an amount by weight equal to or greater than 99% of a compound of Formula (I) ("substantially pure"), which is then used or formulated as described herein. Such "substantially pure" compounds of Formula (I) are also contemplated herein as part of the present invention.

"Stable compound" and "stable structure" are meant to indicate a compound that is sufficiently robust to survive isolation to a useful degree of purity from a reaction mixture, and formulation into an efficacious therapeutic agent. The present invention is intended to embody stable compounds.

"Therapeutically effective amount" is intended to include an amount of a compound of the present invention alone or an amount of the combination of compounds claimed or an amount of a compound of the present invention in combination with other active ingredients effective to act as an inhibitor of DGKα and/or DGKζ, or effective to treat or prevent viral infections and proliferative disorders, such as cancer.

As used herein, "treating" or "treatment" cover the treatment of a disease-state in a mammal, particularly in a human, and include: (a) preventing the disease-state from occurring in a mammal, in particular, when such mammal is predisposed to the disease-state but has not yet been diagnosed as having it; (b) inhibiting the disease-state, i.e., arresting its development; and/or (c) relieving the disease-state, i.e., causing regression of the disease state.

The compounds of the present invention are intended to include all isotopes of atoms occurring in the present compounds. Isotopes include those atoms having the same atomic number but different mass numbers. By way of general example and without limitation, isotopes of hydrogen include deuterium (D) and tritium (T). Isotopes of carbon include ¹³C and ¹⁴C. Isotopically-labeled compounds of the invention can generally be prepared by conventional techniques known to those skilled in the art or by processes analogous to those described herein, using an appropriate isotopically-labeled reagent in place of the non-labeled reagent otherwise employed.

Compounds in accordance with Formula (I) and/or pharmaceutically acceptable salts thereof can be administered by any means suitable for the condition to be treated, which can depend on the need for site-specific treatment or quantity of Formula (I) compound to be delivered.

Also embraced within this invention is a class of pharmaceutical compositions comprising a compound of Formula (I) and/or pharmaceutically acceptable salts thereof; and one or more non-toxic, pharmaceutically-acceptable carriers and/or diluents and/or adjuvants (collectively referred to herein as "carrier" materials) and, if desired, other active ingredients. The compounds of Formula (I) may be administered by any suitable route, preferably in the form of a pharmaceutical composition adapted to such a route, and in a dose effective for the treatment intended. The compounds and compositions of the present invention may, for example, be administered orally, mucosally, or parentally including intravascularly, intravenously, intraperitoneally, subcutaneously, intramuscularly, and intrasternally in dosage unit formulations containing conventional pharmaceutically acceptable carriers, adjuvants, and vehicles. For example, the pharmaceutical carrier may contain a mixture of mannitol or lactose and microcrystalline cellulose. The mixture may contain additional components such as a lubricating agent, e.g. magnesium stearate and a disintegrating agent such as crospovidone. The carrier mixture may be filled into a gelatin capsule or compressed as a tablet. The pharmaceutical composition may be administered as an oral dosage form or an infusion, for example.

For oral administration, the pharmaceutical composition may be in the form of, for example, a tablet, capsule, liquid capsule, suspension, or liquid. The pharmaceutical composition is preferably made in the form of a dosage unit containing a particular amount of the active ingredient. For example, the pharmaceutical composition may be provided as a tablet or capsule comprising an amount of active ingredient in the range of from about 0.1 to 1000 mg, preferably from about 0.25 to 250 mg, and more preferably from about 0.5 to 100 mg. A suitable daily dose for a human or other mammal may vary widely depending on the condition of the patient and other factors, but, can be determined using routine methods.

Any pharmaceutical composition contemplated herein can, for example, be delivered orally via any acceptable and suitable oral preparations. Exemplary oral preparations, include, but are not limited to, for example, tablets, troches, lozenges, aqueous and oily suspensions, dispersible powders or granules, emulsions, hard and soft capsules, liquid capsules, syrups, and elixirs. Pharmaceutical compositions intended for oral administration can be prepared according to any methods known in the art for manufacturing pharmaceutical compositions intended for oral administration. In order to provide pharmaceutically palatable preparations, a pharmaceutical composition in accordance with the invention can contain at least one agent selected from sweetening agents, flavoring agents, coloring agents, demulcents, antioxidants, and preserving agents.

A tablet can, for example, be prepared by admixing at least one compound of Formula (I) and/or at least one pharmaceutically acceptable salt thereof with at least one non-toxic pharmaceutically acceptable excipient suitable for the manufacture of tablets. Exemplary excipients include, but are not limited to, for example, inert diluents, such as, for example, calcium carbonate, sodium carbonate, lactose, calcium phosphate, and sodium phosphate; granulating and disintegrating agents, such as, for example, microcrystalline cellulose, sodium crosscarmellose, corn starch, and alginic acid; binding agents, such as, for example, starch, gelatin, polyvinyl-pyrrolidone, and acacia; and lubricating agents, such as, for example, magnesium stearate, stearic acid, and talc. Additionally, a tablet can either be uncoated, or coated by known techniques to either mask the bad taste of an unpleasant tasting drug, or delay disintegration and absorption of the active ingredient in the gastrointestinal tract thereby sustaining the effects of the active ingredient for a longer period. Exemplary water soluble taste masking materials, include, but are not limited to, hydroxypropyl-methylcellulose and hydroxypropylcellulose. Exemplary time delay materials, include, but are not limited to, ethyl cellulose and cellulose acetate butyrate.

Hard gelatin capsules can, for example, be prepared by mixing at least one compound of Formula (I) and/or at least one salt thereof with at least one inert solid diluent, such as, for example, calcium carbonate; calcium phosphate; and kaolin.

Soft gelatin capsules can, for example, be prepared by mixing at least one compound of Formula (I) and/or at least one pharmaceutically acceptable salt thereof with at least one water soluble carrier, such as, for example, polyethylene glycol; and at least one oil medium, such as, for example, peanut oil, liquid paraffin, and olive oil.

An aqueous suspension can be prepared, for example, by admixing at least one compound of Formula (I) and/or at least one pharmaceutically acceptable salt thereof with at least one excipient suitable for the manufacture of an aqueous suspension. Exemplary excipients suitable for the manufacture of an aqueous suspension, include, but are not limited to, for example, suspending agents, such as, for example, sodium carboxymethylcellulose, methylcellulose, hydroxypropylmethyl-cellulose, sodium alginate, alginic acid, polyvinyl-pyrrolidone, gum tragacanth, and gum acacia; dispersing or wetting agents, such as, for example, a naturally-occurring phosphatide, e.g., lecithin; condensation products of alkylene oxide with fatty acids, such as, for example, polyoxyethylene stearate; condensation products of ethylene oxide with long chain aliphatic alcohols, such as, for example heptadecaethylene-oxycetanol; condensation products of ethylene oxide with partial esters derived from fatty acids and hexitol, such as, for example, polyoxyethylene sorbitol monooleate; and condensation products of ethylene oxide with partial esters derived from fatty acids and hexitol anhydrides, such as, for example, polyethylene sorbitan monooleate. An aqueous suspension can also contain at least one preservative, such as, for example, ethyl and n-propyl p-hydroxybenzoate; at least one coloring agent; at least one flavoring agent; and/or at least one sweetening agent, including but not limited to, for example, sucrose, saccharin, and aspartame.

Oily suspensions can, for example, be prepared by suspending at least one compound of Formula (I) and/or at least one pharmaceutically acceptable salt thereof in either a vegetable oil, such as, for example, arachis oil; olive oil; sesame oil; and coconut oil; or in mineral oil, such as, for example, liquid paraffin. An oily suspension can also contain at least one thickening agent, such as, for example, beeswax; hard paraffin; and cetyl alcohol. In order to provide a palatable oily suspension, at least one of the sweetening agents already described hereinabove, and/or at least one flavoring agent can be added to the oily suspension. An oily suspension can further contain at least one preservative, including, but not limited to, for example, an anti-oxidant, such as, for example, butylated hydroxyanisol, and alpha-tocopherol.

Dispersible powders and granules can, for example, be prepared by admixing at least one compound of Formula (I) and/or at least one pharmaceutically acceptable salt thereof with at least one dispersing and/or wetting agent; at least one suspending agent; and/or at least one preservative. Suitable dispersing agents, wetting agents, and suspending agents are as already described above. Exemplary preservatives include, but are not limited to, for example, anti-oxidants, e.g., ascorbic acid. In addition, dispersible powders and granules can also contain at least one excipient, including, but not limited to, for example, sweetening agents; flavoring agents; and coloring agents.

An emulsion of at least one compound of Formula (I) and/or at least one pharmaceutically acceptable salt thereof can, for example, be prepared as an oil-in-water emulsion. The oily phase of the emulsions comprising compounds of Formula (I) may be constituted from known ingredients in a known manner. The oil phase can be provided by, but is not limited to, for example, a vegetable oil, such as, for example, olive oil and arachis oil; a mineral oil, such as, for example, liquid paraffin; and mixtures thereof. While the phase may comprise merely an emulsifier, it may comprise a mixture of at least one emulsifier with a fat or an oil or with both a fat and an oil. Suitable emulsifying agents include, but are not limited to, for example, naturally-occurring phosphatides, e.g., soy bean lecithin; esters or partial esters derived from fatty acids and hexitol anhydrides, such as, for example, sorbitan monooleate; and condensation products of partial esters with ethylene oxide, such as, for example, polyoxyethylene sorbitan monooleate. Preferably, a hydrophilic emulsifier is included together with a lipophilic emulsifier which acts as a stabilizer. It is also preferred to include both an oil and a fat. Together, the emulsifier(s) with or without stabilizer(s) make-up the so-called emulsifying wax, and the wax together with the oil and fat make up the so-called emulsifying ointment base which forms the oily dispersed phase of the cream formulations. An emulsion can also contain a sweetening agent, a flavoring agent, a preservative, and/or an antioxidant. Emulsifiers and emulsion stabilizers suitable for use in the formulation of the present invention include Tween 60, Span 80, cetostearyl alcohol, myristyl alcohol, glyceryl monostearate, sodium lauryl sulfate, glyceryl distearate alone or with a wax, or other materials well known in the art.

The compounds of Formula (I) and/or at least one pharmaceutically acceptable salt thereof can, for example, also be delivered intravenously, subcutaneously, and/or intramuscularly via any pharmaceutically acceptable and suitable injectable form. Exemplary injectable forms include, but are not limited to, for example, sterile aqueous solutions comprising acceptable vehicles and solvents, such as, for example, water, Ringer's solution, and isotonic sodium chloride solution; sterile oil-in-water microemulsions; and aqueous or oleaginous suspensions.

Formulations for parenteral administration may be in the form of aqueous or non-aqueous isotonic sterile injection solutions or suspensions. These solutions and suspensions may be prepared from sterile powders or granules using one or more of the carriers or diluents mentioned for use in the formulations for oral administration or by using other suitable dispersing or wetting agents and suspending agents. The compounds may be dissolved in water, polyethylene glycol, propylene glycol, ethanol, corn oil, cottonseed oil, peanut oil, sesame oil, benzyl alcohol, sodium chloride, tragacanth gum, and/or various buffers. Other adjuvants and modes of administration are well and widely known in the pharmaceutical art. The active ingredient may also be administered by injection as a composition with suitable carriers including saline, dextrose, or water, or with cyclodextrin (i.e. Captisol), cosolvent solubilization (i.e. propylene glycol) or micellar solubilization (i.e. Tween 80).

The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed, including synthetic mono- or diglycerides. In addition, fatty acids such as oleic acid find use in the preparation of injectables.

A sterile injectable oil-in-water microemulsion can, for example, be prepared by 1) dissolving at least one compound of Formula (I) in an oily phase, such as, for example, a mixture of soybean oil and lecithin; 2) combining the Formula (I) containing oil phase with a water and glycerol mixture; and 3) processing the combination to form a microemulsion.

A sterile aqueous or oleaginous suspension can be prepared in accordance with methods already known in the art. For example, a sterile aqueous solution or suspension can be prepared with a non-toxic parenterally-acceptable diluent or solvent, such as, for example, 1,3-butane diol; and a sterile oleaginous suspension can be prepared with a sterile non-toxic acceptable solvent or suspending medium, such as, for example, sterile fixed oils, e.g., synthetic mono- or diglycerides; and fatty acids, such as, for example, oleic acid.

Pharmaceutically acceptable carriers, adjuvants, and vehicles that may be used in the pharmaceutical compositions of this invention include, but are not limited to, ion exchangers, alumina, aluminum stearate, lecithin, self-emulsifying drug delivery systems (SEDDS) such as d-alpha-tocopherol polyethyleneglycol 1000 succinate, surfactants used in pharmaceutical dosage forms such as Tweens, polyethoxylated castor oil such as CREMOPHOR surfactant (BASF), or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylenepolyoxypropylene-block polymers, polyethylene glycol and wool fat. Cyclodextrins such as alpha-, beta-, and gamma-cyclodextrin, or chemically modified derivatives such as hydroxyalkylcyclodextrins, including 2- and 3-hydroxypropyl-cyclodextrins, or other solubilized derivatives may also be advantageously used to enhance delivery of compounds of the formulae described herein.

The pharmaceutically active compounds of this invention can be processed in accordance with conventional methods of pharmacy to produce medicinal agents for administration to patients, including humans and other mammals. The pharmaceutical compositions may be subjected to conventional pharmaceutical operations such as sterilization and/or may contain conventional adjuvants, such as preservatives, stabilizers, wetting agents, emulsifiers, buffers etc. Tablets and pills can additionally be prepared with enteric coatings. Such compositions may also comprise adjuvants, such as wetting, sweetening, flavoring, and perfuming agents.

The amounts of compounds that are administered and the dosage regimen for treating a disease condition with the compounds and/or compositions of this invention depends on a variety of factors, including the age, weight, sex, the medical condition of the subject, the type of disease, the severity of the disease, the route and frequency of administration, and the particular compound employed. Thus, the dosage regimen may vary widely, but can be determined routinely using standard methods. A daily dose of about 0.001 to 100 mg/kg body weight, preferably between about 0.0025 and about 50 mg/kg body weight and most preferably between about 0.005 to 10 mg/kg body weight, may be appropriate. The daily dose can be administered in one to four doses per day. Other dosing schedules include one dose per week and one dose per two day cycle.

For therapeutic purposes, the active compounds of this invention are ordinarily combined with one or more adjuvants appropriate to the indicated route of administration. If administered orally, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose.

Pharmaceutical compositions of this invention comprise at least one compound of Formula (I) and/or at least one pharmaceutically acceptable salt thereof, and optionally an additional agent selected from any pharmaceutically acceptable carrier, adjuvant, and vehicle. Alternate compositions of this invention comprise a compound of the Formula (I) described herein, and a pharmaceutically acceptable carrier, adjuvant, or vehicle.

### UTILITY

The compounds of Formula (I) are useful for the treatment of cancer.

A combined preparation of a compound of Formula (I), and/or a pharmaceutically acceptable salt thereof, a stereoisomer thereof or a tautomer thereof, and additional therapeutic agent(s) may be used for simultaneous, separate or sequential use in the treatment and/or prophylaxis of multiple diseases or disorders associated with DGK target inhibition in T cells.

In another aspect, the invention provides a compound of Formula (I) for use in a method of treating a patient suffering from or susceptible to a medical condition that is associated with DGK target inhibition in T cells. A number of medical conditions can be treated. The method may comprise administering to the patient a therapeutically effective amount of a composition comprising a compound of Formula (I) and/or a pharmaceutically acceptable salt thereof, a stereoisomer thereof or a tautomer thereof. For example, the compounds described herein may be used to treat or prevent viral infections and proliferative diseases such as cancer.

The compounds for Formula (I) and pharmaceutical compositions comprising at least one compound of Formula (I) are useful in treating or preventing any disease or conditions that are associated with DGK target inhibition in T cells. These may include viral and other infections (*e.g*., skin infections, GI infection, urinary tract infections, genito-urinary infections, systemic infections), and proliferative diseases (*e.g*., cancer). The compounds of Formula (I) and pharmaceutical compositions comprising in at least one compound of Formula (I) may be administered to animals, preferably mammals (*e.g*., domesticated animals, cats, dogs, mice, rats), and more preferably humans. Any method of administration may be used to deliver the compound or pharmaceutical composition to the patient. The compound of Formula (I) or pharmaceutical composition comprising at least compound of Formula (I) may be administered orally. The Formula (I) or pharmaceutical composition comprising at least compound of Formula (I) may be administered parenterally.

The compounds of Formula (I) can inhibit activity of the diacylglycerol kinase alpha and zeta (DGKα/ζ). For example, the compounds of Formula (I) can be used in a method to inhibit activity of DGKα and DGKζ in a cell or in an individual in need of modulation of DGKα and DGKζ by administering an inhibiting amount of a compound of Formula (I) or a salt thereof.

The present invention further provides compounds of Formula (I) for use in methods of treating diseases associated with activity or expression, including abnormal activity and/or overexpression, of DGKα and DGKζ in an individual (*e.g*., patient) by administering to the individual in need of such treatment a therapeutically effective amount or dose of a compound of Formula (I) or a pharmaceutical composition thereof. Example diseases can include any disease, disorder or condition that is directly or indirectly linked to expression or activity of DGKα and DGKζ enzyme, such as over expression or abnormal activity. A DGKα and DGKζ -associated disease can also include any disease, disorder or condition that can be prevented, ameliorated, or cured by modulating DGKα and DGKζ enzyme activity. Examples of DGKα and DGKζ associated diseases include cancer and viral infections such as HIV infection, hepatitis B, and hepatitis C.

The compound(s) of Formula (I) may be sequentially administered prior to administration of the immuno-oncology agent. Compound(s) of Formula (I) may be administered concurrently with the immuno-oncology agent. Compound(s) of Formula (I) may be sequentially administered after administration of the immuno-oncology agent.

Compounds of Formula (I) may be co-formulated with an immuno-oncology agent.

Immuno-oncology agents include, for example, a small molecule drug, antibody, or other biologic or small molecule. Examples of biologic immuno-oncology agents include, but are not limited to, cancer vaccines, antibodies, and cytokines. The antibody may be a monoclonal antibody. The monoclonal antibody may be humanized or human.

The immuno-oncology agent may be (i) an agonist of a stimulatory (including a co-stimulatory) receptor or (ii) an antagonist of an inhibitory (including a co-inhibitory) signal on T cells, both of which result in amplifying antigen-specific T cell responses (often referred to as immune checkpoint regulators).

Certain of the stimulatory and inhibitory molecules are members of the immunoglobulin super family (IgSF). One important family of membrane-bound ligands that bind to co-stimulatory or co-inhibitory receptors is the B7 family, which includes B7-1, B7-2, B7-H1 (PD-L1), B7-DC (PD-L2), B7-H2 (ICOS-L), B7-H3, B7-H4, B7-H5 (VISTA), and B7-H6. Another family of membrane bound ligands that bind to costimulatory or co-inhibitory receptors is the TNF family of molecules that bind to cognate TNF receptor family members, which includes CD40 and CD40L, OX-40, OX-40L, CD70, CD27L, CD30, CD30L, 4-1BBL, CD137 (4-1BB), TRAIL/Apo2-L, TRAILR1/DR4, TRAII,R2/DRS, TRAILR3, TRAILR4, OPG, RANK, RANKL, TWEAKR/Fn14, TWEAK, BAFFR, EDAR, XEDAR, TACI, APRIL, BCMA, LTβR, LIGHT, DcR3, HVEM, VEGI/TL1A, TRAMP/DR3, EDAR, EDA1, XEDAR, EDA2, TNFR1, Lymphotoxin α/TNFβ, TNFR2, TNFα, LTβR, Lymphotoxin α 1β2, FAS, FASL, RELT, DR6, TROY, NGFR.

T cell responses can be stimulated by a combination of a compound of Formula (I) and one or more of (i) an antagonist of a protein that inhibits T cell activation (e.g., immune checkpoint inhibitors) such as CTLA-4, PD-1, PD-L1, PD-L2, LAG-3, TIM-3, Galectin 9, CEACAM-1, BTLA, CD69, Galectin-1, TIGIT, CD113, GPR56, VISTA, 2B4, CD48, GARP, PD1H, LAIR1, TIM-1, and TIM-4, and (ii) an agonist of a protein that stimulates T cell activation such as B7-1, B7-2, CD28, 4-1BB (CD137), 4-1BBL, ICOS, ICOS-L, OX40, OX40L, GITR, GITRL, CD70, CD27, CD40, DR3 and CD28H.

Other agents that can be combined with compounds of Formula (I) for the treatment of cancer include antagonists of inhibitory receptors on NK cells or agonists of activating receptors on NK cells. For example, compounds of Formula (I) can be combined with antagonists of KIR, such as lirilumab.

Yet other agents for combination therapies include agents that inhibit or deplete macrophages or monocytes, including but not limited to CSF-1R antagonists such as CSF-1R antagonist antibodies including RG7155 (WO11/70024, WO11/107553, WO11/131407, WO13/87699, WO13/119716, WO13/132044) or FPA-008 (WO11/140249; WO13169264; WO14/036357).

Compounds of Formula (I) can be used with one or more of agonistic agents that ligate positive costimulatory receptors, blocking agents that attenuate signaling through inhibitory receptors, antagonists, and one or more agents that increase systemically the frequency of anti-tumor T cells, agents that overcome distinct immune suppressive pathways within the tumor microenvironment (e.g., block inhibitory receptor engagement (e.g., PD-L1/PD-1 interactions), deplete or inhibit Tregs (e.g., using an anti-CD25 monoclonal antibody (e.g., daclizumab) or by ex vivo anti-CD25 bead depletion), inhibit metabolic enzymes such as IDO, or reverse/prevent T cell anergy or exhaustion) and agents that trigger innate immune activation and/or inflammation at tumor sites.

The immuno-oncology agent may be a CTLA-4 antagonist, such as an antagonistic CTLA-4 antibody. Suitable CTLA-4 antibodies include, for example, YERVOY (ipilimumab) or tremelimumab.

The immuno-oncology agent may be a PD-1 antagonist, such as an antagonistic PD-1 antibody. Suitable PD-1 antibodies include, for example, OPDIVO (nivolumab), KEYTRUDA (pembrolizumab), or MEDI-0680 (AMP-514; WO2012/145493). The immuno-oncology agent may also include pidilizumab (CT-011), though its specificity for PD-1 binding has been questioned. Another approach to target the PD-1 receptor is the recombinant protein composed of the extracellular domain of PD-L2 (B7-DC) fused to the Fc portion of IgG1, called AMP-224

The immuno-oncology agent may be a PD-L1 antagonist, such as an antagonistic PD-L1 antibody. Suitable PD-L1 antibodies include, for example, MPDL3280A (RG7446; WO2010/077634), durvalumab (MEDI4736), BMS-936559 (WO2007/005874), and MSB0010718C (WO2013/79174).

The immuno-oncology agent may be a LAG-3 antagonist, such as an antagonistic LAG-3 antibody. Suitable LAG3 antibodies include, for example, BMS-986016 (WO10/19570, WO14/08218), or IMP-731 or IMP-321 (WO08/132601, WO09/44273).

The immuno-oncology agent may be a CD137 (4-1BB) agonist, such as an agonistic CD137 antibody. Suitable CD137 antibodies include, for example, urelumab and PF-05082566 (WO12/32433).

The immuno-oncology agent may be a GITR agonist, such as an agonistic GITR antibody. Suitable GITR antibodies include, for example, BMS-986153, BMS-986156, TRX-518 (WO06/105021, WO09/009116) and MK-4166 (WO11/028683).

The immuno-oncology agent may be an IDO antagonist. Suitable IDO antagonists include, for example, INCB-024360 (WO2006/122150, WO07/75598, WO08/36653, WO08/36642), indoximod, BMS-986205, or NLG-919 (WO09/73620, WO09/1156652, WO11/56652, WO12/142237).

The immuno-oncology agent may be an OX40 agonist, such as an agonistic OX40 antibody. Suitable OX40 antibodies include, for example, MEDI-6383 or MEDI-6469.

The immuno-oncology agent may be an OX40L antagonist, such as an antagonistic OX40 antibody. Suitable OX40L antagonists include, for example, RG-7888 (WO06/029879).

The immuno-oncology agent may be a CD40 agonist, such as an agonistic CD40 antibody. The immuno-oncology agent may be a CD40 antagonist, such as an antagonistic CD40 antibody. Suitable CD40 antibodies include, for example, lucatumumab or dacetuzumab.

The immuno-oncology agent may be a CD27 agonist, such as an agonistic CD27 antibody. Suitable CD27 antibodies include, for example, varlilumab.

The immuno-oncology agent may be MGA271 (to B7H3) (WO11/109400).

The combination therapy is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single dosage form having a fixed ratio of each therapeutic agent or in multiple, single dosage forms for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Alternatively, for example, all therapeutic agents may be administered orally or all therapeutic agents may be administered by intravenous injection. Combination therapy also can embrace the administration of the therapeutic agents as described above in further combination with other biologically active ingredients and non-drug therapies (*e.g*., surgery or radiation treatment.) Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

As used herein, the term "cell" is meant to refer to a cell that is *in vitro, ex vivo* or *in vivo.* An *ex vivo* cell can be part of a tissue sample excised from an organism such as a mammal. An *in vitro* cell can be a cell in a cell culture. An *in vivo* cell may be a cell living in an organism such as a mammal.

As used herein, the term "contacting" refers to the bringing together of indicated moieties in an *in vitro* system or an *in vivo* system. For example, "contacting" the DGKα and DGKζ enzyme with a compound of Formula (I) includes the administration of a compound of the present invention to an individual or patient, such as a human, having DGKα and DGKζ, as well as, for example, introducing a compound of Formula (I) into a sample containing a cellular or purified preparation containing DGKα and DGKζ enzyme.

The term " DGKα and DGKζ inhibitor" refers to an agent capable of inhibiting the activity of diacylglycerol kinase alpha and/or diacylglycerol kinase zeta (DGKα and DGKζ) in T cells resulting in T cell stimulation. The DGKα and DGKζ inhibitor may be a reversible or irreversible DGKα and DGKζ inhibitor. "A reversible DGKα and DGKζ inhibitor" is a compound that reversibly inhibits DGKα and DGKζ enzyme activity either at the catalytic site or at a non-catalytic site and "an irreversible DGKα and DGKζ inhibitor" is a compound that irreversibly destroys DGKα and DGKζ enzyme activity by forming a covalent bond with the enzyme.

Types of cancers that may be treated with the compound of Formula (I) include, but are not limited to, brain cancers, skin cancers, bladder cancers, ovarian cancers, breast cancers, gastric cancers, pancreatic cancers, prostate cancers, colon cancers, blood cancers, lung cancers and bone cancers. Examples of such cancer types include neuroblastoma, intestine carcinoma such as rectum carcinoma, colon carcinoma, familiar adenomatous polyposis carcinoma and hereditary non-polyposis colorectal cancer, esophageal carcinoma, labial carcinoma, larynx carcinoma, hypopharynx carcinoma, tongue carcinoma, salivary gland carcinoma, gastric carcinoma, adenocarcinoma, medullary thyroid carcinoma, papillary thyroid carcinoma, renal carcinoma, kidney parenchymal carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeloid leukemia (AML), chronic myeloid leukemia (CML), adult T-cell leukemia lymphoma, diffuse large B-cell lymphoma (DLBCL), hepatocellular carcinoma, gall bladder carcinoma, bronchial carcinoma, small cell lung carcinoma, non-small cell lung carcinoma, multiple myeloma, basalioma, teratoma, retinoblastoma, choroid melanoma, seminoma, rhabdomyosarcoma, craniopharyngioma, osteosarcoma, chondrosarcoma, myosarcoma, liposarcoma, fibrosarcoma, Ewing sarcoma and plasmocytoma.

One or more additional pharmaceutical agents or treatment methods such as, for example, anti-viral agents, chemotherapeutics or other anti-cancer agents, immune enhancers, immunosuppressants, radiation, anti-tumor and anti-viral vaccines, cytokine therapy (*e.g*., IL2 and GM-CSF), and/or tyrosine kinase inhibitors can be optionally used in combination with the compounds of Formula (I) for treatment of DGKα and DGKζ associated diseases, disorders or conditions. The agents can be combined with the present compounds in a single dosage form, or the agents can be administered simultaneously or sequentially as separate dosage forms.

Suitable chemotherapeutic or other anti-cancer agents include, for example, alkylating agents (including, without limitation, nitrogen mustards, ethylenimine derivatives, alkyl sulfonates, nitrosoureas and triazenes) such as uracil mustard, chlormethine, cyclophosphamide (CYTOXAN^{®}), ifosfamide, melphalan, chlorambucil, pipobroman, triethylene-melamine, triethylenethiophosphoramine, busulfan, carmustine, lomustine, streptozocin, dacarbazine, and temozolomide.

In the treatment of melanoma, suitable agents for use in combination with the compounds of Formula (I) may include: dacarbazine (DTIC), optionally, along with other chemotherapy drugs such as carmustine (BCNU) and cisplatin; the "Dartmouth regimen", which consists of DTIC, BCNU, cisplatin and tamoxifen; a combination of cisplatin, vinblastine, and DTIC, temozolomide or YERVOY^{™}. Compounds of Formula (I) may also be combined with immunotherapy drugs, including cytokines such as interferon alpha, interleukin 2, and tumor necrosis factor (TNF) in the treatment of melanoma.

Compounds of Formula (I) may also be used in combination with vaccine therapy in the treatment of melanoma. Antimelanoma vaccines are, in some ways, similar to the anti-virus vaccines which are used to prevent diseases caused by viruses such as polio, measles, and mumps. Weakened melanoma cells or parts of melanoma cells called antigens may be injected into a patient to stimulate the body's immune system to destroy melanoma cells.

Melanomas that are confined to the arms or legs may also be treated with a combination of agents including one or more compounds of Formula (I), using a hyperthermic isolated limb perfusion technique. This treatment protocol temporarily separates the circulation of the involved limb from the rest of the body and injects high doses of chemotherapy into the artery feeding the limb, thus providing high doses to the area of the tumor without exposing internal organs to these doses that might otherwise cause severe side effects. Usually the fluid is warmed to 38.9 °C to 40 °C. Melphalan is the drug most often used in this chemotherapy procedure. This can be given with another agent called tumor necrosis factor (TNF).

Suitable chemotherapeutic or other anti-cancer agents include, for example, antimetabolites (including, without limitation, folic acid antagonists, pyrimidine analogs, purine analogs and adenosine deaminase inhibitors) such as methotrexate, 5-fluorouracil, floxuridine, cytarabine, 6-mercaptopurine, 6-thioguanine, fludarabine phosphate, pentostatine, and gemcitabine.

Suitable chemotherapeutic or other anti-cancer agents further include, for example, certain natural products and their derivatives (for example, vinca alkaloids, antitumor antibiotics, enzymes, lymphokines and epipodophyllotoxins) such as vinblastine, vincristine, vindesine, bleomycin, dactinomycin, daunorubicin, doxorubicin, epirubicin, idarubicin, ara-C, paclitaxel (Taxol), mithramycin, deoxyco-formycin, mitomycin-C, L-asparaginase, interferons (especially IFN-a), etoposide, and teniposide.

Other cytotoxic agents include navelbene, CPT-11, anastrazole, letrazole, capecitabine, reloxafine, and droloxafine.

Also suitable are cytotoxic agents such as epidophyllotoxin; an antineoplastic enzyme; a topoisomerase inhibitor; procarbazine; mitoxantrone; platinum coordination complexes such as cisplatin and carboplatin; biological response modifiers; growth inhibitors; antihormonal therapeutic agents; leucovorin; tegafur; and haematopoietic growth factors.

Other anti-cancer agent(s) include antibody therapeutics such as trastuzumab (HERCEPTIN^{®}), antibodies to costimulatory molecules such as CTLA-4, 4-1BB and PD-1, or antibodies to cytokines (IL-1O or TGF-β).

Other anti-cancer agents also include those that block immune cell migration such as antagonists to chemokine receptors, including CCR2 and CCR4.

Other anti-cancer agents also include those that augment the immune system such as adjuvants or adoptive T cell transfer.

Anti-cancer vaccines include dendritic cells, synthetic peptides, DNA vaccines and recombinant viruses.

The pharmaceutical composition of the invention may optionally include at least one signal transduction inhibitor (STI). A "signal transduction inhibitor" is an agent that selectively inhibits one or more vital steps in signaling pathways, in the normal function of cancer cells, thereby leading to apoptosis. Suitable STIs include, but are not limited to: (i) bcr/abl kinase inhibitors such as, for example, STI 571 (GLEEVEC^{®}); (ii) epidermal growth factor (EGF) receptor inhibitors such as, for example, kinase inhibitors (IRESSA^{®}, SSI-774) and antibodies (Imclone: C225 [Goldstein et al., Clin. Cancer Res., 1:1311-1318 (1995)], and Abgenix: ABX-EGF); (iii) her-2/neu receptor inhibitors such as farnesyl transferase inhibitors (FTI) such as, for example, L-744,832 (Kohl et al., Nat. Med., 1(8):792-797 (1995)); (iv) inhibitors of Akt family kinases or the Akt pathway, such as, for example, rapamycin (see, for example, Sekulic et al., Cancer Res., 60:3504-3513 (2000)); (v) cell cycle kinase inhibitors such as, for example, flavopiridol and UCN-O1 (see, for example, Sausville, Curr. Med. Chem. Anti-Canc. Agents, 3:47-56 (2003)); and (vi) phosphatidyl inositol kinase inhibitors such as, for example, LY294002 (see, for example, Vlahos et al., J. Biol. Chem., 269:5241-5248 (1994)). Alternatively, at least one STI and at least one compound of Formula (I) may be in separate pharmaceutical compositions. At least one compound of Formula (I) and at least one STI may be administered to the patient concurrently or sequentially. In other words, at least one compound of Formula (I) may be administered first, at least one STI may be administered first, or at least one compound of Formula (I) and at least one STI may be administered at the same time. Additionally, when more than one compound of Formula (I) and/or STI is used, the compounds may be administered in any order.

The present invention further provides a pharmaceutical composition for use in the treatment of a chronic viral infection in a patient comprising at least one compound of Formula (I), optionally, at least one chemotherapeutic drug, and, optionally, at least one antiviral agent, in a pharmaceutically acceptable carrier.

Also provided is a compound of Formula (I) for use in a method for treating a chronic viral infection in a patient by administering an effective amount of the above pharmaceutical composition.

At least one compound of Formula (I) and at least one chemotherapeutic agent may be administered to the patient concurrently or sequentially. In other words, at least one compound of Formula (I) may be administered first, at least one chemotherapeutic agent may be administered first, or at least one compound of Formula (I) and the at least one STI may be administered at the same time. Additionally, when more than one compound of Formula (I) and/or chemotherapeutic agent is used, the compounds may be administered in any order. Similarly, any antiviral agent or STI may also be administered at any point in comparison to the administration of the compound of Formula (I).

Chronic viral infections that may be treated using the present combinatorial treatment include, but are not limited to, diseases caused by: hepatitis C virus (HCV), human papilloma virus (HPV), cytomegalovirus (CMV), herpes simplex virus (HSV), Epstein-Barr virus (EBV), varicella zoster virus, coxsackie virus, human immunodeficiency virus (HIV). Notably, parasitic infections (e.g., malaria) may also be treated by the above methods wherein compounds known to treat the parasitic conditions are optionally added in place of the antiviral agents.

Suitable antiviral agents contemplated for use in combination with the compound of Formula (I) can comprise nucleoside and nucleotide reverse transcriptase inhibitors (NRTIs), non-nucleoside reverse transcriptase inhibitors (NNRTIs), protease inhibitors and other antiviral drugs.

Examples of suitable NRTIs include zidovudine (AZT); didanosine (ddl); zalcitabine (ddC); stavudine (d4T); lamivudine (3TC); abacavir (1592U89); adefovir dipivoxil [bis(POM)-PMEA]; lobucavir; BCH-I0652; emitricitabine [(-)-FTC]; beta-L-FD4 (also called beta-L-D4C and named beta-L-2',3'-dideoxy-5-fluoro-cytidene); DAPD, ((-)-beta-D-2,6-diamino-purine dioxolane); and lodenosine (FddA). Typical suitable NNRTIs include nevirapine (BI-RG-587); delaviradine (BHAP, U-90152); efavirenz (DMP-266); PNU-142721; AG-1549; MKC-442 (1-(ethoxy-methyl)-5-(1-methylethyl)-6-(phenylmethyl)-(2,4(1H,3H)-pyrimidinedione); and (+)-calanolide A (NSC-675451) and B. Typical suitable protease inhibitors include saquinavir (Ro 31-8959); ritonavir (ABT-538); indinavir (MK-639); nelfnavir (AG-1343); amprenavir (141W94); lasinavir; DMP-450; BMS-2322623; ABT-378; and AG-1549. Other antiviral agents include hydroxyurea, ribavirin, IL-2, IL-12, pentafuside and Yissum Project No.11607.

Pharmaceutical kits useful, for example, in the treatment or prevention of DGKα and DGKζ -associated diseases or disorders, and other diseases referred to herein may include one or more containers containing a pharmaceutical composition comprising a therapeutically effective amount of a compound of Formula (I). Such kits can further include, if desired, one or more of various conventional pharmaceutical kit components, such as, for example, containers with one or more pharmaceutically acceptable carriers, additional containers, as will be readily apparent to those skilled in the art. Instructions, either as inserts or as labels, indicating quantities of the components to be administered, guidelines for administration, and/or guidelines for mixing the components, can also be included in the kit.

The combination therapy is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single dosage form having a fixed ratio of each therapeutic agent or in multiple, single dosage forms for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Alternatively, for example, all therapeutic agents may be administered orally or all therapeutic agents may be administered by intravenous injection. Combination therapy also can embrace the administration of the therapeutic agents as described above in further combination with other biologically active ingredients and non-drug therapies (e.g., surgery or radiation treatment). Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

The invention also provides pharmaceutically acceptable compositions which comprise a therapeutically effective amount of one or more of the compounds of Formula (I), formulated together with one or more pharmaceutically acceptable carriers (additives) and/or diluents, and optionally, one or more additional therapeutic agents described above.

The compounds of this invention can be administered for any of the uses described herein by any suitable means, for example, orally, such as tablets, capsules (each of which includes sustained release or timed release formulations), pills, powders, granules, elixirs, tinctures, suspensions (including nanosuspensions, microsuspensions, spray-dried dispersions), syrups, and emulsions; sublingually; bucally; parenterally, such as by subcutaneous, intravenous, intramuscular, or intrasternal injection, or infusion techniques (*e.g*., as sterile injectable aqueous or non-aqueous solutions or suspensions); nasally, including administration to the nasal membranes, such as by inhalation spray; topically, such as in the form of a cream or ointment; or rectally such as in the form of suppositories. They can be administered alone, but generally will be administered with a pharmaceutical carrier selected on the basis of the chosen route of administration and standard pharmaceutical practice.

The phrase "pharmaceutically acceptable carrier" as used herein means a pharmaceutically acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation, including, *i.e*., adjuvant, excipient or vehicle, such as diluents, preserving agents, fillers, flow regulating agents, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms; and not injurious to the patient.

The term "pharmaceutical composition" means a composition comprising a compound of the invention in combination with at least one additional pharmaceutically acceptable carrier.

Pharmaceutically acceptable carriers are formulated according to a number of factors well within the purview of those of ordinary skill in the art. These include, without limitation: the type and nature of the active agent being formulated; the subject to which the agent-containing composition is to be administered; the intended route of administration of the composition; and the therapeutic indication being targeted. Pharmaceutically acceptable carriers include both aqueous and non-aqueous liquid media, as well as a variety of solid and semi-solid dosage forms. Such carriers can include a number of different ingredients and additives in addition to the active agent, such additional ingredients being included in the formulation for a variety of reasons, *e.g*., stabilization of the active agent, binders, etc., well known to those of ordinary skill in the art. Descriptions of suitable pharmaceutically acceptable carriers, and factors involved in their selection, are found in a variety of readily available sources such as, for example, Allen, L. V. Jr. et al. Remington: The Science and Practice of Pharmacy (2 Volumes), 22nd Edition (2012), Pharmaceutical Press.

The dosage regimen for the compounds of the present invention will, of course, vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired.

By way of general guidance, the daily oral dosage of each active ingredient, when used for the indicated effects, will range between about 0.001 to about 5000 mg per day, preferably between about 0.01 to about 1000 mg per day, and most preferably between about 0.1 to about 250 mg per day. Intravenously, the most preferred doses will range from about 0.01 to about 10 mg/kg/minute during a constant rate infusion. Compounds of this invention may be administered in a single daily dose, or the total daily dosage may be administered in divided doses of two, three, or four times daily.

The compounds are typically administered in admixture with suitable pharmaceutical diluents, excipients, or carriers (collectively referred to herein as pharmaceutical carriers) suitably selected with respect to the intended form of administration, *e.g*., oral tablets, capsules, elixirs, and syrups, and consistent with conventional pharmaceutical practices.

Dosage forms (pharmaceutical compositions) suitable for administration may contain from about 1 milligram to about 2000 milligrams of active ingredient per dosage unit. In these pharmaceutical compositions the active ingredient will ordinarily be present in an amount of about 0.1-95% by weight based on the total weight of the composition.

A typical capsule for oral administration may contain at least one of the compounds of the present invention (250 mg), lactose (75 mg), and magnesium stearate (15 mg). The mixture is passed through a 60 mesh sieve and packed into a No. L gelatin capsule.

A typical injectable preparation is produced by aseptically placing at least one of the compounds of the present invention (250 mg) into a vial, aseptically freeze-drying and sealing. For use, the contents of the vial are mixed with 2 mL of physiological saline, to produce an injectable preparation.

The present invention includes within its scope pharmaceutical compositions comprising, as an active ingredient, a therapeutically effective amount of at least one of the compounds of the present invention, alone or in combination with a pharmaceutical carrier. Optionally, compounds of the present invention can be used alone, in combination with other compounds of the invention, or in combination with one or more other therapeutic agent(s), *e.g*., an anticancer agent or other pharmaceutically active material.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion or metabolism of the particular compound being employed, the rate and extent of absorption, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the therapeutic effect and gradually increase the dosage until the effect is achieved.

In general, a suitable daily dose of a compound of the invention will be that amount of the compound which is the lowest dose effective to produce a therapeutic effect. Such an effective dose will generally depend upon the factors described above. Generally, oral, intravenous, intracerebroventricular and subcutaneous doses of the compounds of this invention for a patient will range from about 0.01 to about 50 mg per kilogram of body weight per day.

If desired, the effective daily dose of the active compound may be administered as two, three, four, five, six or more sub-doses administered separately at appropriate intervals throughout the day, optionally, in unit dosage forms. In certain aspects of the invention, dosing is one administration per day.

While it is possible for a compound of the present invention to be administered alone, it is preferable to administer the compound as a pharmaceutical formulation (composition).

The above other therapeutic agents, when employed in combination with the compounds of the present invention, may be used, for example, in those amounts indicated in the Physicians' Desk Reference (PDR) or as otherwise determined by one of ordinary skill in the art. In the methods of the present invention, such other therapeutic agent(s) may be administered prior to, simultaneously with, or following the administration of the inventive compounds.

### METHODS OF PREPARATION

The compounds of the present invention may be synthesized by many methods available to those skilled in the art of organic chemistry. General synthetic schemes for preparing compounds of the present invention are described below. These schemes are illustrative and are not meant to limit the possible techniques one skilled in the art may use to prepare the compounds disclosed herein. Different methods to prepare the compounds of the present invention will be evident to those skilled in the art. Examples of compounds of the present invention prepared by methods described in the general schemes are given in the Examples section set out hereinafter. Preparation of homochiral examples may be carried out by techniques known to one skilled in the art. For example, homochiral compounds may be prepared by separation of racemic products or diastereomers by chiral phase preparative HPLC. Alternatively, the example compounds may be prepared by methods known to give enantiomerically or diastereomerically enriched products.

The reactions and techniques described in this section are performed in solvents appropriate to the reagents and materials employed and are suitable for the transformations being effected. Also, in the description of the synthetic methods given below, it is to be understood that all proposed reaction conditions, including choice of solvent, reaction atmosphere, reaction temperature, duration of the experiment and work up procedures, are chosen to be the conditions standard for that reaction, which should be readily recognized by one skilled in the art. It is understood by one skilled in the art of organic synthesis that the functionality present on various portions of the molecule must be compatible with the reagents and reactions proposed. Such restrictions to the substituents that are compatible with the reaction conditions will be readily apparent to one skilled in the art, with alternatives required when incompatible substituents are present. This will sometimes require a judgment to modify the order of the synthetic steps or to select one particular process scheme over another in order to obtain a compound of the invention. It will also be recognized that another major consideration in the planning of any synthetic route in this field is the judicious choice of a protecting group used for protection of reactive functional groups present in the compounds described in this invention. An authoritative account describing the many alternatives to the trained practitioner is Wuts and Greene, *Greene's Protective Groups in Organic* Synthesis, Fourth Edition, Wiley and Sons (2007).

Methodologies that can be employed in the syntheses of intermediates useful in the preparation of examples of the current invention are shown in the scheme below.

Benzooxazine-2,4(1H)-diones of the type shown can be treated with strong base and a methylating reagent such as methyl iodide to afford 1-methyl-2H-benzo[d][1,3]oxazine-2,4(1H)-diones. These can be treated with activated acetates, for example nitro acetates, malonates and cyano acetates to generate 1-methyl-3-nitroquinoline-2,4(1H,3H)-diones, 1-methyl-3-carboxyethylquinoline-2,4(1H,3H)-diones and 1-methyl-3-cyanoquinoline-2,4(1H,3H)-diones, respectively. The 3-ester derivatives can be further transformed by basic hydrolysis and acidic decarboxylation to provide 6-fluoro-4-hydroxy-1-methylquinolin-2(1H)-one.

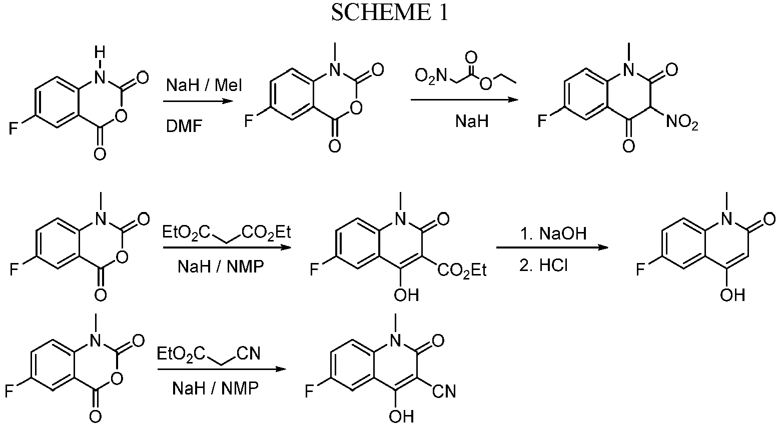

Alternatively, quinoline diones can be prepared from substituted 2-aminobenzoic acids. For example, methyl 2-amino-5-bromobenzoate can be transformed into 4-hydroxy-1-methyl-2-oxo-1,2-dihydroquinoline-6-carbonitrile in four steps.

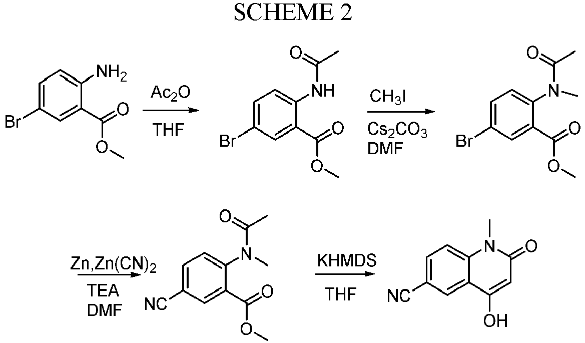

Such quinoline diones can in turn be converted to the related 4-chloro or 4-triflate derivatives that can be reacted with a diversity of functionalized piperazines to afford protected compounds such as tert-butyl 4-(6-fluoro-1-methyl-2-oxo-1,2-dihydroquinolin-4-yl)piperazine-1-carboxylate or examples of the current invention.

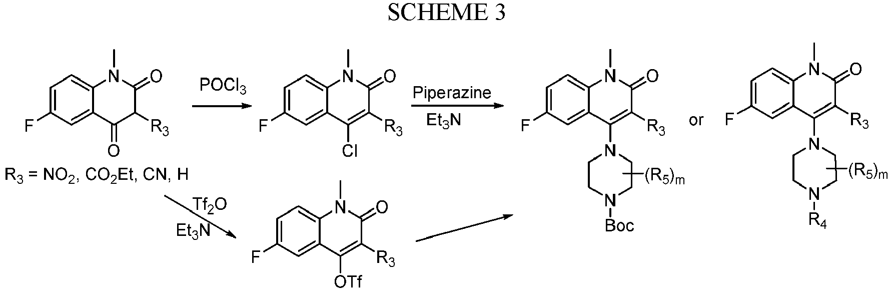

Protected piperazines such as tert-butyl 4-(6-fluoro-1-methyl-2-oxo-1,2-dihydroquinolin-4-yl)piperazine-1-carboxylate can be further transformed by deprotection of the piperazine with, for example, TFA in dichloromethane and reaction with an electrophile such as 3-fluoro benzyl bromide to provide 6-fluoro-4-{4-[(3-fluorophenyl)methyl]piperazin-1-yl}-1-methyl-3-nitro-1,2-dihydroquinolin-2-one.

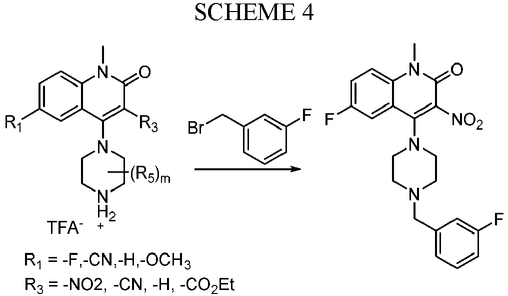

Compounds of the invention can also be prepared by the reaction of quinoline diones or amino quinolones with electrophiles such as phosphorous oxychloride in DMF, nitric acid in acetic acid or N-bromosuccinimide in DMF to give, for example, 4-(4-benzhydrylpiperazin-1-yl)-1-methyl-2-oxo-1,2-dihydroquinoline-3-carbaldehyde, 4-hydroxy-1-methyl-3-nitroquinolin-2(1H)-one and tert-butyl 4-(3-bromo-6-fluoro-1-methyl-2-oxo-1,2-dihydroquinolin-4-yl)piperazine-1-carboxylate, respectively. These compounds can be further transformed as necessary to give compounds of the invention. Examples of the invention can be prepared from 4-hydroxy-1-methyl-3-nitroquinolin-2(1H)-one by following the steps outlined in Scheme 3.

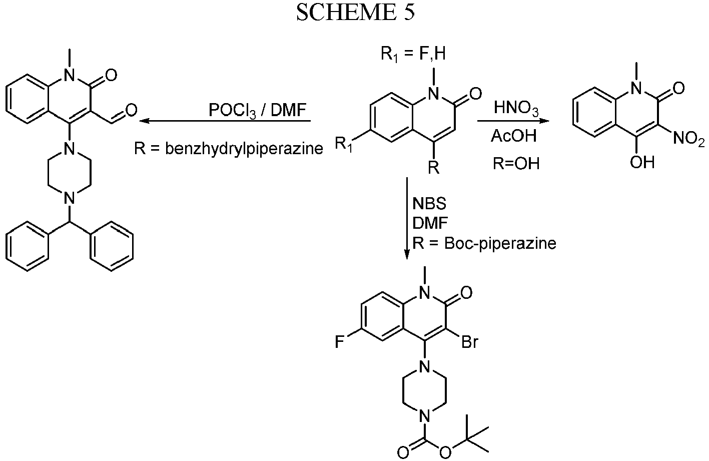

The tert-butyl 4-(3-bromo-6-fluoro-1-methyl-2-oxo-11,2-dihydroquinolin-4-yl)piperazine-1-carboxylate can be reacted with aryl and heteroaryl boronic acids or stanines in the presence of a suitable catalyst to provide, for example, tert-butyl 4-(6-fluoro-1-methyl-2-oxo-3-(pyridin-4-yl)-1,2-dihydroquinolin-4-yl)piperazine-1-carboxylate. These examples can be further transformed by deprotection and alkylation of the piperazine as shown in Scheme 3.

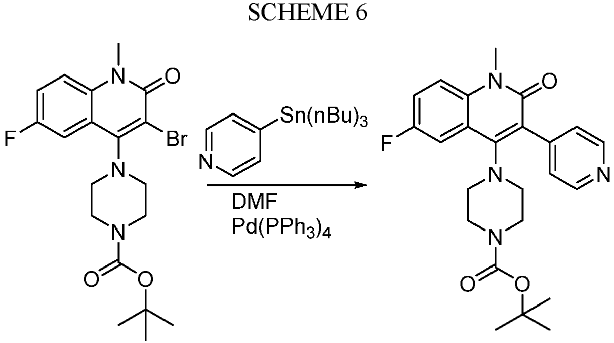

### EXAMPLES

The following examples illustrate the particular and preferred embodiments of the present invention and do not limit the scope of the present invention. Chemical abbreviations and symbols as well as scientific abbreviations and symbols have their usual and customary meanings unless otherwise specified. Additional abbreviations employed in the Examples and elsewhere in this application are defined above. Common intermediates are generally useful for the preparation of more than one Example and are identified sequentially (e.g., Intermediate 1, Intermediate 2, etc.) and are abbreviated as Int. 1 or I1, Int. 2 or I2, etc. Compounds of the Examples are identified by the example and step in which they were prepared (e.g., "1-A" denotes the Example 1, step A), or by the example only where the compound is the title compound of the example (for example, "1" denotes the title compound of Example 1). In some instances alternate preparations of intermediates or examples are described. Frequently chemists skilled in the art of synthesis may devise alternative preparations which may be desirable based on one or more considerations such as shorter reaction time, less expensive starting materials, ease of operation or isolation, improved yield, amenable to catalysis, avoidance of toxic reagents, accessibility of specialized instrumentation, and decreased number of linear steps, etc. The intent of describing alternative preparations is to further enable the preparation of the examples of this invention. In some instances some functional groups in the outlined examples and claims may be replaced by well-known bioisosteric replacements known in the art, for example, replacement of a carboxylic acid group with a tetrazole or a phosphate moiety. ¹H NMR data collected in deuterated dimethyl sulfoxide used water suppression in the data processing. The reported spectra are uncorrected for the effects of water suppression. Protons adjacent to the water suppression frequency of 3.35 ppm exhibit diminished signal intensity.

### ABBREVIATIONS

- Ac: acetyl
- AcOH: acetic acid
- AczO: acetic anhydride
- anhyd.: anhydrous
- aq.: aqueous
- Boc: *tert*-butoxycarbonyl
- BOP: benzotriazol-1-yloxytris-(dimethylamino)-phosphonium hexafluorophosphate
- Bu: butyl
- DCM: dichloromethane
- DEA: diethylamine
- DIEA or DIPEA: diisopropylethylamine
- DMF: dimethylformamide
- DMSO: dimethyl sulfoxide
- dppf: 1,1'-bis(diphenylphosphino)ferrocene
- EDTA: ethylenediaminetetraacetic acid
- Et: ethyl
- Et₃N: triethyl amine
- EtOAc: ethyl acetate
- EtOH: ethanol
- h, hours or hrs: hour(s)
- HCl: hydrochloric acid
- HPLC: high pressure liquid chromatography
- LC: liquid chromatography
- LCMS: liquid chromatography- mass spectrometry
- M: molar
- M⁺¹: (M+H)⁺
- Me: methyl
- MeI: methyl iodide
- MeOH: methanol
- Mesyl-Cl: methanesulfonyl chloride
- MHz: megahertz
- mins: minute(s)
- mM: millimolar
- MS: mass spectrometry
- n or N: normal
- NBS: *N*-bromosuccinimide
- NH₄OAc: ammonium acetate
- KHMDS: potassium bis(trimethylsilyl)amide
- nM: nanomolar
- NMP: *N*-methylpyrrolidinone
- Pd₂(dba)₃: tris-(dibenzylideneacetone)dipalladium
- pet ether: petroleum ether
- Ph: phenyl
- POCl₃: phosphorous oxychloride
- rt or Ret time: retention time
- sat.: saturated
- TEA: triethylamine
- Tetrakis: tetrakis(triphenylphosphine)palladium(0)
- TFA: trifluoroacetic acid
- Tf₂O: trifluoromethylsulfonic anhydride
- THF: tetrahydrofuran

### INTERMEDIATE 1

### 6-fluoro-1-methyl-2H-benzo[d][1,3]oxazine-2,4(1H)-dione

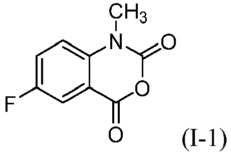

To a solution of 6-fluoro-1H-benzo[d][1,3]oxazine-2,4-dione (10.2 g, 56.3 mmol) in DMF (60 mL), 60% dispersion of sodium hydride (2.70 g, 67.6 mmol) in mineral oil was added in portions at 0 °C. The reaction mixture was warmed to room temperature and stirred for 30 min. Then methyl iodide (4.23 mL, 67.6 mmol) was added dropwise. The reaction mixture was stirred at room temperature overnight. The reaction was quenched with water. Diethyl ether was added to the reaction mixture. A light yellow solid was collected as 6-fluoro-1-methyl-1H-benzo[d] [1,3]oxazine-2,4-dione (9.55 g, 48.9 mmol, 87 % yield). LC/MS LC/MS Column: Phenomenex LUNA C18, 2.0 X 50 mm, 3-mm particles; Mobile Phase A: 5:95 acetonitrile:water with 10 mM ammonium acetate; Mobile Phase B: 95:5 acetonitrile:water with 10 mM ammonium acetate; Temperature: 40 °C; Gradient: 0%B, 0-100% B over 4 minutes, then a 1 minute hold at 100% B; Flow: 0.8 mL/min; Detection: UV at 220 nm. Retention time 1.982 min, 196.0 (M+H⁺). ¹H NMR (400 MHz, DMSO-d₆) δ 7.82-7.73 (m, 2H), 7.56-7.46 (m, 1H), 3.47 (s, 3H).

### INTERMEDIATE 2

### 6-fluoro-1-methyl-3-nitroquinoline-2,4(1H,3H)-dione

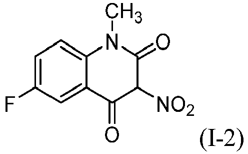

To a solution of ethyl 2-nitroacetate (3.38 g, 25.4 mmol) in NMP (15 mL) in a round-bottomed flask at 0 °C, 60% dispersion of sodium hydride (1.107 g, 27.7 mmol) in mineral oil was added in portions. The reaction mixture was stirred 5 minutes at 0 °C, and then for 15 minutes at room temperature. Next, 6-fluoro-1-methyl-1H-benzo[d][1,3]oxazine-2,4-dione (4.5 g, 23.06 mmol) was added and the reaction mixture was heated at 120 °C for 2 hr. LC/MS shown completion of reaction. The reaction was quenched with ice-water. The reaction mixture was acidified with 1 N HCl solution. Ethyl ether was added and a yellow solid was collected as 6-fluoro-1-methyl-3-nitroquinoline-2,4(1H,3H)-dione (2.69 g, 11.29 mmol, 49.0 % yield). LC/MS LC/MS Column: Phenomenex LUNA C18, 2.0 X 50 mm, 3-mm particles; Mobile Phase A: 5:95 acetonitrile:water with 10 mM ammonium acetate; Mobile Phase B: 95:5 acetonitrile:water with 10 mM ammonium acetate; Temperature: 40 °C; Gradient: 0%B, 0-100% B over 4 minutes, then a 1 minute hold at 100% B; Flow: 0.8 mL/min; Detection: UV at 220 nm. Retention time 1.488 min, 237.1 (M-H⁻). ¹H NMR (400 MHz, DMSO-d₆) δ 7.85 (dd, *J*=9.2, 2.6 Hz, 1H), 7.72-7.47 (m, 2H), 6.73 (s, br, 1H), 3.58 (s, 3H).

### INTERMEDIATE 3

### 4-chloro-6-fluoro-1-methyl-3-nitroquinolin-2(1H)-one

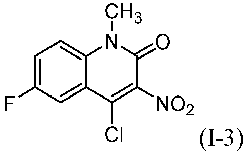

In a sealed tube, 6-fluoro-1-methyl-3-nitroquinoline-2,4(1H,3H)-dione (1.4 g, 5.88 mmol) and phosphorus oxychloride (10 mL, 107 mmol) were added. The reaction mixture heated at 95 °C for 4 hr. The reaction mixture was concentrated and the residue poured onto ice-water and neutralized with saturated NaHCOs solution. The mixture was extracted using dichloromethane (2 x 40 mL) and the organic layers were combined, dried (MgSO₄) and concentrated to give 4-chloro-6-fluoro-1-methyl-3-nitroquinolin-2(1H)-one as an orange solid (1.28 g, 4.99 mmol, 85 % yield). LC/MS Column: Phenomenex LUNA C18, 2.0 X 50 mm, 3-mm particles; Mobile Phase A: 5:95 acetonitrile:water with 10 mM ammonium acetate; Mobile Phase B: 95:5 acetonitrile:water with 10 mM ammonium acetate; Temperature: 40 °C; Gradient: 0%B, 0-100% B over 4 minutes, then a 1 minute hold at 100% B; Flow: 0.8 mL/min; Detection: UV at 220 nm. Retention time 2.648 min, 257.0 (M+H⁺). ¹H NMR (500 MHz, DMSO-d₆) δ 7.93 (d, *J*=9.5 Hz, 1H), 7.86-7.79 (m, 2H), 3.72 (s, 3H).

### INTERMEDIATE 4

### tert-butyl 4-(6-fluoro-1-methyl-3-nitro-2-oxo-1,2-dihydroquinolin-4-yl)piperazine-1 - carboxylate

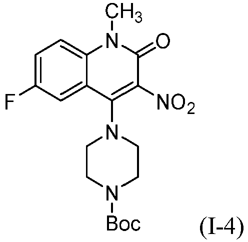

To a solution of 4-chloro-6-fluoro-1-methyl-3-nitroquinolin-2(1H)-one (600 mg, 2.338 mmol) in DMF (8 mL), tert-butyl piperazine-1-carboxylate (435 mg, 2.338 mmol) and triethylamine(0.978 mL, 7.01 mmol) were added. The reaction mixture was stirred at room temperature overnight. LC/MS shown completion of reaction. The reaction was quenched with water. A yellow solid was collected as the product tert-butyl 4-(6-fluoro-1-methyl-3-nitro-2-oxo-1,2-dihydroquinolin-4-yl)piperazine-1-carboxylate (765 mg, 1.882 mmol, 81% yield). LC/MS LC/MS Column: Phenomenex LUNA C18, 2.0 X 50 mm, 3-mm particles; Mobile Phase A: 5:95 acetonitrile:water with 10 mM ammonium acetate; Mobile Phase B: 95:5 acetonitrile:water with 10 mM ammonium acetate; Temperature: 40 °C; Gradient: 0%B, 0-100% B over 4 minutes, then a 1 minute hold at 100% B; Flow: 0.8 mL/min; Detection: UV at 220 nm. Retention time 3.0 min, 407.1 (M+H⁺). ¹H NMR (500 MHz, DMSO-d₆) δ 7.79-7.63 (m, 3H), 3.65 (s, 3H), 3.58 (br. s., 4H), 3.07 (br. s., 4H), 1.43 (s, 9H).

### INTERMEDIATE 5

### 6-fluoro-1-methyl-3-nitro-4-(piperazin-1-yl)quinolin-2(1H)-one, TFA

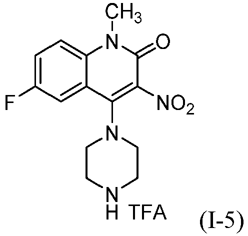

To a solution of tert-butyl 4-(6-fluoro-1-methyl-3-nitro-2-oxo-1,2-dihydroquinolin-4-yl)piperazine-1-carboxylate (2.1 g, 5.17 mmol) in CH₂Cl₂ (10 mL), TFA (5 mL, 64.9 mmol) was added. The reaction mixture was stirred at room temperature overnight. LC/MS shown completion of the reaction. The reaction mixture was concentrated and ethyl acetate was added. A yellow solid was obtained as the product 6-fluoro-1-methyl-3-nitro-4-(piperazin-1-yl)quinolin-2(1H)-one, TFA (1.72 g, 4.09 mmol, 79 % yield). LC/MS Column: Phenomenex LUNA C18, 2.0 X 50 mm, 3-mm particles; Mobile Phase A: 10:90 methanol:water with 0.1% TFA; Mobile Phase B: 90: 10 methanol:water with 0.1% TFA; Gradient: 0-100 % B over 4 minutes, then a 1 minute hold at 100% B; Flow: 0.8 mL/min; Detection: UV at 220 nm. Retention time 1.62 min, 307.2 (M+H⁺). ¹H NMR (400 MHz, DMSO-d₆) δ 7.73-7.67 (m, 2H), 7.64 (d, *J*=10.0 Hz, 1H), 3.65 (s, 3H), 3.11-3.03 (m, 4H), 2.93 (br. s., 4H).

### EXAMPLE 1

### 6-fluoro-4-{4-[(3-fluorophenyl)methyl]piperazin-1-yl}-1-methyl-3-nitro-1,2-dihydroquinolin-2-one

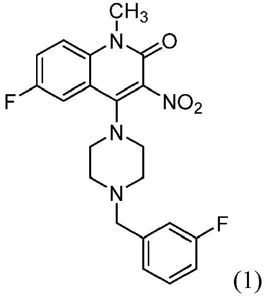

A solution of 6-fluoro-1-methyl-3-nitro-4-(piperazin-1-yl)quinolin-2(1H)-one, TFA (340 mg, 816 µmol) and DIPEA (646 mL, 3.6 mmol) in DMF (17.0 mL) was prepared. To 3-fluoro benzyl bromide weighed into a 16 × 100 mm threaded vial was added 500 µL of the 6-fluoro-1-methyl-3-nitro-4-(piperazin-1-yl)quinolin-2(1H)-one, TFA/ DIPEA solution. The vial was capped and allowed to shake at room temperature until the reaction was complete as measured by LCMS analysis (approximately 24 hours). A 500 µL aliquot of DMF was added to the vial and the crude material was purified via preparative LC/MS with the following conditions: Column: XBridge C18, 19 × 200 mm, 5-µm particles; Mobile Phase A: 5:95 acetonitrile: water with 10-mM ammonium acetate; Mobile Phase B: 95:5 acetonitrile: water with 10-mM ammonium acetate; Gradient: 45-85% B over 15 minutes, then a 5-minute hold at 100% B; Flow: 20 mL/min. Fractions containing the product were combined and dried via centrifugal evaporation. LC\MS conditions Column: Phenomenex LUNA C18, 2.0 × 50 mm, 3-mm particles; Mobile Phase A: 10:90 methanol:water with 0.1% TFA; Mobile Phase B: 90:10 methanol:water with0.1% TFA; Gradient: 0-100 % B over 4 minutes, then a 1 minute hold at 100% B; Flow: 0.8 mL/min; Detection: UV at 220 nm. Observed MS Ion 415.1, retention time 1.23 minutes.

### INTERMEDIATE 6

### Ethyl 6-fluoro-1-methyl-2,4-dioxo-1,2,3,4-tetrahydroquinoline-3-carboxylate

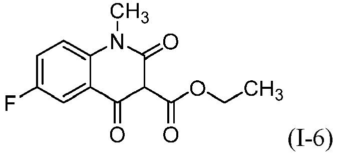

To a solution of diethyl malonate (0.903 g, 5.64 mmol) in NMP (10 mL) in a round-bottomed flask at 0 °C, a 60% dispersion of sodium hydride (0.246 g, 6.15 mmol) in mineral oil was added in portions. The reaction mixture was stirred 5 minutes at 0 °C, then 15 minutes at room temperature. Next, 6-fluoro-1-methyl-1H-benzo[d][1,3]oxazine-2,4-dione (1 g, 5.12 mmol) was added and the reaction mixture was heated at 120 °C for 2 hr. LC/MS shown completion of reaction. The reaction was quenched with ice-water and acidified with 1 N HCl solution. Ethyl ether was added and a beige solid was collected as the product ethyl 6-fluoro-1-methyl-2,4-dioxo-1,2,3,4-tetrahydroquinoline-3-carboxylate (245 mg, 0.924 mmol, 18.03 % yield). LC/MS Column: Phenomenex LUNA C18, 2.0 × 50 mm, 3-mm particles; Mobile Phase A: 5:95 acetonitrile:water with 10 mM ammonium acetate; Mobile Phase B: 95:5 acetonitrile:water with 10 mM ammonium acetate; Temperature: 40 °C; Gradient: 0%B, 0-100% B over 4 minutes, then a 1 minute hold at 100% B; Flow: 0.8 mL/min; Detection: UV at 220 nm. Retention time 1.660 min, 264.1 (M-H⁻). ¹H NMR (400 MHz, DMSO-d₆) δ 7.77 (dd, *J*=9.3, 2.9 Hz, 1H), 7.68-7.54 (m, 2H), 4.32 (q, *J*=7.1 Hz, 2H), 3.56 (s, 3H), 1.30 (t, *J*=7.1 Hz, 3H).

### INTERMEDIATE 7

### 6-fluoro-4-hydroxy-1-methylquinolin-2(1H)-one

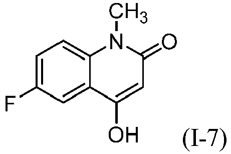

A suspension of ethyl 6-fluoro-4-hydroxy-1-methyl-2-oxo-1,2-dihydroquinoline-3-carboxylate (6.5 g, 24.51 mmol) in 2 M sodium hydroxide (150 mL, 300 mmol) was heated to 100 °C for 12 hours. The reaction mixture was cooled to room temperature and 50 mL of 6 N HCl was added dropwise with stirring. After approximately 30 min the solids were collected by filtration. The filtrand was washed with water and then dried under vacuum overnight. The solid was further dried by suspending in toluene and evaporation to dryness (2×) to give the product as an off-white solid (4.93 g, 24.51 mmol, 100 % yield). m/z; 194 (M+H)⁺.

### INTERMEDIATE 8

### 4-chloro-6-fluoro-1-methyl quinolin-2(1H)-one

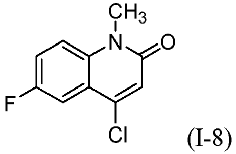

In a dried vial equipped with a magnetic stirrer, 6-fluoro-1-methylquinoline-2,4(1H,3H)-dione (1000 mg, 5.18 mmol) was added followed by the addition of phosphorus oxychloride (5790 µL, 62.1 mmol). The mixture was maintained under an atmosphere of nitrogen and was placed in an oil bath at 100 °C for a period of 1 h. The reaction mixture was removed from the oil bath and allowed to cool to room temperature before being evaporated under vacuum. The residue was dissolved/suspended in acetonitrile and placed in an ice-bath. Ice crystals were added and the reaction mixture was allowed to warm to room temperature overnight. The reaction mixture was concentrated in vacuo to remove acetonitrile. The resulting suspension was filtered and the product collected by filtration. The tan solid was dried under vacuum at 50 °C to give the title compound as a reddish-tan colored solid (974 mg). ¹H NMR (400 MHz, DMSO-d₆) δ 7.82-7.58 (m, 3H), 7.05 (s, 1H), 3.64 (s, 3H).

### INTERMEDIATE 9

### tert-butyl 4-(6-fluoro-1-methyl-2-oxo-1,2-dihydroquinolin-4-yl)piperazine-1-carboxylate

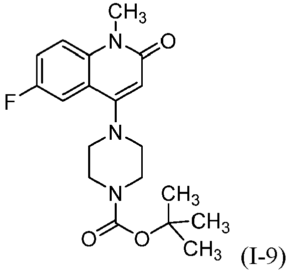

4-Chloro-6-fluoro-1-methylquinolin-2(1H)-one (940 mg, 4.44 mmol) and tert-butyl piperazine-1-carboxylate (910 mg, 4.89 mmol) were dissolved in a mixture of DMA (20 mL) and t-BuOH (5.00 mL). Cesium carbonate (4342 mg, 13.33 mmol) was added with (2-dicyclohexylphosphino-2',6'-diisopropoxy-1,1'-biphenyl)[2-(2'-amino-1,1'-biphenyl)]palladium(ii) methanesulfonate (186 mg, 0.222 mmol). The reaction mixture was degassed and then flushed with nitrogen and then heated at 85 °C overnight. The reaction mixture was diluted with 0.1 M EDTA solution and EtOAc was added. The mixture was filtered through Celite to remove colloidal, precipitated palladium. The organic layer was then separated, and the aqueous mixture re-extracted with EtOAc (2x). The combined extracts were washed once with brine, then dried over MgSO₄, filtered and concentrated in vacuo to give the crude product as a dark purple colored, viscous oil. The residue was dissolved in DCM and adsorbed onto silica gel. The material was then fractionated using flash chromatography on silica gel eluting with 90-100% EtOAc in Hexanes. Homogeneous fractions were combined to give the product as a colorless oil, 960 mg. ¹H NMR (400 MHz, DMSO-d₆) δ 7.63-7.44 (m, 3H), 6.13 (s, 1H), 3.57 (s, 7H), 2.99 (br t, J=4.6 Hz, 4H), 1.44 (s, 9H).

### INTERMEDIATE 10

### tert-butyl 4-(3-bromo-6-fluoro-1-methyl-2-oxo-1,2-dihydroquinolin-4-yl)piperazine-1-carboxylate

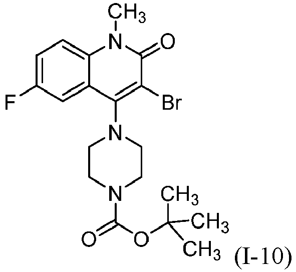

Tert-butyl 4-(6-fluoro-1-methyl-2-oxo-1,2-dihydroquinolin-4-yl)piperazine-1-carboxylate (98 mg, 0.271 mmol) was dissolved in DMF (2 mL) in a round bottomed flask maintained under an atmosphere of nitrogen. The solution was cooled to 0 °C and N-bromosuccinimide (43.9 mg, 0.247 mmol) was added in a single portion. The mixture was then removed from the ice-bath, and allowed to warm slightly over 15 min. The solvent was then removed under high vacuum and the resultant residue was dissolved in DCM, adsorbed onto SiO₂ and then chromatographed using 40% EtOAc in hexanes. Homogeneous fractions were combined and evaporated in vacuo to give the product as a colorless film (98 mg, 88% yield). ¹H NMR (400 MHz, chloroform-d) δ 7.80-7.73 (m, 1H), 7.39-7.30 (m, 2H), 3.78 (s, 3H), 1.51 (s, 9H). DCM signal was observed at 5.3 ppm.

### INTERMEDIATE 11

### tert-butyl 4-(6-fluoro-1-methyl-2-oxo-3-(pyridin-4-yl)-1,2-dihydroquinolin-4-yl) piperazine-1-carboxylate

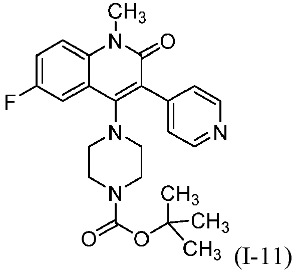

Tert-butyl 4-(3-bromo-6-fluoro-1-methyl-2-oxo-1,2-dihydroquinolin-4-yl) piperazine-1-carboxylate (562 mg, 1.276 mmol) and 4-(tributylstannyl)pyridine (0.392 mL, 1.276 mmol) were dissolved in anhydrous DMF (10 mL). The reaction flask was evacuated and flushed with nitrogen three times. Tetrakis (147 mg, 0.128 mmol) was then added, and the evacuation/flush procedure was repeated twice. The reaction mixture was heated at 100 °C under nitrogen in a sealed tube for 48 h. The reaction mixture was then fractionated using flash chromatography using 5% MeOH in DCM. Homogeneous fractions were combined and evaporated in vacuo to give the product as a yellow colored oil, 928 mg.

### INTERMEDIATE 12

### 6-fluoro-1-methyl-4-(piperazin-1-yl)-3-(pyridin-4-yl)quinolin-2(1H)-one

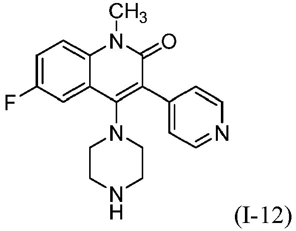

Trifluoroacetic acid (8.15 mL, 106 mmol) was added to a solution of tert-butyl 4-(6-fluoro-1-methyl-2-oxo-3-(pyridin-4-yl)-1,2-dihydroquinolin-4-yl)piperazine-1-carboxylate (928 mg, 2.116 mmol) in DCM (8 mL). The reaction mixture was stirred at room temperature for a period of 1 h. The mixture was then diluted with water and the strongly acidic aqueous layer separated from the dichloromethane solution. The organic layer was re-extracted with additional water (the aqueous extract remained highly acidic). The combined aqueous extracts were combined and then basified (pH-14) by the addition of solid KOH. The resultant mixture was extracted using DCM (3x) and the combined extracts dried over MgSO₄, filtered and evaporated in vacuo to give an oil that solidified on standing overnight (243 mg).

### EXAMPLE 2

### 6-fluoro-4-{4-[(3-fluoro-5-methylphenyl)methyl]piperazin-1-yl}-1-methyl-3-(pyridin-4-yl)-1,2-dihydroquinolin-2-one

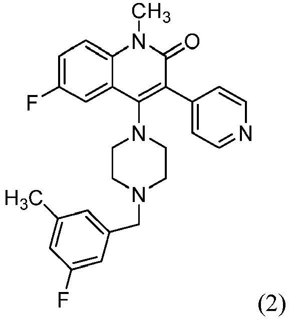

To a solution of 6-fluoro-1-methyl-4-(piperazin-1-yl)-3-(pyridin-4-yl)quinolin-2(1H)-one (22 mg, 0.065 mmol) in DMF (1.5 mL), 4-formyl-3-hydroxybenzonitrile (14.35 mg, 0.098 mmol) was added. The reaction mixture was stirred at room temperature for 1 hr after which sodium cyanoborohydride (8.18 mg, 0.130 mmol) was added. The reaction mixture was stirred at room temperature overnight. The reaction product was purified via preparative LC/MS with the following conditions: Column: XBridge C18, 19 × 200 mm, 5-µm particles; Mobile Phase A: 5:95 acetonitrile: water with 10-mM ammonium acetate; Mobile Phase B: 95:5 acetonitrile: water with 10-mM ammonium acetate; Gradient: 35-75% B over 20 minutes, then a 5-minute hold at 100% B; Flow: 20 mL/min. Fractions containing the product were combined and dried via centrifugal evaporation. The yield of the product was 13.5 mg, and its estimated purity by LCMS analysis was 100%. Analytical LC/MS was used to determine the final purity. Injection 1 conditions: Column: Waters XBridge C18, 2.1 mm × 50 mm, 1.7 mm particles; Mobile Phase A: 5:95 acetonitrile:water with 0.1 % trifluoroacetic acid; Mobile Phase B: 95:5 acetonitrile:water with 0.1 % trifluoroacetic acid; Temperature: 50 °C; Gradient: 0 %B to 100 %B over 3 min, then a 0.75 min hold at 100 %B; Flow: 1 mL/min; Detection: MS and UV (220 nm). Injection 1 results: Purity: 100.0 %; Observed Mass: 461.22; Retention Time: 1.48 min. Injection 2 conditions: Column: Waters XBridge C18, 2.1 mm × 50 mm, 1.7 µm particles; Mobile Phase A: 5:95 acetonitrile:water with 10 mM ammonium acetate; Mobile Phase B: 95:5 acetonitrile:water with 10 mM ammonium acetate; Temperature: 50 °C; Gradient: 0 %B to 100 %B over 3 min, then a 0.75 min hold at 100 %B; Flow: 1 mL/min; Detection: MS and UV (220 nm). Injection 2 results: Purity: 100.0 %; Observed Mass: 461.2; Retention Time: 2. 26 min. ¹H NMR (500 MHz, DMSO-d₆) δ 8.74 (d, J=6.6 Hz, 2H), 7.79 (d, J=6.6 Hz, 2H), 7.70-7.63 (m, 2H), 7.62-7.53 (m, 1H), 7.00-6.92 (m, 1H), 6.89 (br d, J=8.8 Hz, 2H), 3.63 (s, 3H), 3.49 (s, 2H), 2.82 (br s, 4H), 2.50-2.44 (m, 4H), 2.31 (s, 3H).

### INTERMEDIATE 13

### 4-(piperazin-1-ylmethyl)-1H-indole, 2 TFA

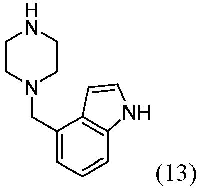

To a solution of tert-butyl piperazine-1-carboxylate (481 mg, 2.58 mmol) in DMF (1.5 mL), 1H-indole-4-carbaldehyde (250 mg, 1.722 mmol) was added. The reaction mixture was stirred at room temperature for 1 hr. Then sodium cyanoborohydride (325 mg, 5.17 mmol) was added and the reaction mixture was stirred at room temperature for 6 days. The reaction mixture was then added to methanol, filtered and the filtrate was purified by Prep. HPLC using CH₃CN-H₂O-TFA buffer system. Fractions were collected and concentrated under speed-vac overnight. LC/MS showed that most of the material was without the boc group. The material was dissolved in dichloromethane (5 mL) and 1 mL of TFA was added. The reaction mixture was stirred at room temperature for 3 hr and concentrated to afford 4-(piperazin-1-ylmethyl)-1H-indole, 2 TFA (420 mg, 0.947 mmol, 55.0 % yield) as a white solid. LC/MS Column: Phenomenex LUNA C18, 2.0 × 50 mm, 3-mm particles; Mobile Phase A: 5:95 acetonitrile:water with 10 mM ammonium acetate; Mobile Phase B: 95:5 acetonitrile:water with 10 mM ammonium acetate; Temperature: 40 °C; Gradient: 0%B, 0-100% B over 4 minutes, then a 1 minute hold at 100% B; Flow: 0.8 mL/min; Detection: UV at 220 nm. Retention time1.577 min, 216.1 (MH⁺).

### EXAMPLE 3

### 4-(4-((1H-indol-4-yl)methyl)piperazin-1-yl)-6-fluoro-1-methyl-3-nitroquinolin-2(1H)-one

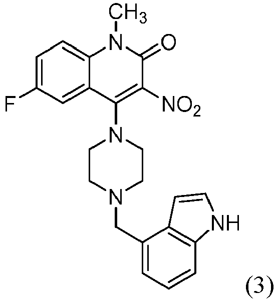

To a solution of 4-chloro-6-fluoro-1-methyl-3-nitroquinolin-2(1H)-one (150 mg, 0.585 mmol) in DMF (5 mL), 4-(piperazin-1-ylmethyl)-1H-indole, 2 TFA (285 mg, 0.643 mmol) and triethylamine (0.326 mL, 2.338 mmol) were added. The reaction mixture was stirred at room temperature over the weekend. LC/MS shown completion of the reaction. The crude product was purified by Prep. HPLC using CH₃CN-H₂O-TFA buffer system. Fractions were collected and concentrated under speed-vac overnight. The product was purified again by Prep. HPLC using CH₃CN-H₂O-ammonium acetate buffer system. Fractions were collected and concentrated under speed-vac overnight. A yellow solid was obtained as final product 4-(4-((1H-indol-4-yl)methyl)piperazin-1-yl)-6-fluoro-1-methyl-3-nitroquinolin-2(1H)-one (180 mg, 0.413 mmol, 70.7 % yield). LC/MS Column: Phenomenex LUNA C18, 2.0 × 50mm, 3-mm particles; Mobile Phase A: 5:95 acetonitrile:water with 10 mM ammonium acetate; Mobile Phase B: 95:5 acetonitrile:water with 10 mM ammonium acetate; Temperature: 40 °C; Gradient: 0%B, 0-100% B over 4 minutes, then a 1 minute hold at 100% B; Flow: 0.8 mL/min; Detection: UV at 220 nm. Retention time 3.1 min, 436.1 (MH⁺).

### EXAMPLE 4

### 4-{4-[(1-ethyl-1H-indol-4-yl)methyl]piperazin-1-yl}-6-fluoro-1-methyl-3-nitro-1,2-dihydroquinolin-2-one

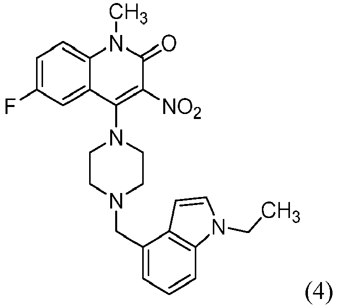

To a solution of 4-(4-((1H-indol-4-yl)methyl)piperazin-1-yl)-6-fluoro-1-methyl-3-nitroquinolin-2(1H)-one (15 mg, 0.034 mmol) in DMF (1.5 mL), a 60% dispersion of sodium hydride (2.76 mg, 0.069 mmol) in mineral oil was added. The reaction mixture was stirred at room temperature for 15 min. Then iodoethane (10.75 mg, 0.069 mmol) was added. The reaction mixture was stirred at room temperature overnight. LC/MS shown completion of reaction. The reaction was quenched with methanol. The reaction mixture was filtered and the filtrate was purified via preparative LC/MS with the following conditions: Column: XBridge C18, 19 × 200 mm, 5-µm particles; Mobile Phase A: 5:95 acetonitrile: water with 10-mM ammonium acetate; Mobile Phase B: 95:5 acetonitrile: water with 10-mM ammonium acetate; Gradient: 20-100% B over 15 minutes, then a 5-minute hold at 100% B; Flow: 20 mL/min. Fractions containing the product were combined and dried via centrifugal evaporation. LC\MS conditions Column: Phenomenex LUNA C18, 2.0 × 50 mm, 3-mm particles; Mobile Phase A: 5:95 acetonitrile:water with 10 mM ammonium acetate; Mobile Phase B: 95:5 acetonitrile:water with 10 mM ammonium acetate; Temperature: 40 °C; Gradient: 0%B, 0-100% B over 4 minutes, then a 1 minute hold at 100% B; Flow: 0.8 mL/min; Detection: UV at 220 nm: Observed MS Ion 464.1, retention time 2.28 minutes. ¹H NMR (500 MHz, DMSO-d₆) δ 7.80-7.65 (m, 2H), 7.63-7.49 (m, 1H), 7.45-7.32 (m, 2H), 7.17-7.06 (m, 1H), 7.03-6.94 (m, 1H), 6.73-6.51 (m, 1H), 4.20 (q, *J*=9.3 Hz, 2H), 3.83 (s, 2H), 3.64 (s, 3H), 3.16-3.09 (m, 4H), 2.71-2.59 (m, 4H), 1.37 (t, *J*=9.3 Hz, 3H).

### EXAMPLE 5

### 4-(4-benzyl-3-oxopiperazin-1-yl)-6-fluoro-1-methyl-3-nitro-1,2-dihydroquinolin-2-one

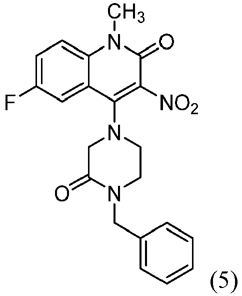

4-Chloro-6-fluoro-1-methyl-3-nitroquinolin-2(1H)-one (25 mg, 0.097 mmol), 1-benzylpiperazin-2-one (20.39 mg, 0.107 mmol) and Hunig's Base (0.037 mL, 0.214 mmol) were dissolved in DMF (2 mL). The reaction mixture was stirred at room temperature overnight. The product containing solution was fractionated using preparative LC/MS under the following conditions: Column: XBridge C18, 19 × 200 mm, 5-µm particles; Mobile Phase A: 5:95 acetonitrile: water with 10-mM ammonium acetate; Mobile Phase B: 95:5 acetonitrile: water with 10-mM ammonium acetate; Gradient: 21-61% B over 20 minutes, then a 4-minute hold at 100% B; Flow: 20 mL/min. Fractions containing the product were combined and dried via centrifugal evaporation. The yield of the product was 24.1 mg, and its estimated purity by LCMS analysis was 100%. Analytical LC/MS was used to determine the final purity. Injection 1 conditions: Column: Waters XBridge C18, 2.1 mm × 50 mm, 1.7 µm particles; Mobile Phase A: 5:95 acetonitrile:water with 10 mM ammonium acetate; Mobile Phase B: 95:5 acetonitrile:water with 10 mM ammonium acetate; Temperature: 50 °C; Gradient: 0 %B to 100 %B over 3 min, then a 0.75 min hold at 100 %B; Flow: 1 mL/min; Detection: MS and UV (220 nm). Injection 1 results: Purity: 100.0 %; Observed Mass: 411.2; Retention Time: 1.6 min. Injection 2 conditions: Column: Waters XBridge C18, 2.1 mm × 50 mm, 1.7 µm particles; Mobile Phase A: 5:95 acetonitrile:water with 0.1 % trifluoroacetic acid; Mobile Phase B: 95:5 acetonitrile:water with 0.1 % trifluoroacetic acid; Temperature: 50 °C; Gradient: 0 %B to 100 %B over 3 min, then a 0.75 min hold at 100 %B; Flow: 1 mL/min; Detection: MS and UV (220 nm). Injection 2 results: Purity: 100.0 %; Observed Mass: 411.02; Retention Time: 1.6 min. ¹H NMR (500 MHz, DMSO-d₆) δ 7.77-7.61 (m, 3H), 7.43-7.36 (m, 2H), 7.35-7.27 (m, 3H), 4.62 (s, 2H), 3.88 (s, 2H), 3.66 (s, 3H), 2.56 (s, 4H).

### INTERMEDIATE 14

### 4-hydroxy-1-methyl-2-oxo-1,2-dihydroquinoline-3-carbonitrile

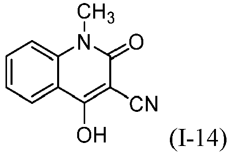

In a round bottom flask, methyl 2-cyanoacetate (4.95 g, 50 mmol) was dissolved in NMP (40 mL) and the mixture was cooled to 0 °C. Sodium hydride (1.818 g, 50.0 mmol) was added and the reaction mixture was stirred for 5 minutes at 0 °C, and then 15 minutes at room temperature. At room temperature, 1-methyl-1H-benzo[d][1,3]oxazine-2,4-dione (8.86 g, 50.0 mmol) was added and the reaction mixture was heated at 120 °C for 2 hours. LCMS analysis showed the reaction was complete. The reaction mixture was diluted with 60 mL of diethyl ether and the mixture was poured onto 50 mL ice and 100 mL 1 N HCl. The resulting solids were collected (6 g). As the ether evaporated, additional solids formed in the filtrate and these solids were collected separately (2.2 g). NMR and LCMS of both solid samples were identical and consistent with the product. LC/MS 2.15 min., M⁺¹: 201.2, Exact Mass: 200.1. Start % B = 0, Final % B = 100, Gradient Time = 4 minutes, Flow Rate = 0.8 mL/min, Wavelength = 220 nm, Solvent Pair = Water -Methanol-0.1% TFA, Solvent A = 90% Water -10% Methanol-0.1% TFA, Solvent B = 10% Water -90% Methanol-0.1% TFA, Column = Phenomenex-Luna 2.0 × 50 mm 3 µm. ¹H NMR (400 MHz, DMSO-d₆) δ 8.11 (dd, J=8.1, 1.2 Hz, 1H), 7.77 (ddd, J=8.6, 7.2, 1.6 Hz, 1H), 7.56 (d, J=8.6 Hz, 1H), 7.34 (t, J=7.6 Hz, 1H), 3.57 (s, 3H).

### INTERMEDIATE 15

### 4-chloro-1-methyl-2-oxo-1,2-dihydroquinoline-3-carbonitrile

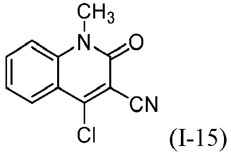

In a round bottom flask, 1-methyl-2,4-dioxo-1,2,3,4-tetrahydroquinoline-3-carbonitrile (6 g, 30.0 mmol) and phosphorus oxychloride (13.97 ml, 150 mmol) were combined at room temperature. The reaction mixture was heated at 110 °C for 40 minutes. After cooling to room temperature, the volatiles were removed on a Rotovap. To the crude material, 50 mL diethyl ether was added and the volatiles were again removed on a Rotovap. Water was slowly added with vigorous stirring and the solids collected by filtration. The residue was place under vacuum overnight to provide the title compound (4.6 g) as a pale yellow solid. ¹H NMR (400 MHz, CHLOROFORM-d) δ 8.14 (dd, J=8.2, 1.3 Hz, 1H), 7.82 (ddd, J=8.6, 7.1, 1.5 Hz, 1H), 7.51-7.42 (m, 2H), 3.79 (s, 3H).

### EXAMPLE 7

### 4-[(2 S, SR)-4-[bis(4-fluorophenyl)methyl]-2, 5-dimethylpiperazin-1-yl]-1-methyl-2-oxo-1,2-dihydroquinoline-6-carbonitrile (homochiral)

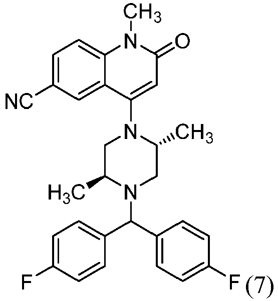

To a stirred solution of 4-((2S,5R)-2,5-dimethylpiperazin-1-yl)-1-methyl-2-oxo-1,2-dihydroquinoline-6-carbonitrile (0.08 g, 0.270 mmol) in acetonitrile (5 mL) at room temperature were added DIPEA (0.141 mL, 0.810 mmol) and 4,4'-(bromomethylene) bis(fluorobenzene) (0.115 g, 0.405 mmol). The reaction mixture was heated at 85 °C for 16 h. The reaction mixture cooled to room temperature and the solvent was removed under reduced pressure. The residue was dissolved in ethyl acetate (100 mL). The organic layer was washed with brine, dried over Na₂SO₄ and concentrated under reduced pressure to yield the crude product which was purified by reverse phase preparative HPLC. Method: Column: Waters XBridge C18, 150 mm × 19 mm, 5-µm particles; Mobile Phase A: 5:95 acetonitrile: water with 10-mM ammonium acetate; Mobile Phase B: 95:5 acetonitrile: water with 10-mM ammonium acetate; Gradient: a 0-minute hold at 15% B, 15-65% B over 25 minutes, then a 5-minute hold at 100% B; Flow Rate: 15mL/min. The fraction was concentrated under reduced pressure to purify 4-((2S,5R)-4-(bis(4-fluorophenyl)methyl)-2,5-dimethylpiperazin-1-yl)-1-methyl-2-oxo-1,2-dihydroquinoline-6-carbonitrile (50 mg, 0.100 mmol, 37.2 % yield). LCMS: *m*/*z* = 499.2 (M+H); rt 2.426 min. LCMS Method Column: XBridge BEH XP C18 (50 × 2.1) mm, 2.5 µm Mobile phase A: 95% water: 5% acetonitrile; 10 mM NH₄OAC, Mobile phase B: 5% Water:95% Acetonitrile; 10 mM NH₄OAC Flow: 1.1 mL/min, Temp: 50 °C, ¹H NMR (400 MHz, DMSO-*d*₆) δ ppm 8.12 (d, *J*=1.71 Hz, 1 H) 7.97 (dd, *J*=8.80, 1.71 Hz, 1 H) 7.66 (d, *J*=8.80 Hz, 1 H) 7.51-7.62 (m, 4 H) 7.15 (dt, J=17.61, 8.80 Hz, 4 H) 6.06 (s, 1 H) 4.82 (s, 1 H) 3.75-3.83 (m, 1 H) 3.55-3.65 (m, 1 H) 3.33 (s, 3 H) 3.01-3.11 (m, 2 H) 2.65 (dd, *J*=11.86, 2.57 Hz, 1 H) 2.27 (dd, *J*=11.49, 1.96 Hz, 1 H) 1.09 (d, *J*=6.60 Hz, 3 H) 1.04 (d, *J*=6.36 Hz, 3 H).

### INTERMEDIATE 16

### 6-methoxy-1-methyl-2H-benzo[d][1,3]oxazine-2,4(1H)-dione

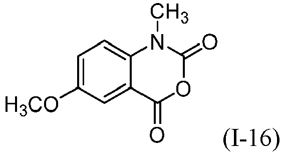

To a solution of 6-methoxy-1H-benzo[d][1,3]oxazine-2,4-dione (3 g, 15.53 mmol) in DMF (20 mL), 60% dispersion of sodium hydride (0.745 g, 18.64 mmol) in mineral oil was added in portions at 0 °C. The reaction mixture was warmed to room temperature and stirred for 30 min. Then methyl iodide (1.165 mL, 18.64 mmol) was added dropwise. The reaction mixture was then stirred at room temperature overnight. The reaction was quenched with water. Diethyl ether was added to the mixture. The product, 6-methoxy-1-methyl-1H-benzo[d][1,3]oxazine-2,4-dione (3.11 g, 15.01 mmol, 97 % yield), was collected as a beige solid. LC/MS Column: Phenomenex LUNA C18, 2.0 × 50 mm, 3-mm particles; Mobile Phase A: 5:95 acetonitrile:water with 10 mM ammonium acetate; Mobile Phase B: 95:5 acetonitrile:water with 10 mM ammonium acetate; Temperature: 40 °C; Gradient: 0%B, 0-100% B over 4 minutes, then a 1 minute hold at 100% B; Flow: 0.8 mL/min; Detection: UV at 220 nm. Retention time 2.032 min, 208.0 (M+H⁺). ¹H NMR (400 MHz, DMSO-d₆) δ 7.68-7.23 (m, 3H), 3.85 (s, 3H), 3.46 (s, 3H).

### INTERMEDIATE 17

### 6-methoxy-1-methyl-3-nitroquinoline-2,4(1H,3H)-dione

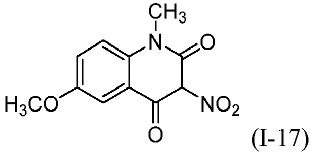

To a solution of ethyl 2-nitroacetate (2.120 g, 15.93 mmol) in NMP (10 mL) in a round-bottomed flask at 0 °C, a 60% dispersion of sodium hydride (0.695 g, 17.38 mmol) in mineral oil was added in portions. The reaction mixture was stirred for 5 minutes at 0 °C, then 15 minutes at room temperature. Then 6-methoxy-1-methyl-1H-benzo[d][1,3]oxazine-2,4-dione (3 g, 14.48 mmol) was added and the reaction mixture was heated at 120 °C for 3 hr. LC/MS shown completion of reaction. The reaction was quenched with ice-water. The mixture was acidified with 1 N HCl solution. Ethyl ether was added and 6-methoxy-1-methyl-3-nitroquinoline-2,4(1H,3H)-dione (1.66 g, 6.63 mmol, 45.8 % yield) as a yellow solid. LC/MS Column: Phenomenex LUNA C18, 2.0 × 50 mm, 3-mm particles; Mobile Phase A: 5:95 acetonitrile:water with 10 mM ammonium acetate; Mobile Phase B: 95:5 acetonitrile:water with 10 mM ammonium acetate; Temperature: 40 °C; Gradient: 0%B, 0-100% B over 4 minutes, then a 1 minute hold at 100% B; Flow: 0.8 mL/min; Detection: UV at 220 nm. Retention time 1.613 min, 251.1 (M+H⁺). ¹H NMR (400 MHz, DMSO-d₆) δ 7.61 (d, *J*=2.7 Hz, 1H), 7.54 (d, *J*=9.3 Hz, 1H), 7.39 (dd, *J*=9.2, 2.8 Hz, 1H), 3.85 (s, 3H), 3.59 (s, 3H).

### INTERMEDIATE 18

### 4-chloro-6-methoxy-1-methyl-3-nitroquinolin-2(1H)-one

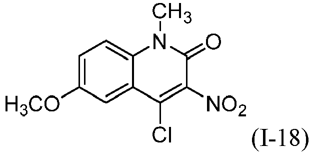

In a sealed tube, 6-methoxy-1-methyl-3-nitroquinoline-2,4(1H,3H)-dione (1.65 g, 6.59 mmol) and phosphorus oxychloride (10 mL, 107 mmol) were added. The reaction mixture was heated at 95 °C for 4 hr. The reaction mixture was then concentrated and the residue was poured into ice-water and neutralized with NaHCO₃ solid. The mixture was extracted with dichloromethane (2 × 40 mL) and the organic layers were combined, dried (MgSO₄) and concentrated to give 4-chloro-6-methoxy-1-methyl-3-nitroquinolin-2(1H)-one (1.58 g, 5.88 mmol, 89 % yield) as an orange solid. LC/MS Column: Phenomenex LUNA C18, 2.0 × 50 mm, 3-mm particles; Mobile Phase A: 5:95 acetonitrile:water with 10 mM ammonium acetate; Mobile Phase B: 95:5 acetonitrile:water with 10 mM ammonium acetate; Temperature: 40 °C; Gradient: 0%B, 0-100% B over 4 minutes, then a 1 minute hold at 100% B; Flow: 0.8 mL/min; Detection: UV at 220 nm. Retention time 2.703 min, 269.1 (M+H⁺). ¹H NMR (500 MHz, DMSO-d₆) δ 7.76 (d, J=9.2 Hz, 1H), 7.57 (d, J=9.5 Hz, 1H), 7.47 (s, 1H), 3.91 (s, 3H), 3.72 (s, 3H).

### EXAMPLE 8

### 6-methoxy-1-methyl-4-{4-[(naphthalen-1-yl)methyl]piperazin-1-yl}-3-nitro-1,2-dihydroquinolin-2-one

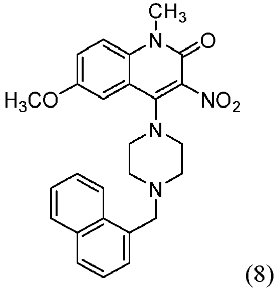

To a solution of 4-chloro-6-methoxy-1-methyl-3-nitroquinolin-2(1H)-one (15 mg, 0.056 mmol) in DMF (1 mL), 1-(naphthalen-1-ylmethyl)piperazine (12.64 mg, 0.056 mmol) and triethylamine (0.023 mL, 0.168 mmol) were added. The reaction mixture was stirred at room temperature overnight. LC/MS shown completion of reaction. To the reaction mixture was then added CH₃CN. The reaction mixture was purified via preparative LC/MS with the following conditions: Column: XBridge C18, 19 × 200 mm, 5-µm particles; Mobile Phase A: 5:95 acetonitrile: water with 10-mM ammonium acetate; Mobile Phase B: 95:5 acetonitrile: water with 10-mM ammonium acetate; Gradient: 50-90% B over 15 minutes, then a 5-minute hold at 100% B; Flow: 20 mL/min. Fractions containing the product were combined and dried via centrifugal evaporation. LC\MS: Column: Phenomenex LUNA C18, 2.0 × 50 mm, 3-mm particles; Mobile Phase A: 5:95 acetonitrile:water with 10 mM ammonium acetate; Mobile Phase B: 95:5 acetonitrile:water with 10 mM ammonium acetate; Temperature: 40 °C; Gradient: 0%B, 0-100% B over 4 minutes, then a 1 minute hold at 100% B; Flow: 0.8 mL/min; Detection: UV at 220 nm. Observed MS Ion 459.2, retention time 2.34 minutes. ¹H NMR (500 MHz, DMSO-d₆) δ 8.31 (d, *J*=8.4 Hz, 1H), 7.95-7.89 (m, 1H), 7.88-7.83 (m, 1H), 7.61-7.37 (m, 6H), 7.36-7.31 (m, 1H), 4.00 (s, 2H), 3.88 (s, 3H), 3.60 (s, 3H), 3.13 (br, s, 4H), 2.69 (br, s, 4H).

### INTERMEDIATE 22

### Ethyl 6-fluoro-1-methyl-2-oxo-4-(((trifluoromethyl)sulfonyl)oxy)-1,2-dihydroquinoline-3-carboxylate

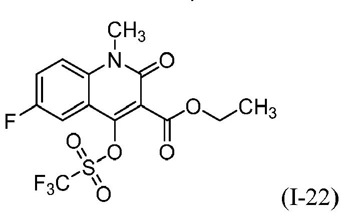

1,1,1-Trifluoro-N-phenyl-N-(trifluoromethyl)sulfonyl methanesulfonamide (404 mg, 1.131 mmol) was added to a solution of ethyl 6-fluoro-1-methyl-2,4-dioxo-1,2,3,4-tetrahydroquinoline-3-carboxylate (100 mg, 0.377 mmol) and DIEA (0.263 mL, 1.508 mmol) in THF (3 mL). The resulting solution was stirred at room temperature over the weekend. LC/MS shown completion of reaction. The reaction mixture was concentrated. The crude product was purified by Biotage column using hexanes to 40% ethyl acetate in hexanes as eluent. The product came out at -30% ethyl acetate in hexanes. Fractions were collected and concentrated to give ethyl 6-fluoro-1-methyl-2-oxo-4-(((trifluoromethyl)sulfonyl)oxy)-1,2-dihydroquinoline-3-carboxylate (71 mg, 0.179 mmol, 47.4 % yield) a light yellow solid. LC/MS Column: Phenomenex LUNA C18, 2.0 × 50 mm, 3-mm particles; Mobile Phase A: 10:90 methanol:water with 0.1% TFA; Mobile Phase B: 90:10 methanol:water with 0.1% TFA; Gradient: 0-100 % B over 4 minutes, then a 1 minute hold at 100% B; Flow: 0.8 mL/min; Detection: UV at 220 nm. Retention time 3.9 min, 397.9 (M+H⁺). ¹H NMR (400 MHz, chloroform-d) δ 7.60 (dd, *J*=8.3, 2.7 Hz, 1H), 7.55-7.44 (m, 2H), 4.48 (q, *J*=7.1 Hz, 2H), 3.78 (s, 3H), 1.44 (t, *J*=7.2 Hz, 3H).

### BIOLOGICAL ASSAYS

The pharmacological properties of the compounds of this invention may be confirmed by a number of biological assays. The exemplified biological assays, which follow, have been carried out with compounds of the invention.

### Assay 1: In vitro DGK Inhibition Assays - Method A

The DGKα and DGKζ reactions were performed using extruded liposomes (DGKα and DGKζ LIPGLO assays). The reactions were carried out in 50 mM MOPS pH 7.5, 100 mM NaCl, 10 mM MgClz, 1 µM CaClz, and 1 mM DTT (assay buffer). The lipid substrate concentrations were 2 mM PS, 0.25 mM DAG, and 2.75 mM PC for the extruded liposome reactions. The reactions were carried out in 150 µM ATP. The enzyme concentrations for the DGKα and DGKζ were 5 nM.

The compound inhibition studies were carried out as follows: 50 nL droplets of each test compound (top concentration 10 mM with 11 point, 3-fold dilution series for each compound) solubilized in DMSO were transferred to wells of a white 1536 well plate (Corning 3725). A 5 mL enzyme/substrate solution at 2× final reaction concentration was prepared by combining 2.5 mL 4× enzyme solution (20 nM DGKα or DGKζ (prepared as described below) in assay buffer) and 2.5 mL of 4× liposome solution (compositions described below) and incubated at room temperature for 10 minutes. Next, 1 µL 2× enzyme/substrate solution was added to wells containing the test compound and reactions were initiated with the addition of 1 µL 300 uM ATP. The reactions were allowed to proceed for 1 hr, after which 2 µL Glo Reagent (Promega V9101) was added and incubated for 40 minutes. Next, 4 µL Kinase Detection Reagent was added and incubated for 30 minutes. Luminescence was recorded using an EnVision microplate reader. The percent inhibition was calculated from the ATP conversion generated by no enzyme control reactions for 100 % inhibition and vehicle-only reactions for 0 % inhibition. The compounds were evaluated at 11 concentrations to determine IC₅₀.

### 4× Liposome Preparation

The lipid composition was 5 mol% DAG (Avanti 8008110), 40 mol% PS (Avanti 840035P), and 55 mol% PC (Avanti 850457) at a total lipid concentration of 15.2 mg/mL for the 4× liposome solution. The PC, DAG, and PS were dissolved in chloroform, combined, and dried in vacuo to a thin film. The lipids were hydrated to 20 mM in 50 mM MOPS pH 7.5, 100 mM NaCl, 5 mM MgCl₂, and were freeze-thawed five times. The lipid suspension was extruded through a 100 nm polycarbonate filter eleven times. Dynamic light scattering was carried out to confirm liposome size (50-60 nm radius). The liposome preparation was stored at 4 °C for as long as four weeks.

### Baculovirus Expression of Human DGKα and DGKζ

Human DGK-alpha-TVMV-His-pFBgate and human DGK-zeta-transcript variant-2-TVMV-His-pFBgate baculovirus samples were generated using the Bac-to-Bac baculovirus expression system (Invitrogen) according to the manufacturer's protocol. The DNA used for expression of DGK-alpha and DGK-zeta have SEQ ID NOs: 1 and 3, respectively. Baculovirus amplification was achieved using infected Sf9 cells at 1: 1500 virus/cell ratios, and grown for 65 hours at 27 °C post-transfection.

The expression scale up for each protein was carried out in the Cellbag 50L WAVE-Bioreactor System 20/50 from GE Healthcare Bioscience. 12 L of 2 × 10⁶ cells/mL Sf9 cells (Expression System, Davis, CA) grown in ESF921 insect medium (Expression System) were infected with virus stock at 1:200 virus/cell ratios, and grown for 66-68 hours at 27 °C post-infection. The infected cell culture was harvested by centrifugation at 2000 rpm for 20 min 4 °C in a SORVALL^{®} RC12BP centrifuge. The cell pellets were stored at -70 °C until purification.

### Purification of human DGK-alpha and DGK-zeta

Full length human DGKα and DGKζ, each expressed containing a TVMV-cleavable C-terminal Hexa-His tag sequence (SEQ ID NOs: 2 and 4, respectively) and produced as described above, were purified from Sf9 baculovirus-infected insect cell paste. The cells were lysed using nitrogen cavitation method with a nitrogen bomb (Parr Instruments), and the lysates were clarified by centrifugation. The clarified lysates were purified to ~90 % homogeneity, using three successive column chromatography steps on an ÄKTA Purifier Plus system. The three steps column chromatography included nickel affinity resin capture (i.e. HisTrap FF crude, GE Healthcare), followed by size exclusion chromatography (i.e. HiLoad 26/600 Superdex 200 prep grade, GE Healthcare for DGK-alpha, and HiPrep 26/600 Sephacryl S 300_HR, GE Healthcare for DGK-zeta). The third step was ion exchange chromatography, and differed for the two isoforms. DGKα was polished using Q-Sepharose anion exchange chromatography (GE Healthcare). DGKζ was polished using SP Sepharose cation exchange chromatography (GE Healthcare). The proteins were delivered at concentrations of ≥2 mg/mL. The formulation buffers were identical for both proteins: 50 mM Hepes, pH 7.2, 500 mM NaCl, 10 % v/v glycerol, 1 mM TCEP, and 0.5 mM EDTA.

### Assay 2: In vitro DGK Inhibition Assays - Method B

The DGKα and DGKζ reactions were performed using either extruded liposome (DGKα and DGKζ LIPGLO assays). The reactions were carried out in 50 mM MOPS pH 7.5, 100 mM NaCl, 10 mM MgClz, 1 µM CaClz, and 1 mM DTT (assay buffer). The lipid substrate concentrations were 2 mM PS, 0.25 mM DAG, and 2.75 mM PC for the extruded liposome reactions (5 mM total lipid). The reactions were carried out in 150 µM ATP. The enzyme concentrations for the DGKα and DGKζ were 5 nM.

The compound inhibition studies were carried out as follows: 25 nL droplets of each test compound (top concentration 10 mM with 11 point, 3-fold dilution series for each compound) solubilized in DMSO were transferred to wells of a white 1536 well plate (Corning 3725). A 5 mL enzyme/lipid substrate solution at 2× final reaction concentration was prepared by combining 2.5 mL 4× enzyme solution (20 nM DGKα or DGKζ (prepared as described below) in assay buffer) and 2.5 mL of 4× detergent/lipid micelle solution (compositions described below) and incubated at room temperature for 10 minutes. Next, 1 µL 2× enzyme/ lipid substrate solution was added to wells containing the test compound and reactions were initiated with the addition of 1 µL 300 uM ATP. The reactions were allowed to proceed for 2 hr, after which 2 µL Glo Reagent (Promega V9101) was added and incubated for 40 minutes. Next, 4 µL Kinase Detection Reagent was added and incubated for 30 minutes. Luminescence was recorded using an EnVision microplate reader. The percent inhibition was calculated from the ATP conversion generated by no enzyme control reactions for 100 % inhibition and vehicle-only reactions for 0 % inhibition. The compounds were evaluated at 11 concentrations to determine IC₅₀.

### 2× Liposome Preparation

The lipid composition was 5 mol% DAG (Avanti 8008110), 40 mol% PS (Avanti 840035P), and 55 mol% PC (Avanti 850457) at a total lipid concentration of 7-8 mg/mL for the liposome solution. The PC, DAG, and PS were dissolved in chloroform, combined, and dried in vacuo to a thin film. The lipids were hydrated to 20 mM in 50 mM MOPS pH 7.5, 100 mM NaCl, 5 mM MgCl₂, and were freeze-thawed five times. The lipid suspension was extruded through a 100 nm polycarbonate filter 10-12 times. Dynamic light scattering was carried out to confirm liposome size (50-60 nm radius). The liposome preparation was stored at 4 °C for as long as four weeks.

### Baculovirus Expression of near full length Human DGKα and full length DGKζ

Human MA-hDGKα-(S9-S727)-Ct-TVMV-His-pFBgate and full length human DGK-ζ-transcript variant-2-TVMV-His-pFBgate baculovirus samples were generated using the Bac-to-Bac baculovirus expression system (Invitrogen) according to the manufacturer's protocol (note: MA- in name of DGKα reagents indicates two extra amino acids added prior to Ser-9). The DNA used for expression of the DGK-α(9-727) and DGK-ζ have SEQ ID NOs: 5 and 3, respectively. Baculovirus amplification was achieved using infected Sf9 cells at 1:1500 virus/cell ratios, and grown for 65 hours at 27 °C post-transfection.

The expression scale up for the near full length DGK-α(9-727) protein was carried out in 2 L flasks, and the full length DGKζ was done using a Cellbag 50 L WAVE-Bioreactor System 20/50 from GE Healthcare Bioscience. The proteins were expressed at different volumes using similar conditions. For expression of DGKα(9-727), 2 × 2 L flasks each containing 0.8 L final volume of culture media were used, and DGKζ was grown at 12 L scale in a 50 L Cellbag. For each, an initial density of 2 × 10⁶ cells/mL Sf9 cells (Expression System, Davis, CA) was seeded in ESF921 insect medium (Expression System), infected with virus stock at 1:200 virus/cell ratios, and grown for 66-68 hours at 27 °C post-infection. The infected cell cultures were harvested by centrifugation at 2000 rpm for 20 min 4 °C in a SORVALL^{®} RC12BP centrifuge. The cell pellets were stored at -80 °C until purification.

### Purification of human DGK-alpha and DGK-zeta

Human DGKα(9-727) and full length DGKζ, each expressed containing a TVMV-cleavable C-terminal Hexa-His tag sequence (SEQ ID NOs: 2 and 4, respectively) and produced as described above, were purified from Sf9 baculovirus-infected insect cell paste. The cell pastes were thawed and suspended in buffer (50 mM HEPES, pH 7.2, 300 mM NaCl, 10% v/v glycerol, 1 mM TCEP containing benzonase and protease inhibitors), to 1: 10 v/v of original culture volume. Lysis was accomplished using the nitrogen cavitation method with a nitrogen bomb (Parr Instruments), and the lysates were clarified by high speed centrifugation. The clarified lysates were purified to -90% homogeneity, using two or three successive column chromatography steps, respectively, on an ÄKTA Purifier Plus system. Both isoforms were purified by nickel affinity purification with imidazole gradient elution (i.e. HisTrap FF, GE Healthcare), followed by size exclusion chromatography (i.e. HiLoad 26/600 Superdex 200 prep grade, GE Healthcare, for DGKα(9-727), and HiPrep 26/600 Sephacryl S 300 HR, GE Healthcare, for DGKζ). These two steps yielded DGKα(9-727) at >90% purity. Achieving similar purity for full length DGKζ required a third step, employing cation exchange chromatography (SP Sepharose FF, GE Healthcare), and eluting with a NaCl gradient. The final formulation buffers were similar for both proteins, with DGKα(9-727) prepared in 50 mM Hepes, pH 7.3, 300 mM NaCl, 10% v/v glycerol, and 1 mM TCEP, and full length DGKζ prepared in 50 mM Hepes, pH 7.3, 500 mM NaCl, 5% v/v glycerol, and 1 mM TCEP. The proteins were concentrated to 1-2 mg/mL, flash frozen, and kept at -80 °C for long term storage.

### Assay 3: Raji CD4 T cell IL2 Assay

A 1536-well IL-2 assay was performed in 4 µL volume using pre-activated CD4 T cells and Raji cells. Prior to the assay, CD4 T cells were pre-activated by treatment with α-CD3, α-CD28 and PHA at 1.5 µg/mL, 1 µg/m L, and 10 µg/mL, respectively. Raji cells were treated with Staphylococcal enterotoxin B (SEB) at 10,000 ng/mL. Serially diluted compounds were first transferred to 1536-well assay plate (Corning, #3727), followed by addition of 2 µL of pre-activated CD4 T cells (final density at 6000 cells/well) and 2 µL of SEB-treated Raji cells (2000 cells/well). After 24 hours incubation at a 37 °C/5% CO₂ incubator, 4 µl of IL-2 detection reagents were added to the assay plate (Cisbio, #64IL2PEC). The assay plates were read on an Envision reader. To assess compound cytotoxicity, either Raji or CD4 T cells were incubated with the serially diluted compounds. After 24 hours incubation, 4 µL of Cell Titer Glo (Promega, #G7572) were added, and the plates were read on an Envision reader. The 50 % effective concentration (IC₅₀) was calculated using the four-parameter logistic formula y = A+((B-A)/(1+((C/x)^D))), where A and B denote minimal and maximal % activation or inhibition, respectively, C is the IC₅₀, D is hill slope and x represent compound concentration.

### Assay 4: CellTiter-Glo CD8 T Cell Proliferation Assay

Frozen naive human CD8 T cells were thawed in RPMI+10 % FBS, incubated for 2 h in 37 °C, and counted. The 384-well tissue culture plate was coated overnight at 4 °C with 20 µl anti-human CD3 at 0.1 µg/mL in plain RPMI, which was removed off the plate before 20k/40 µL CD8 T cells with 0.5 µg/ml soluble anti-human CD28 were added to each well. The compounds were echoed to the cell plate immediately after the cells were plated. After 72 h incubation at 37 °C incubator, 10 µL CellTiter-glo reagent (Promega catalog number G7570) was added to each well. The plate was vigorously shaken for 5 mins, incubated at room temperature for another 15 mins and read on Envision for CD8 T cell proliferation. In analysis, 0.1 µg/mL anti-CD3 and 0.5 µg/mL anti-CD28 stimulated CD8 T cell signal was background. The reference compound, 8-(4-(bis(4-fluorophenyl)methyl) piperazin-1-yl)-5-methyl-7-nitro-6-oxo-5,6-dihydro-1,5-naphthyridine-2-carbonitrile, at 3 µM was used to set the 100 % range and EC₅₀ was at absolute 50 % to normalize the data.

### Assay 5: DGK AP1-Reporter Assay

The Jurkat AP1-luciferase Reporter was generated using the Cignal Lenti AP 1 Reporter (luc) Kit from SABiosciences (CLS-011L).

The compounds were transferred from an Echo LDV plate to individual wells of a 384-well plate (white, solid-bottom, opaque PE CulturPlate 6007768) using an Echo550 instrument. The sample size was 30 nL per well; and one destination plate per source plate. The cell suspensions were prepared by transferring 40 mL cells (2x 20 mL) to clean 50 mL conical tubes. The cells were concentrated by centrifugation (1200 rpm; 5 mins; ambient temperature). The supernatant was removed and all cells were suspended in RPMI (Gibco 11875) +10 % FBS to make a 1.35×10⁶ cells/ml concentration. The cells were added manually using a multi-channel pipette, 30 µL/well of cell suspension to a 384-well TC plate containing the compounds, 4.0×10⁴ cells per well. The cell plates were incubated for 20 minutes at 37 °C and 5% CO₂.

During the incubation, anti-CD3 antibody (αCD3) solutions were prepared by mixing 3 µL aCD3 (1.3 mg/mL) with 10 mL medium [final cone = 0.4 µg/mL] . Next, 1.5 µl aCD3 (1.3 mg/mL) was mixed with 0.5 mL medium [final conc = 4 µg/ml]. After 20 minutes, 10 µL medium was added to all wells in column 1, wells A to M, and 10 µL αCD3 (4ug/mL) per well was added in column 1, rows N to P for reference. Then using a multi-channel pipette, 10 µL αCD3 (0.4ug/mL) per well was added. The αCD3 stimulated +/- compound-treated cells were incubated at 37 °C, 5% CO₂ for 6 hours.

During this incubation period, Steady-Glo (Promega E2520) reagent was slowly thawed to ambient temperature. Next, 20 µL Steady-Glo reagent per well was added using a multi-drop Combi-dispenser. Bubbles were removed by centrifugation (2000 rpm, ambient temperature, 10 secs). The cells were incubated at room temperature for 5 minutes. Samples were characterized by measuring the Relative Light Units (RLU) with an using Envision Plate Reader Instrument on a luminescence protocol. The data was analyzed using the reference compound, 8-(4-(bis(4-fluorophenyl)methyl)piperazin-1-yl)-5-methyl-7-nitro-6-oxo-5,6-dihydro-1,5-naphthyridine-2-carbonitrile, to normalize 100 % inhibition.

### Assay 6: Murine Cytotoxic T Lymphocyte Assay

An antigen-specific cytolytic T-cell (CTL) assay was developed to evaluate functionally the ability of DGKα and DGKζ inhibitors to enhance effector T cell mediated tumor cell killing activity. CD8+ T-cells isolated from the OT-1 transgenic mouse recognize antigen presenting cells, MC38, that present the ovalbumin derived peptide SIINFEKL. Recognition of the cognate antigen initiates the cytolytic activity of the OT-1 antigen-specific CD8+ T cells.

Functional CTL cells were generated as follows: OT-1 splenocytes from 8-12 week old mice were isolated and expanded in the presence of the SIINFEKL peptide at 1 µg/mL and mIL2 at 10 U/mL. After three days, fresh media with mIL2 U/ml was added. On day 5 of the expansion, the CD8+ T cells were isolated and ready for use. Activated CTL cells may be stored frozen for 6 months. Separately, one million MC38 tumor cells were pulsed with 1 µg/mL of SIINFEKL-OVA peptide for 3 hours at 37 °C. The cells were washed (3×) with fresh media to remove excess peptide. Finally, CTL cells that were pretreated with DGK inhibitors for 1 hour in a 96-well U bottom plate were combined with the antigen loaded MC38 tumor cells at a 1:10 ratio. The cells were then spun at 700 rpm for 5 min and placed in an incubator overnight at 37 °C. After 24 hours, the supernatant was collected for analysis of IFN-γ cytokine levels by AlphaLisa purchased from Perkin Elmer.

### Assay 7: PHA Proliferation Assay

Phytohaemagglutinin (PHA)-stimulated blast cells from frozen stocks were incubated in RPMI medium (Gibco, ThermoFisher Scientific, Waltham, MA) supplemented with 10 % fetal bovine serum (Sigma Aldrich, St. Louis, MO) for one hour prior to adding to individual wells of a 384-well plate (10,000 cells per well). The compounds were transferred to individual wells of a 384-well plate and the treated cells are maintained at 37 °C, 5% CO₂ for 72 h in culture medium containing human IL2 (20 ng/mL) prior to measuring growth using MTS reagent [3-(4,5-dimethyl-2-yl)-5-(3-carboxymethoxyphenyl)-2-(4-sulfophenyl)-2H-tetrazolium] following manufacturer's instructions (Promega, Madison, WI). Percent inhibition was calculated comparing values between IL2 stimulated (0 % inhibition) and unstimulated control (100 % inhibition). Inhibition concentration (IC₅₀) determinations were calculated based on 50 % inhibition on the fold-induction between IL2 stimulated and unstimulated treatments.

### Assay 8: Human CD8 T cells IFN-γ Assay

Frozen naive human CD8 T cells were thawed in AIM-V media, incubated for 2 h in 37 °C, and counted. The 384-well tissue culture plate was coated overnight at 4 °C with 20 µL anti-human CD3 at 0.05 µg/mL in PBS, which was removed off the plate before 40,000 cells per 40 microliters CD8 T cells with 0.1 µg/mL soluble anti-human CD28 were added to each well. The compounds were transferred using an Echo liquid handler to the cell plate immediately after the cells were plated. After 20 h incubation at 37 °C incubator, 3 microliters per well supernatants transferred into a new 384-well white assay plate for cytokine measurement.

Interferon-γ (IFN-γ) was quantitated using the AlphLISA kit (Cat#AL217) as described by the manufacturer manual (Perkin Elmer). The counts from each well were converted to IFN-γ concentration (pg/mL). The compound EC₅₀ values were determined by setting 0.05 µg/mL anti-CD3 plus 0.1 µg/mL anti-CD28 as the baseline, and co-stimulation of 3 µM of the reference compound, 8-(4-(bis(4-fluorophenyl)methyl) piperazin-1-yl)-5-methyl-7-nitro-6-oxo-5,6-dihydro-1,5-naphthyridine-2-carbonitrile, with anti-CD3 plus anti-CD28 as 100 % activation.

### Assay 9: Human CD8 T cells pERK Assay

Frozen naive human CD8 T cells were thawed in AIM-V media, incubated for 2 h in 37 °C, and counted. The CD8 positive T cells were added to 384-well tissue culture plate at 20,000 cells per well in AIM-V media. One compound was added to each well, then bead bound anti-human CD3 and anti-CD28 mAb were added at final concentration of 0.3 µg/mL. The cells were incubated at 37 °C for 10 minutes. The reaction was stopped by adding lysis buffer from the AlphaLISA Surefire kit. (Perkin Elmer, cat# ALSU-PERK-A). Lysate (5 µL per well) was transferred into a new 384-well white assay plate for pERK activation measurement.

Compound EC₅₀ was determined as setting anti-CD3 plus anti-CD28 as baseline, and co-stimulation of 3 µM 8-(4-(bis(4-fluorophenyl)methyl)piperazin-1-yl)-5-methyl-7-nitro-6-oxo-5,6-dihydro-1,5-naphthyridine-2-carbonitrile with anti-CD3 plus anti-CD28 as 100 % activation.

### Assay 10: Human Whole Blood IFN-γ Assay

Human venous whole blood (22.5 µL per well), obtained from healthy donors, was pre-treated with compounds for one hour at 37 °C in a humidified 95% air/5% CO₂ incubator. The blood was stimulated with 2.5 µL anti-human CD3 and anti-CD28 mAb at a final concentration of 1 µg/mL each for 24 hours at 37 °C. IFN-γ in the supernatants was measured using AlphLISA kit (Cat#AL217).

Compound EC₅₀ determined as setting anti-CD3 plus anti-CD28 as baseline, and co-stimulation of 3 µM of the reference compound, 8-(4-(bis(4-fluorophenyl)methyl) piperazin-1-yl)-5-methyl-7-nitro-6-oxo-5,6-dihydro-1,5-naphthyridine-2-carbonitrile, with anti-CD3 plus anti-CD28 as 100 % activation.

### Assay 11: DGK Human Whole Blood pERK Assay

Human whole blood ERK phosphorylation assay was performed with human venous whole blood obtained from healthy donors (drawn with Heparin as anticoagulant). Serial dilutions of compounds (11 points, 3-fold) in DMSO were added to 384 well plates at 20 nL/well using an ECHO 550 acoustic dispenser (Labcyte) to achieve final starting concentration of 20 µM in assay. Heparinized human whole blood was added to the compound plate at 9 µL per well and incubated for one hour at 37 °C in a humidified 95%, air/5% CO₂ incubator. After one hour of compound incubation, 1 µL of human anti-CD3 antibody (in-house) in the presence of cross-linking antibody goat antimouse IgG (4 µg/mL) was added to the well at 1 µg/mL final concentration for stimulation of pathway and additionally incubated for 15 minutes at 37 °C. Stimulation was stopped by adding 90 µL Fix/Lyse buffer (BD 558049). Cells were washed and stained with anti-CD8 PE (BD 555635) antibodies for 60 minutes at room temperature, washed again, and permeabilized on ice using Perm III buffer (BD 558050) for 30 minutes. Cells were then stained with an Alexa Fluor^{®} 647 anti-ERK1/2 Phospho (Thr202/Tyr204) Antibody (Bioleged 675504) for 60 minutes at 1:50 dilution. Samples were washed and resuspended in dPBS containing 1% BSA (dPBS, Gibco 14190136; BSA, Sigma-Aldrich A9205). Samples analyzed using the Intellicyt^{®} iQue Screener PLUS. The pERK activation was quantitated by the percentage of pERK positive population within CD8 positive population. Calculations of compound potencies were based on internal compound at 20 µM concentration as a 100% activation, and anti-CD3 control as a 0% activation.

**TABLE A**

| In vitro DGK Inhibition IC₅₀ Activity Values | | |
|---|---|---|
| Ex. | DGKα LIPGLO | DGKζ LIPGLO |
| No. | IC₅₀ (µM) | IC₅₀ (µM) |
| 1 | 0.0073 | 41 |
| 2 | 0.051 | 35 |
| 4 | 0.15 | 7.9 |
| 5 | 0.50 | >240 |
| 7 | 1.3 | >250 |
| 8 | 39 | 82 |

Table A lists in vitro DGK inhibition IC₅₀ activity values measured in the DGKα and DGKζ liposome (LIPGLO) assays.

The compounds of the present invention possess activity as an inhibitor(s) of one or both of the DGKα and DGKζ enzymes, and therefore, may be used in the treatment of diseases associated with the inhibition of DGKα and DGKζ activity.
Nucleotide sequence encoding hDGKα-(M1-S735)-Ct-TVMV-His: 
1 ATGGCCAAGG AGAGGGGCCT AATAAGCCCC AGTGATTTTG CCCAGCTGCA
51 AAAATACATG GAATACTCCA CCAAAAAGGT CAGTGATGTC CTAAAGCTCT
101 TCGAGGATGG CGAGATGGCT AAATATGTCC AAGGAGATGC CATTGGGTAC
151 GAGGGATTCC AGCAATTCCT GAAAATCTAT CTCGAAGTGG ATAATGTTCC
201 CAGACACCTA AGCCTGGCAC TGTTTCAATC CTTTGAGACT GGTCACTGCT
251 TAAATGAGAC AAATGTGACA AAAGATGTGG TGTGTCTCAA TGATGTTTCC
301 TGCTACTTTT CCCTTCTGGA GGGTGGTCGG CCAGAAGACA AGTTAGAATT
351 CACCTTCAAG CTGTACGACA CGGACAGAAA TGGGATCCTG GACAGCTCAG
401 AAGTGGACAA AATTATCCTA CAGATGATGC GAGTGGCTGA ATACCTGGAT
451 TGGGATGTGT CTGAGCTGAG GCCGATTCTT CAGGAGATGA TGAAAGAGAT
501 TGACTATGAT GGCAGTGGCT CTGTCTCTCA AGCTGAGTGG GTCCGGGCTG
551 GGGCCACCAC CGTGCCACTG CTAGTGCTGC TGGGTCTGGA GATGACTCTG
601 AAGGACGACG GACAGCACAT GTGGAGGCCC AAGAGGTTCC CCAGACCAGT
651 CTACTGCAAT CTGTGCGAGT CAAGCATTGG TCTTGGCAAA CAGGGACTGA
701 GCTGTAACCT CTGTAAGTAC ACTGTTCACG ACCAGTGTGC CATGAAAGCC
751 CTGCCTTGTG AAGTCAGCAC CTATGCCAAG TCTCGGAAGG ACATTGGTGT
801 CCAATCACAT GTGTGGGTGC GAGGAGGCTG TGAGTCCGGG CGCTGCGACC
851 GCTGTCAGAA AAAGATCCGG ATCTACCACA GTCTGACCGG GCTGCATTGT
901 GTATGGTGCC ACCTAGAGAT CCACGATGAC TGCCTGCAAG CGGTGGGCCA
951 TGAGTGTGAC TGTGGGCTGC TCCGGGATCA CATCCTGCCT CCATCTTCCA
1001 TCTATCCCAG TGTCCTGGCC TCTGGACCGG ATCGTAAAAA TAGCAAAACA
1051 AGCCAGAAGA CCATGGATGA TTTAAATTTG AGCACCTCTG AGGCTCTGCG
1101 GATTGACCCT GTTCCTAACA CCCACCCACT TCTCGTCTTT GTCAATCCTA
1151 AGAGTGGCGG GAAGCAGGGG CAGAGGGTGC TCTGGAAGTT CCAGTATATA
1201 TTAAACCCTC GACAGGTGTT CAACCTCCTA AAGGATGGTC CTGAGATAGG
1251 GCTCCGATTA TTCAAGGATG TTCCTGATAG CCGGATTTTG GTGTGTGGTG
1301 GAGACGGCAC AGTAGGCTGG ATTCTAGAGA CCATTGACAA AGCTAACTTG
1351 CCAGTTTTGC CTCCTGTTGC TGTGTTGCCC CTGGGTACTG GAAATGATCT
1401 GGCTCGATGC CTAAGATGGG GAGGAGGTTA TGAAGGACAG AATCTGGCAA
1451 AGATCCTCAA GGATTTAGAG ATGAGTAAAG TGGTACATAT GGATCGATGG
 
1501 TCTGTGGAGG TGATACCTCA ACAAACTGAA GAAAAAAGTG ACCCAGTCCC
1551 CTTTCAAATC ATCAATAACT ACTTCTCTAT TGGCGTGGAT GCCTCTATTG
1601 CTCATCGATT CCACATCATG CGAGAGAAAT ATCCGGAGAA GTTCAACAGC
1651 AGAATGAAGA ACAAGCTATG GTACTTCGAA TTTGCCACAT CTGAATCCAT
1701 CTTCTCAACA TGCAAAAAGC TGGAGGAGTC TTTGACAGTT GAGATCTGTG
1751 GGAAACCGCT GGATCTGAGC AACCTGTCCC TAGAAGGCAT CGCAGTGCTA
1801 AACATCCCTA GCATGCATGG TGGCTCCAAC CTCTGGGGTG ATACCAGGAG
1851 ACCCCATGGG GATATCTATG GGATCAACCA GGCCTTAGGT GCTACAGCTA
1901 AAGTCATCAC CGACCCTGAT ATCCTGAAAA CCTGTGTACC AGACCTAAGT
1951 GACAAGAGAC TGGAAGTGGT TGGGCTGGAG GGTGCAATTG AGATGGGCCA
2001 AATCTATACC AAGCTCAAGA ATGCTGGACG TCGGCTGGCC AAGTGCTCTG
2051 AGATCACCTT CCACACCACA AAAACCCTTC CCATGCAAAT TGACGGAGAA
2101 CCCTGGATGC AGACGCCCTG TACAATCAAG ATCACCCACA AGAACCAGAT
2151 GCCCATGCTC ATGGGCCCAC CCCCCCGCTC CACCAATTTC TTTGGCTTCT
2201 TGAGCGGATC CTCGGAGACA GTGCGGTTTC AGGGACACCA CCACCATCAC
2251 CACTGA
(SEQ ID NO: 1)
Amino acid sequence of hDGKα-(M1-S735)-Ct-TVMV-His: 
0001 MAKERGLISP SDFAQLQKYM EYSTKKVSDV LKLFEDGEMA KYVQGDAIGY EGFQQFLKIY 0060
0061 LEVDNVPRHL SLALFQSFET GHCLNETNVT KDVVCLNDVS CYFSLLEGGR PEDKLEFTFK 0120
0121 LYDTDRNGIL DSSEVDKIIL QMMRVAEYLD WDVSELRPIL QEMMKEIDYD GSGSVSQAEW 0180
0181 VRAGATTVPL LVLLGLEMTL KDDGQHMWRP KRFPRPVYCN LCESSIGLGK QGLSCNLCKY 0240
0241 TVHDQCAMKA LPCEVSTYAK SRKDIGVQSH VWVRGGCESG RCDRCQKKIR IYHSLTGLHC 0300
0301 VWCHLEIHDD CLQAVGHECD CGLLRDHILP PSSIYPSVLA SGPDRKNSKT SQKTMDDLNL 0360
0361 STSEALRIDP VPNTHPLLVF VNPKSGGKQG QRVLWKFQYI LNPRQVFNLL KDGPEIGLRL 0420
0421 FKDVPDSRIL VCGGDGTVGW ILETIDKANL PVLPPVAVLP LGTGNDLARC LRWGGGYEGQ 0480
0481 NLAKILKDLE MSKVVHMDRW SVEVIPQQTE EKSDPVPFQI INNYFSIGVD ASIAHRFHIM 0540
0541 REKYPEKFNS RMKNKLWYFE FATSESIFST CKKLEESLTV EICGKPLDLS NLSLEGIAVL 0600
0601 NIPSMHGGSN LWGDTRRPHG DIYGINQALG ATAKVITDPD ILKTCVPDLS DKRLEVVGLE 0660
0661 GAIEMGQIYT KLKNAGRRLA KCSEITFHTT KTLPMQIDGE PWMQTPCTIK ITHKNQMPML 0720
0721 MGPPPRSTNF FGFLSGSSET VRFQGHHHHH H 0751
(SEQ ID NO: 2)
Nucleotide sequence encoding hDGKζ-(M1-A928)-transcript variant-2 Ct-TVMV-His: 
1 ATGGAGCCGC GGGACGGTAG CCCCGAGGCC CGGAGCAGCG ACTCCGAGTC
51 GGCTTCCGCC TCGTCCAGCG GCTCCGAGCG CGACGCCGGT CCCGAGCCGG
101 ACAAGGCGCC GCGGCGACTC AACAAGCGGC GCTTCCCGGG GCTGCGGCTC
151 TTCGGGCACA GGAAAGCCAT CACGAAGTCG GGCCTCCAGC ACCTGGCCCC
201 CCCTCCGCCC ACCCCTGGGG CCCCGTGCAG CGAGTCAGAG CGGCAGATCC
251 GGAGTACAGT GGACTGGAGC GAGTCAGCGA CATATGGGGA GCACATCTGG
301 TTCGAGACCA ACGTGTCCGG GGACTTCTGC TACGTTGGGG AGCAGTACTG
351 TGTAGCCAGG ATGCTGCAGA AGTCAGTGTC TCGAAGAAAG TGCGCAGCCT
401 GCAAGATTGT GGTGCACACG CCCTGCATCG AGCAGCTGGA GAAGATAAAT
451 TTCCGCTGTA AGCCGTCCTT CCGTGAATCA GGCTCCAGGA ATGTCCGCGA
501 GCCAACCTTT GTACGGCACC ACTGGGTACA CAGACGACGC CAGGACGGCA
551 AGTGTCGGCA CTGTGGGAAG GGATTCCAGC AGAAGTTCAC CTTCCACAGC
601 AAGGAGATTG TGGCCATCAG CTGCTCGTGG TGCAAGCAGG CATACCACAG
651 CAAGGTGTCC TGCTTCATGC TGCAGCAGAT CGAGGAGCCG TGCTCGCTGG
 
701 GGGTCCACGC AGCCGTGGTC ATCCCGCCCA CCTGGATCCT CCGCGCCCGG
751 AGGCCCCAGA ATACTCTGAA AGCAAGCAAG AAGAAGAAGA GGGCATCCTT
801 CAAGAGGAAG TCCAGCAAGA AAGGGCCTGA GGAGGGCCGC TGGAGACCCT
851 TCATCATCAG GCCCACCCCC TCCCCGCTCA TGAAGCCCCT GCTGGTGTTT
901 GTGAACCCCA AGAGTGGGGG CAACCAGGGT GCAAAGATCA TCCAGTCTTT
951 CCTCTGGTAT CTCAATCCCC GACAAGTCTT CGACCTGAGC CAGGGAGGGC
1001 CCAAGGAGGC GCTGGAGATG TACCGCAAAG TGCACAACCT GCGGATCCTG
1051 GCGTGCGGGG GCGACGGCAC GGTGGGCTGG ATCCTCTCCA CCCTGGACCA
1101 GCTACGCCTG AAGCCGCCAC CCCCTGTTGC CATCCTGCCC CTGGGTACTG
1151 GCAACGACTT GGCCCGAACC CTCAACTGGG GTGGGGGCTA CACAGATGAG
1201 CCTGTGTCCA AGATCCTCTC CCACGTGGAG GAGGGGAACG TGGTACAGCT
1251 GGACCGCTGG GACCTCCACG CTGAGCCCAA CCCCGAGGCA GGGCCTGAGG
1301 ACCGAGATGA AGGCGCCACC GACCGGTTGC CCCTGGATGT CTTCAACAAC
1351 TACTTCAGCC TGGGCTTTGA CGCCCACGTC ACCCTGGAGT TCCACGAGTC
1401 TCGAGAGGCC AACCCAGAGA AATTCAACAG CCGCTTTCGG AATAAGATGT
1451 TCTACGCCGG GACAGCTTTC TCTGACTTCC TGATGGGCAG CTCCAAGGAC
1501 CTGGCCAAGC ACATCCGAGT GGTGTGTGAT GGAATGGACT TGACTCCCAA
1551 GATCCAGGAC CTGAAACCCC AGTGTGTTGT TTTCCTGAAC ATCCCCAGGT
1601 ACTGTGCGGG CACCATGCCC TGGGGCCACC CTGGGGAGCA CCACGACTTT
1651 GAGCCCCAGC GGCATGACGA CGGCTACCTC GAGGTCATTG GCTTCACCAT
1701 GACGTCGTTG GCCGCGCTGC AGGTGGGCGG ACACGGCGAG CGGCTGACGC
1751 AGTGTCGCGA GGTGGTGCTC ACCACATCCA AGGCCATCCC GGTGCAGGTG
1801 GATGGCGAGC CCTGCAAGCT TGCAGCCTCA CGCATCCGCA TCGCCCTGCG
1851 CAACCAGGCC ACCATGGTGC AGAAGGCCAA GCGGCGGAGC GCCGCCCCCC
1901 TGCACAGCGA CCAGCAGCCG GTGCCAGAGC AGTTGCGCAT CCAGGTGAGT
1951 CGCGTCAGCA TGCACGACTA TGAGGCCCTG CACTACGACA AGGAGCAGCT
2001 CAAGGAGGCC TCTGTGCCGC TGGGCACTGT GGTGGTCCCA GGAGACAGTG
2051 ACCTAGAGCT CTGCCGTGCC CACATTGAGA GACTCCAGCA GGAGCCCGAT
2101 GGTGCTGGAG CCAAGTCCCC GACATGCCAG AAACTGTCCC CCAAGTGGTG
2151 CTTCCTGGAC GCCACCACTG CCAGCCGCTT CTACAGGATC GACCGAGCCC
2201 AGGAGCACCT CAACTATGTG ACTGAGATCG CACAGGATGA GATTTATATC
2251 CTGGACCCTG AGCTGCTGGG GGCATCGGCC CGGCCTGACC TCCCAACCCC
2301 CACTTCCCCT CTCCCCACCT CACCCTGCTC ACCCACGCCC CGGTCACTGC
2351 AAGGGGATGC TGCACCCCCT CAAGGTGAAG AGCTGATTGA GGCTGCCAAG
2401 AGGAACGACT TCTGTAAGCT CCAGGAGCTG CACCGAGCTG GGGGCGACCT
2451 CATGCACCGA GACGAGCAGA GTCGCACGCT CCTGCACCAC GCAGTCAGCA
2501 CTGGCAGCAA GGATGTGGTC CGCTACCTGC TGGACCACGC CCCCCCAGAG
2551 ATCCTTGATG CGGTGGAGGA AAACGGGGAG ACCTGTTTGC ACCAAGCAGC
2601 GGCCCTGGGC CAGCGCACCA TCTGCCACTA CATCGTGGAG GCCGGGGCCT
2651 CGCTCATGAA GACAGACCAG CAGGGCGACA CTCCCCGGCA GCGGGCTGAG
2701 AAGGCTCAGG ACACCGAGCT GGCCGCCTAC CTGGAGAACC GGCAGCACTA
2751 CCAGATGATC CAGCGGGAGG ACCAGGAGAC GGCTGTGGGA TCCTCGGAGA
2801 CAGTGCGGTT TCAGGGACAC CACCACCATC ACCACTGA
(SEQ ID NO: 3)
Amino acid sequence of hDGKζ-(M1-A928)-transcript variant-2 Ct-TVMV-His: 
0001 MEPRDGSPEA RSSDSESASA SSSGSERDAG PEPDKAPRRL NKRRFPGLRL FGHRKAITKS 0060
0061 GLQHLAPPPP TPGAPCSESE RQIRSTVDWS ESATYGEHIW FETNVSGDFC YVGEQYCVAR 0120
0121 mLQKSVSRRK CAACKIVVHT PCIEQLEKIN FRCKPSFRES GSRNVREPTF VRHHWVHRRR 0180
0181 QDGKCRHCGK GFQQKFTFHS KEIVAISCSW CKQAYHSKVS CFMLQQIEEP CSLGVHAAVV 0240
0241 IPPTWILRAR RPQNTLKASK KKKRASFKRK SSKKGPEEGR WRPFIIRPTP SPLMKPLLVF 0300
0301 VNPKSGGNQG AKIIQSFLWY LNPRQVFDLS QGGPKEALEM YRKVHNLRIL ACGGDGTVGW 0360
0361 ILSTLDQLRL KPPPPVAILP LGTGNDLART LNWGGGYTDE PVSKILSHVE EGNVVQLDRW 0420
 
0421 DLHAEPNPEA GPEDRDEGAT DRLPLDVFNN YFSLGFDAHV TLEFHESREA NPEKFNSRFR 0480
0481 NKMFYAGTAF SDFLMGSSKD LAKHIRVVCD GMDLTPKIQD LKPQCVVFLN IPRYCAGTMP 0540
0541 WGHPGEHHDF EPQRHDDGYL EVIGFTMTSL AALQVGGHGE RLTQCREVVL TTSKAIPVQV 0600
0601 DGEPCKLAAS RIRIALRNQA TMVQKAKRRS AAPLHSDQQP VPEQLRIQVS RVSMHDYEAL 0660
0661 HYDKEQLKEA SVPLGTVVVP GDSDLELCRA HIERLQQEPD GAGAKSPTCQ KLSPKWCFLD 0720
0721 ATTASRFYRI DRAQEHLNYV TEIAQDEIYI LDPELLGASA RPDLPTPTSP LPTSPCSPTP 0780
0781 RSLQGDAAPP QGEELIEAAK RNDFCKLQEL HRAGGDLMHR DEQSRTLLHH AVSTGSKDVV 0840
0841 RYLLDHAPPE ILDAVEENGE TCLHQAAALG QRTICHYIVE AGASLMKTDQ QGDTPRQRAE 0900
0901 KAQDTELAAY LENRQHYQMI QREDQETAVG SSETVRFQGH HHHHH 0945
(SEQ ID NO: 4)
Nucleotide sequence encoding MA-hDGKα-(S9-S727)-Ct-TVMV-His: 
0001 ATGGCTTCCC CAAGCGACTT CGCCCAGCTG CAGAAGTACA TGGAATACAG CACCAAGAAG 0060
0061 GTGTCTGACG TCCTGAAGCT GTTCGAGGAC GGTGAAATGG CTAAGTACGT CCAGGGCGAC 0120
0121 GCTATCGGAT ACGAGGGATT CCAGCAGTTC CTGAAGATCT ACCTGGAAGT GGACAACGTC 0180
0181 CCCAGGCACC TGTCACTGGC TCTGTTCCAG TCCTTCGAGA CTGGCCACTG CCTGAACGAA 0240
0241 ACCAACGTCA CTAAGGACGT GGTCTGCCTG AACGACGTGA GCTGCTACTT CTCTCTGCTG 0300
0301 GAGGGTGGCA GACCAGAGGA CAAGCTGGAA TTCACCTTCA AGCTGTACGA CACTGACCGC 0360
0361 AACGGAATCC TGGACTCCAG CGAAGTGGAC AAGATCATCC TGCAGATGAT GCGTGTCGCT 0420
0421 GAGTACCTGG ACTGGGACGT GAGCGAACTG AGGCCTATCC TGCAGGAGAT GATGAAGGAA 0480
0481 ATCGACTACG ACGGCTCTGG ATCAGTGTCC CAGGCTGAGT GGGTCCGCGC TGGTGCTACC 0540
0541 ACTGTGCCAC TGCTGGTCCT GCTGGGACTG GAAATGACCC TGAAGGACGA CGGTCAGCAC 0600
0601 ATGTGGCGCC CAAAGCGTTT CCCCAGGCCA GTCTACTGCA ACCTGTGCGA GTCTTCAATC 0660
0661 GGTCTGGGCA AGCAGGGCCT GTCATGCAAC CTGTGCAAGT ACACCGTGCA CGACCAGTGC 0720
0721 GCTATGAAGG CCCTGCCCTG CGAGGTCTCA ACTTACGCTA AGTCCCGTAA GGACATCGGA 0780
0781 GTGCAGTCAC ACGTGTGGGT CAGGGGAGGT TGCGAATCCG GTAGATGCGA CCGCTGCCAG 0840
0841 AAGAAGATCC GTATCTACCA CTCCCTGACC GGACTGCACT GCGTCTGGTG CCACCTGGAG 0900
0901 ATCCACGACG ACTGCCTGCA GGCCGTGGGA CACGAATGCG ACTGCGGTCT GCTGCGTGAC 0960
0961 CACATCCTGC CTCCCTCCAG CATCTACCCT TCAGTCCTGG CTTCCGGTCC CGACAGGAAG 1020
1021 AACAGCAAGA CCTCTCAGAA GACTATGGAC GACCTGAACC TGAGCACCTC TGAGGCCCTG 1080
1081 CGCATCGACC CTGTGCCCAA CACTCACCCA CTGCTGGTGT TCGTCAACCC TAAGAGCGGC 1140
1141 GGAAAGCAGG GTCAGAGAGT CCTGTGGAAG TTCCAGTACA TCCTGAACCC ACGCCAGGTG 1200
1201 TTCAACCTGC TGAAGGACGG CCCTGAGATC GGACTGAGAC TGTTCAAGGA CGTGCCCGAC 1260
1261 TCTCGCATCC TCGTCTGCGG TGGCGACGGT ACTGTGGGAT GGATCCTGGA AACTATCGAC 1320
1321 AAGGCTAACC TGCCAGTGCT GCCACCTGTG GCTGTCCTGC CACTGGGAAC CGGTAACGAC 1380
1381 CTGGCTCGTT GCCTGCGTTG GGGAGGTGGC TACGAGGGAC AGAACCTGGC CAAGATCCTG 1440
1441 AAGGACCTGG AAATGAGCAA GGTGGTCCAC ATGGACAGAT GGTCTGTGGA GGTCATCCCA 1500
1501 CAGCAGACTG AGGAAAAGTC AGACCCAGTC CCTTTCCAGA TCATCAACAA CTACTTCAGC 1560
1561 ATCGGTGTGG ACGCTTCTAT CGCCCACAGA TTCCACATCA TGCGCGAGAA GTACCCTGAA 1620
1621 AAGTTCAACT CCCGCATGAA GAACAAGCTG TGGTACTTCG AGTTCGCTAC CTCAGAATCC 1680
1681 ATCTTCTCAA CTTGCAAGAA GCTGGAGGAA TCCCTGACCG TCGAGATCTG CGGCAAGCCT 1740
1741 CTGGACCTGT CAAACCTGTC CCTGGAAGGC ATCGCTGTGC TGAACATCCC AAGCATGCAC 1800
1801 GGAGGTTCTA ACCTCTGGGG CGACACTAGG AGGCCTCACG GTGACATCTA CGGCATCAAC 1860
1861 CAGGCCCTGG GAGCTACCGC CAAGGTCATC ACTGACCCCG ACATCCTGAA GACCTGCGTG 1920
 
1921 CCAGACCTGA GCGACAAGCG TCTGGAGGTG GTCGGACTGG AGGGTGCCAT CGAAATGGGC 1980
1981 CAGATCTACA CTAAGCTGAA GAACGCTGGA AGGAGACTGG CCAAGTGCTC TGAGATCACC 2040
2041 TTCCACACCA CTAAGACTCT GCCTATGCAG ATCGACGGTG AACCCTGGAT GCAGACCCCA 2100
2101 TGCACTATCA AGATCACCCA CAAGAACCAG ATGCCCATGC TGATGGGTCC TCCTCCTCGC 2160
2161 TCTGGATCTT CAGAAACTGT GAGGTTCCAG GGCCACCACC ACCACCACCA CTGA 2214
(SEQ ID NO: 5)
Amino acid sequence of MA-hDGKα-(S9-S727)-Ct-TVMV-His:

0001 MASPSDFAQL QKYMEYSTKK VSDVLKLFED GEMAKYVQGD AIGYEGFQQF LKIYLEVDNV 0060
0061 PRHLSLALFQ SFETGHCLNE TNVTKDVVCL NDVSCYFSLL EGGRPEDKLE FTFKLYDTDR 0120
0121 NGILDSSEVD KIILQMMRVA EYLDWDVSEL RPILQEMMKE IDYDGSGSVS QAEWVRAGAT 0180
0181 TVPLLVLLGL EMTLKDDGQH MWRPKRFPRP VYCNLCESSI GLGKQGLSCN LCKYTVHDQC 0240
0241 AMKALPCEVS TYAKSRKDIG VQSHVWVRGG CESGRCDRCQ KKIRIYHSLT GLHCVWCHLE 0300
0301 IHDDCLQAVG HECDCGLLRD HILPPSSIYP SVLASGPDRK NSKTSQKTMD DLNLSTSEAL 0360
0361 RIDPVPNTHP LLVFVNPKSG GKQGQRVLWK FQYILNPRQV FNLLKDGPEI GLRLFKDVPD 0420
0421 SRILVCGGDG TVGWILETID KANLPVLPPV AVLPLGTGND LARCLRWGGG YEGQNLAKIL 0480
0481 KDLEMSKVVH MDRWSVEVIP QQTEEKSDPV PFQIINNYFS IGVDASIAHR FHIMREKYPE 0540
0541 KFNSRMKNKL WYFEFATSES IFSTCKKLEE SLTVEICGKP LDLSNLSLEG IAVLNIPSMH 0600
0601 GGSNLWGDTR RPHGDIYGIN QALGATAKVI TDPDILKTCV PDLSDKRLEV VGLEGAIEMG 0660
0661 QIYTKLKNAG RRLAKCSEIT FHTTKTLPMQ IDGEPWMQTP CTIKITHKNQ MPMLMGPPPR 0720
0721 SGSSETVRFQ GHHHHHH 0737
(SEQ ID NO: 6)

## Claims

1. A compound of Formula (I): 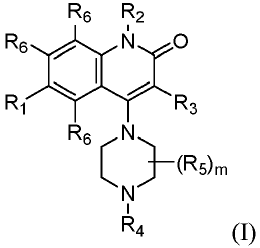 or a salt thereof, wherein:
R₁ is F, Cl, Br, -CN, C₁₋₃ alkyl substituted with zero to 4 R₁ₐ, C₃₋₄ cycloalkyl substituted with zero to 4 R₁ₐ, C₁₋₃ alkoxy substituted with zero to 4 R₁ₐ, -C(O)NRₐRₐ, -NRₐRₐ, -S(O)ₙRₑ, or -P(O)RₑRₑ;
each R₁ₐ is independently F, Cl, -CN, -OH, -OCH₃, or -NRₐRₐ;
each Rₐ is independently H or C₁₋₃ alkyl;
each Rₑ is independently C₃₋₄ cycloalkyl or C₁₋₃ alkyl substituted with zero to 4 R₁ₐ;
R₂ is H, C₁₋₃ alkyl substituted with zero to 4 R₂ₐ, C₂₋₃ alkenyl substituted with zero to 4 R₂ₐ, or C₃₋₄ cycloalkyl substituted with zero to 4 R₂ₐ;
each R₂ₐ is independently F, Cl, -CN, -OH, -O(C₁₋₂ alkyl), C₃₋₄ cycloalkyl, C₃₋₄ alkenyl, or C₃₋₄ alkynyl;
R₃ is H, F, Cl, Br, -CN, C₁₋₃ alkyl, C₁₋₂ fluoroalkyl, C₃₋₄ cycloalkyl, C₃₋₄ fluorocycloalkyl, -NO₂, or pyridinyl substituted with zero to 2 R₃ₐ;
each R₃ₐ is halo, -CN, C₁₋₃ alkyl, or C₁₋₃ alkoxy;
R₄ is -CH₂R₄ₐ, -CH₂CH₂R₄ₐ, -CH₂CHR₄ₐR_{4d}, -CHR₄ₐR_{4b}, or -CR₄ₐR_{4b}R_{4c};
R₄ₐ and R_{4b} are independently:
(i) C₁₋₆ alkyl substituted with zero to 4 substituents independently selected from F, Cl, -CN, -OH, -OCH₃, -SCH₃, C₁₋₃ fluoroalkoxy, -NRₐRₐ, -S(O)₂Rₑ, or -NRₐS(O)₂Rₑ;
(ii) C₃₋₆ cycloalkyl, heterocyclyl, phenyl, or heteroaryl, each substituted with zero to 4 substituents independently selected from F, Cl, Br, -CN, -OH, C₁₋₆ alkyl, C₁₋₃ fluoroalkyl, C₁₋₄ hydroxyalkyl, -(CH₂)₁₋₂O(C₁₋₃ alkyl), C₁₋₄ alkoxy, -O(C₁₋₄ hydroxyalkyl), -O(CH)₁₋₃O(C₁₋₃ alkyl), C₁₋₃ fluoroalkoxy, -O(CH)₁₋₃NR_{c}R_{c}, -OCH₂CH=CH₂, -OCH₂C≡CH, -C(O)(C₁₋₄ alkyl), -C(O)OH, -C(O)O(C₁₋₄ alkyl), -NR_{c}R_{c}, -NRₐS(O)₂(C₁₋₃ alkyl), -NRₐC(O)(C₁₋₃ alkyl), -NRₐC(O)O(C₁₋₄ alkyl), -P(O)(C₁₋₃ alkyl)₂, -S(O)₂(C₁₋₃ alkyl), -O(CH₂)₁₋₂(C₃₋₆ cycloalkyl), -O(CH₂)₁₋₂(morpholinyl), cyclopropyl, cyanocyclopropyl, methylazetidinyl, acetylazetidinyl, (tert-butoxycarbonyl)azetidinyl, triazolyl, tetrahydropyranyl, morpholinyl, thiophenyl, methylpiperidinyl, and R_{d}; or
(iii) C₁₋₄ alkyl substituted with one cyclic group selected from C₃₋₆ cycloalkyl, heterocyclyl, aryl, and heteroaryl, said cyclic group substituted with zero to 3 substituents independently selected from F, Cl, Br, -OH, -CN, C₁₋₆ alkyl, C₁₋₃ fluoroalkyl, C₁₋₃ alkoxy, C₁₋₃ fluoroalkoxy, -OCH₂CH=CH₂, OCH₂C≡CH, -NR_{c}R_{c}, -NRₐS(O)₂(C₁₋₃ alkyl), -NRₐC(O)(C₁₋₃ alkyl), -NRₐC(O)O(C₁₋₄ alkyl), and C₃₋₆ cycloalkyl;
or R₄ₐ and R_{4b} together with the carbon atom to which they are attached form a C₃₋₆ cycloalkyl or a 3- to 6-membered heterocyclyl, each substituted with zero to 3 R_{f};
each R_{f} is independently F, Cl, Br, -OH, -CN, C₁₋₆ alkyl, C₁₋₃ fluoroalkyl, C₁₋₃ alkoxy, C₁₋₃ fluoroalkoxy, -OCH₂CH=CH₂, -OCH₂C≡CH, -NR_{c}R_{c}, or a cyclic group selected from C₃₋₆ cycloalkyl, 3- to 6-membered heterocyclyl, phenyl, monocyclic heteroaryl, and bicyclic heteroaryl, each cyclic group substituted with zero to 3 substituents independently selected from F, Cl, Br, -OH, -CN, C₁₋₆ alkyl, C₁₋₃ fluoroalkyl, C₁₋₃ alkoxy, C₁₋₃ fluoroalkoxy, and -NR_{c}R_{c};
R_{4c} is C₁₋₆ alkyl or C₃₋₆ cycloalkyl, each substituted with zero to 4 substituents independently selected from F, Cl, -OH, C₁₋₂ alkoxy, C₁₋₂ fluoroalkoxy, and -CN;
R_{4d} is -OCH₃;
each R_{c} is independently H or C₁₋₂ alkyl;
R_{d} is phenyl substituted with zero to 1 substituent selected from F, Cl, -CN, -CH₃, and -OCH₃;
each R₅ is independently F, Cl, -CN, -OH, C₁₋₆ alkyl substituted with zero to 4 R_{g}, C₁₋₃ alkoxy substituted with zero to 4 R_{g}, C₂₋₄ alkenyl substituted with zero to 4 R_{g}, C₂₋₄ alkynyl substituted with zero to 4 R_{g}, C₃₋₄ cycloalkyl substituted with zero to 4 R_{g}, phenyl substituted with zero to 4 R_{g}, oxadiazolyl substituted with zero to 3 R_{g}, pyridinyl substituted with zero to 4 R_{g}, -(CH₂)₁₋₂(heterocyclyl substituted with zero to 4 R_{g}), -(CH₂)₁₋₂NR_{c}C(O)(C₁₋₄ alkyl), -(CH₂)₁₋₂NR_{c}C(O)O(C₁₋₄ alkyl), -(CH₂)₁₋₂NR_{c}S(O)₂(C₁₋₄ alkyl), -C(O)(C₁₋₄ alkyl), -C(O)OH, -C(O)O(C₁₋₄ alkyl), -C(O)O(C₃₋₄ cycloalkyl), -C(O)NRₐRₐ, or -C(O)NRₐ(C₃₋₄ cycloalkyl), or two R₅ attached to the same carbon atom form =O;
each R_{g} is independently F, Cl, -CN, -OH, C₁₋₃ alkoxy, C₁₋₃ fluoroalkoxy, -O(CH₂)₁₋₂O(C₁₋₂ alkyl), C₃₋₅ cycloalkyl, or -NR_{c}R_{c};
each R₆ is H, F, Cl, -CN, -CH₃, -CH₂F, -CHF₂, -CF₃, or -OCH₃;
m is zero, 1, 2, or 3; and
n is zero, 1, or 2.

2. The compound according to Claim 1 or a salt thereof, wherein:
R₁ is F, Cl, Br, -CN, C₁₋₃ alkyl substituted with zero to 4 R₁ₐ, cyclopropyl substituted with zero to 3 R₁ₐ, C₁₋₃ alkoxy substituted with zero to 3 R₁ₐ, -C(O)NRₐRₐ, -NRₐRₐ, -S(O)ₙCH₃, or -P(O)(CH₃)₂;
each R₁ₐ is independently F, Cl, or -CN;
each Rₐ is independently H or C₁₋₃ alkyl;
R₂ is H, C₁₋₂ alkyl substituted with zero to 2 R₂ₐ, or C₂₋₃ alkenyl substituted with zero to 2 R₂a;
each R₂ₐ is independently F, Cl, -CN, -OH, -O(C₁₋₂ alkyl), cyclopropyl, C₃₋₄ alkenyl, or C₃₋₄ alkynyl;
R₃ is H, F, Cl, Br, -CN, C₁₋₂ alkyl, C₁₋₂ fluoroalkyl, C₃₋₄ cycloalkyl, -NO₂, or pyridinyl substituted with zero to 2 R₃ₐ;
R₄ₐ and R_{4b} are independently:
(i) C₁₋₄ alkyl substituted with zero to 4 substituents independently selected from F, Cl, -CN, -OH, -OCH₃, -SCH₃, C₁₋₃ fluoroalkoxy, and -NRₐRₐ;
(ii) C₃₋₆ cycloalkyl, heterocyclyl, phenyl, or heteroaryl, each substituted with zero to 4 substituents independently selected from F, Cl, Br, -CN, -OH, C₁₋₆ alkyl, C₁₋₃ fluoroalkyl, -CH₂OH, -(CH₂)₁₋₂O(C₁₋₂ alkyl), C₁₋₄ alkoxy, -O(C₁₋₄ hydroxyalkyl), -O(CH)₁₋₂O(C₁₋₂ alkyl), C₁₋₃ fluoroalkoxy, -O(CH)₁₋₂NR_{c}R_{c}, -OCH₂CH=CH₂, -OCH2C-CH, -C(O)(C₁₋₄ alkyl), -C(O)OH, -C(O)O(C₁₋₄ alkyl), -NR_{c}R_{c}, -NRₐS(O)₂(C₁₋₃ alkyl), -NRₐC(O)(C₁₋₃ alkyl), -NRₐC(O)O(C₁₋₄ alkyl), -P(O)(C₁₋₂ alkyl)₂, -S(O)₂(C₁₋₃ alkyl), -O(CH₂)₁₋₂(C₃₋₄ cycloalkyl), -O(CH₂)₁₋₂(morpholinyl), cyclopropyl, cyanocyclopropyl, methylazetidinyl, acetylazetidinyl, (*tert-*butoxycarbonyl)azetidinyl, triazolyl, tetrahydropyranyl, morpholinyl, thiophenyl, methylpiperidinyl, and R_{d}; or
(iii) C₁₋₃ alkyl substituted with one cyclic group selected from C₃₋₆ cycloalkyl, heterocyclyl, phenyl, and heteroaryl, said cyclic group substituted with zero to 3 substituents independently selected from F, Cl, Br, -OH, -CN, C₁₋₃ alkyl, C₁₋₂ fluoroalkyl, C₁₋₃ alkoxy, C₁₋₂ fluoroalkoxy, -OCH₂CH=CH₂, OCH₂C≡CH, -NR_{c}R_{c}, -NRₐS(O)₂(C₁₋₃ alkyl), -NRₐC(O)(C₁₋₃ alkyl), -NRₐC(O)O(C₁₋₄ alkyl), and C₃₋₄ cycloalkyl;
or R₄ₐ and R_{4b} together with the carbon atom to which they are attached, form a C₃₋₆ cycloalkyl or a 3- to 6-membered heterocyclyl, each substituted with zero to 3 R_{f};
each R_{f} is independently F, Cl, Br, -OH, -CN, C₁₋₄ alkyl, C₁₋₂ fluoroalkyl, C₁₋₃ alkoxy, C₁₋₂ fluoroalkoxy, -OCH₂CH=CH₂, OCH₂C≡CH, -NR_{c}R_{c}, or a cyclic group selected from C₃₋₆ cycloalkyl, 3- to 6-membered heterocyclyl, phenyl, monocyclic heteroaryl, and bicyclic heteroaryl, each cyclic group substituted with zero to 3 substituents independently selected from F, Cl, Br, -OH, -CN, C₁₋₄ alkyl, C₁₋₂ fluoroalkyl, C₁₋₃ alkoxy, C₁₋₂ fluoroalkoxy, and -NR_{c}R_{c};
R_{4c} is C₁₋₄ alkyl or C₃₋₆ cycloalkyl, each substituted with zero to 4 substituents independently selected from F, Cl, -OH, C₁₋₂ alkoxy, C₁₋₂ fluoroalkoxy, and -CN;
each R₅ is independently F, -CN, -OH, C₁₋₅ alkyl substituted with zero to 4 R_{g}, C₁₋₂ alkoxy substituted with zero to 3 R_{g}, C₂₋₃ alkenyl substituted with zero to 4 R_{g}, C₂₋₃ alkynyl substituted with zero to 4 R_{g}, C₃₋₄ cycloalkyl substituted with zero to 4 R_{g}, phenyl substituted with zero to 3 R_{g}, oxadiazolyl substituted with zero to 3 R_{g}, pyridinyl substituted with zero to 3 R_{g}, -(CH₂)₁₋₂(heterocyclyl substituted with zero to 4 R_{g}), -(CH₂)₁₋₂NR_{c}C(O)(C₁₋₄ alkyl), -(CH₂)₁₋₂NR_{c}C(O)O(C₁₋₄ alkyl), -(CH₂)₁₋₂NR_{c}S(O)₂(C₁₋₄ alkyl), -C(O)(C₁₋₄ alkyl), -C(O)OH, -C(O)O(C₁₋₄ alkyl), -C(O)O(C₃₋₄ cycloalkyl), -C(O)NRₐRₐ, or -C(O)NRₐ(C₃₋₄ cycloalkyl), or two R₅ attached to the same carbon atom form =O;
each R₆ is H, F, or -CH₃; and
m is zero, 1, 2, or 3.

3. The compound according to claim 1 or a salt thereof, wherein:
R₁ is F, -CN, or -OCH₃;
R₂ is H, -CH₃, -CH₂CN, -CH₂CH₂F, or -CH₂CH=CH₂;
R₃ is H, -CN, -CH₂OH, -C(O)OCH₂CH₃, -NO₂, or pyridinyl;
R₄ is -CH₂R₄ₐ or -CHR₄ₐR_{4b};
R₄ₐ is phenyl, naphthalenyl, or indolyl, each substituted with zero to 2 substituents independently selected from F, -CH₃, -CH₂CH₃, and -OCH₃;
R_{4b} is phenyl or fluorophenyl;
each R₅ is -CH₃, or two R₅ attached to the same carbon atom form =O; and
m is zero, 1, or 2.

4. The compound according to claim 1 or a salt thereof, having the structure: 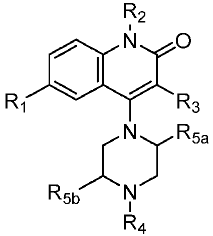 wherein:
R₅ₐ and R_{5b} are independently selected from R₅.

5. The compound according to claim 1 or a salt thereof, having the structure: 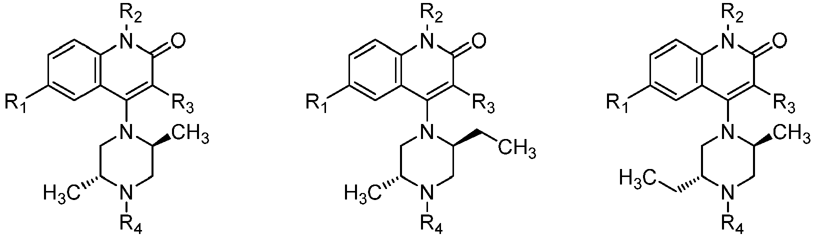 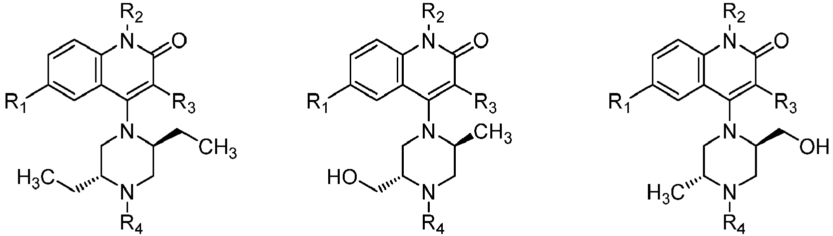 or

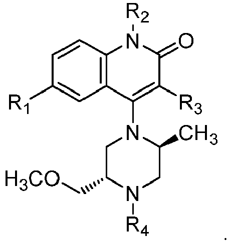

6. The compound according to claim 1 or a salt thereof, wherein R₄ is -CH₂R₄ₐ or -CH₂CH₂R₄ₐ.

7. The compound according to claim 1 or a salt thereof, wherein R₄ is -CH₂R₄ₐ.

8. The compound according to claim 1 or a salt thereof, wherein R₄ is -CHR₄ₐR_{4b} or -CR₄ₐR_{4b}R_{4c}.

9. The compound according to claim 1 or a salt thereof, wherein R₄ is -CHR₄ₐR_{4b}.

10. The compound according to claim 1 or a salt thereof, wherein R₄ is -CH₂R₄ₐ or -CHR₄ₐR_{4b}.

11. The compound according to claim 1 or a salt thereof, wherein said compound is:
6-fluoro-4-{4-[(3-fluorophenyl)methyl]piperazin-1-yl}-1-methyl-3-nitro-1,2-dihydroquinolin-2-one (1);
6-fluoro-4-{4-[(3-fluoro-5-methylphenyl)methyl]piperazin-1-yl}-1-methyl-3-(pyridin-4-yl)-1,2-dihydroquinolin-2-one (2);
4-(4-((1H-indol-4-yl)methyl)piperazin-1-yl)-6-fluoro-1-methyl-3-nitroquinolin-2(1H)-one (3);
4-{4-[(1-ethyl-1H-indol-4-yl)methyl]piperazin-1-yl}-6-fluoro-1-methyl-3-nitro-1,2-dihydroquinolin-2-one (4);
4-(4-benzyl-3-oxopiperazin-1-yl)-6-fluoro-1-methyl-3-nitro-1,2-dihydroquinolin-2-one (5);
4-[(2 S, SR)-4-[bis(4-fluorophenyl)methyl]-2, 5-dimethylpiperazin-1-yl]-1-methyl-2-oxo-1,2-dihydroquinoline-6-carbonitrile (7); or
6-methoxy-1-methyl-4-{4-[(naphthalen-1-yl)methyl]piperazin-1-yl}-3-nitro-1,2-dihydroquinolin-2-one (8).

12. A pharmaceutical composition comprising a compound according to any of claims 1 to 11 or a pharmaceutically-acceptable salt thereof; and a pharmaceutically acceptable carrier.

13. A compound according to any of claims 1 to 11, or a pharmaceutically-acceptable salt thereof, for use in therapy.

14. A compound according to any one of claims 1-11 or a pharmaceutically-acceptable salt thereof, for use in the treatment of cancer or viral infections.

15. The compound or pharmaceutically-acceptable salt thereof for use according to claim 14, wherein said cancer is selected from cancer of the colon, pancreatic cancer, breast cancer, prostate cancer, lung cancer, ovarian cancer, cervical cancer, renal cancer, cancer of the head and neck, lymphoma, leukemia and melanoma.

## Patentansprüche

1. Verbindung der Formel (I): 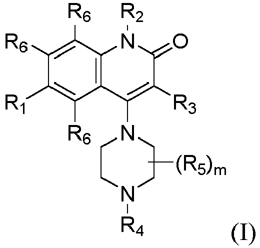 oder ein Salz davon, wobei:
R₁ F, Cl, Br, -CN, C₁₋₃-Alkyl, substituiert mit null bis 4 R₁ₐ, C₃₋₄-Cycloalkyl, substituiert mit null bis 4 R₁ₐ, C₁₋₃-Alkoxy, substituiert mit null bis 4 R₁ₐ, -C(O)NRₐRₐ, -NRₐRₐ, -S(O)ₙRₑ oder -P(O)RₑRₑ ist;
jedes R₁ₐ unabhängig F, Cl, -CN, -OH, -OCH₃ oder -NRₐRₐ ist;
jedes Rₐ unabhängig H oder C₁₋₃-Alkyl ist;
jedes Rₑ unabhängig C₃₋₄-Cycloalkyl oder C₁₋₃-Alkyl, substituiert mit null bis 4 R₁ₐ, ist;
R₂ H, C₁₋₃-Alkyl, substituiert mit null bis 4 R₂ₐ, C₂₋₃-Alkenyl, substituiert mit null bis 4 R₂ₐ, oder C₃₋₄-Cycloalkyl, substituiert mit null bis 4 R₂ₐ, ist;
jedes R₂ₐ unabhängig F, Cl, -CN, -OH, -O(C₁₋₂-Alkyl), C₃₋₄-Cycloalkyl, C₃₋₄-Alkenyl oder C₃₋₄-Alkynyl ist;
R₃ H, F, Cl, Br, -CN, C₁₋₃-Alkyl, C₁₋₂-Fluoralkyl, C₃₋₄-Cycloalkyl, C₃₋₄-Fluorcycloalkyl, -NO₂ oder Pyridinyl, substituiert mit null bis 2 R₃ₐ, ist;
jedes R₃ₐ Halo, -CN, C₁₋₃-Alkyl, oder C₁₋₃-Alkoxy ist;
R₄ -CH₂R₄ₐ, -CH₂CH₂R₄ₐ, -CH₂CHR₄ₐR_{4d}, -CHR₄ₐR_{4b} oder -CR₄ₐR_{4b}R_{4c} ist;
R₄ₐ und R_{4b} unabhängig Folgendes sind:
(i) C₁₋₆-Alkyl, substituiert mit null bis 4 Substituenten, die unabhängig ausgewählt sind aus F, Cl, -CN, -OH, -OCH₃, -SCH₃, C₁₋₃-Fluoralkoxy, -NRₐRₐ, -S(O)₂Rₑ oder -NRₐS(O)₂Rₑ;
(ii) C₃₋₆-Cycloalkyl, Heterocyclyl, Phenyl oder Heteroaryl, jedes substituiert mit null bis 4 Substituenten, die unabhängig ausgewählt sind aus F, Cl, Br, -CN, -OH, C₁₋₆-Alkyl, C₁₋₃-Fluoralkyl, C₁₋₄-Hydroxyalkyl, -(CH₂)₁₋₂O(C₁₋₃-Alkyl), C₁₋₄-Alkoxy, -O(C₁₋₄-Hydroxyalkyl), -O(CH)₁₋₃O(C₁₋₃-Alkyl), C₁₋₃-Fluoralkoxy, -O(CH)₁₋₃NR_{c}R_{c}, -OCH₂CH=CH₂, -OCH₂C=CH, -C(O)(C₁₋₄-Alkyl), -C(O)OH, -C(O)O(C₁₋₄-Alkyl), -NR_{c}R_{c}, -NRₐS(O)₂(C₁₋₃-Alkyl), -NRₐC(O)(C₁₋₃-Alkyl), -NRₐC(O)O(C₁₋₄-Alkyl), -P(O)(C₁₋₃-Alkyl)₂, -S(O)₂(C₁₋₃-Alkyl), -O(CH₂)₁₋₂(C₃₋₆-Cycloalkyl), -O(CH₂)₁₋₂(Morpholinyl), Cyclopropyl, Cyancyclopropyl, Methylazetidinyl, Acetylazetidinyl, (*tert-*Butoxycarbonyl)azetidinyl, Triazolyl, Tetrahydropyranyl, Morpholinyl, Thiophenyl, Methylpiperidinyl und R_{d}; oder
(iii) C₁₋₄-Alkyl, substituiert mit einer zyklischen Gruppe, die ausgewählt ist aus C₃₋₆-Cycloalkyl, Heterocyclyl, Aryl und Heteroaryl, wobei die zyklische Gruppe mit null bis 3 Substituenten substituiert ist, die unabhängig ausgewählt sind aus F, Cl, Br, -OH, -CN, C₁₋₆-Alkyl, C₁₋₃-Fluoralkyl, C₁₋₃-Alkoxy, C₁₋₃-Fluoralkoxy, -OCH₂CH=CH₂, -OCH₂C≡CH, -NR_{c}R_{c}, -NRₐS(O)₂(C₁₋₃-Alkyl), -NRₐC(O)(C₁₋₃-Alkyl), -NRₐC(O)O(C₁₋₄-Alkyl) und C₃₋₆-Cycloalkyl;
oder R₄ₐ und R_{4b} zusammen mit dem Kohlenstoffatom, an das sie angehängt sind, ein C₃₋₆-Cycloalkyl oder ein 3- bis 6-gliedriges Heterocyclyl, jedes substituiert mit null bis 3 R_{f}, bilden,
jedes R_{f} unabhängig F, Cl, Br, -OH, -CN, C₁₋₆-Alkyl, C₁₋₃-Fluoralkyl, C₁₋₃-Alkoxy, C₁₋₃-Fluoralkoxy, -OCH₂CH=CH₂, -OCH₂C=CH, -NR_{c}R_{c} oder eine zyklische Gruppe ist, die ausgewählt ist aus C₃₋₆-Cycloalkyl, 3- bis 6-gliedrigem Heterocyclyl, Phenyl, monozyklischem Heteroaryl und bizyklischem Heteroaryl, wobei jede zyklische Gruppe mit null bis 3 Substituenten substituiert ist, die unabhängig ausgewählt sind aus F, Cl, Br, -OH, -CN, C₁₋₆-Alkyl, C₁₋₃-Fluoralkyl, C₁₋₃-Alkoxy, C₁₋₃-Fluoralkoxy und -NR_{c}R_{c};
R_{4c} C₁₋₆-Alkyl oder C₃₋₆-Cycloalkyl, jedes substituiert mit null bis 4 Substituenten, die unabhängig ausgewählt sind aus F, Cl, -OH, C₁₋₂-Alkoxy, C₁₋₂-Fluoralkoxy und -CN, ist;
R₄ₐ -OCH₃ ist;
jedes Rₑ unabhängig H oder C₁₋₂-Alkyl ist;
R_{d} Phenyl, substituiert mit null bis 1 Substituenten, der ausgewählt ist aus F, Cl, -CN, -CH₃ und -OCH₃, ist;
jedes R₅ unabhängig F, Cl, -CN, -OH, C₁₋₆-Alkyl, substituiert mit null bis 4 R_{g}, C₁₋₃-Alkoxy, substituiert mit null bis 4 R_{g}, C₂₋₄-Alkenyl, substituiert mit null bis 4 R_{g}, C₂₋₄-Alkynyl, substituiert mit null bis 4 R_{g}, C₃₋₄-Cycloalkyl, substituiert mit null bis 4 R_{g}, Phenyl, substituiert mit null bis 4 R_{g}, Oxadiazolyl, substituiert mit null bis 3 R_{g}, Pyridinyl, substituiert mit null bis 4 R_{g}, -(CH₂)₁₋₂(Heterocyclyl, substituiert mit null bis 4 R_{g}), -(CH₂)₁₋₂NR_{c}C(O)(C₁₋₄-Alkyl), -(CH₂)₁₋₂NR_{c}C(O)O(C₁₋₄-Alkyl), -(CH₂)₁₋₂NR_{c}S(O)₂(C₁₋₄-Alkyl), -C(O)(C₁₋₄-Alkyl), -C(O)OH, -C(O)O(C₁₋₄-Alkyl), -C(O)O(C₃₋₄-Cycloalkyl), -C(O)NRₐRₐ oder -C(O)NRₐ(C₃₋₄-Cycloalkyl) ist oder zwei R₅, angehängt an das gleiche Kohlenstoffatom, =O bilden;
jedes R_{g} unabhängig F, Cl, -CN, -OH, C₁₋₃-Alkoxy, C₁₋₃-Fluoralkoxy, -O(CH₂)₁₋₂O(C₁₋₂-Alkyl), C₃₋₅-Cycloalkyl, oder -NR_{c}R_{c} ist;
jedes R₆ H, F, Cl, -CN, -CH₃, -CH₂F, -CHF₂, -CF₃ oder -OCH₃ ist;
m null, 1, 2 oder 3 ist und
n null, 1 oder 2 ist.

2. Verbindung nach Anspruch 1 oder ein Salz davon, wobei:
R₁ F, Cl, Br, -CN, C₁₋₃-Alkyl, substituiert mit null bis 4 R₁ₐ, Cyclopropyl, substituiert mit null bis 3 R₁ₐ, C₁₋₃-Alkoxy, substituiert mit null bis 3 R₁ₐ, -C(O)NRₐRₐ, -NRₐRₐ, -S(O)ₙCH₃ oder -P(O)(CH₃)₂ ist;
jedes R₁ₐ unabhängig F, Cl oder -CN ist;
jedes Rₐ unabhängig H oder C₁₋₃-Alkyl ist;
R₂ H, C₁₋₂-Alkyl, substituiert mit null bis 2 R₂ₐ, oder C₂₋₃-Alkenyl, substituiert mit null bis 2 R₂ₐ, ist;
jedes R₂ₐ unabhängig F, Cl, -CN, -OH, -O(C₁₋₂-Alkyl), Cyclopropyl, C₃₋₄-Alkenyl oder C₃₋₄-Alkynyl ist;
R₃ H, F, Cl, Br, -CN, C₁₋₂-Alkyl, C₁₋₂-Fluoralkyl, C₃₋₄-Cycloalkyl, -NO₂ oder Pyridinyl, substituiert mit null bis 2 R₃a, ist;
R₄ₐ und R_{4b} unabhängig Folgendes sind:
(i) C₁₋₄-Alkyl, substituiert mit null bis 4 Substituenten, die unabhängig ausgewählt sind aus F, Cl, -CN, -OH, -OCH₃, -SCH₃, C₁₋₃-Fluoralkoxy und -NRₐRₐ;
(ii) C₃₋₆-Cycloalkyl, Heterocyclyl, Phenyl oder Heteroaryl, jedes substituiert mit null bis 4 Substituenten, die unabhängig ausgewählt sind aus F, Cl, Br, -CN, -OH, C₁₋₆-Alkyl, C₁₋₃-Fluoralkyl, -CH₂OH, -(CH₂)₁₋₂O(C₁₋₂-Alkyl), C₁₋₄-Alkoxy, -O(C₁₋₄-Hydroxyalkyl), -O(CH)₁₋₂O(C₁₋₂-Alkyl), C₁₋₃-Fluoralkoxy, -O(CH)₁₋₂NR_{c}R_{c}, -OCH₂CH=CH₂, -OCH₂C=CH, -C(O)(C₁₋₄-Alkyl), -C(O)OH, -C(O)O(C₁₋₄-Alkyl), -NR_{c}R_{c}, -NRₐS(O)₂(C₁₋₃-Alkyl), -NRₐC(O)(C₁₋₃-Alkyl), -NRₐC(O)O(C₁₋₄-Alkyl), - P(O)(C₁₋₂-Alkyl)₂, -S(O)₂(C₁₋₃-Alkyl), -O(CH₂)₁₋₂(C₃₋₄-Cycloalkyl), -O(CH₂)₁₋₂(Morpholinyl), Cyclopropyl, Cyancyclopropyl, Methylazetidinyl, Acetylazetidinyl, (*tert-*Butoxycarbonyl)azetidinyl, Triazolyl, Tetrahydropyranyl, Morpholinyl, Thiophenyl, Methylpiperidinyl und R_{d}, oder
(iii) C₁₋₃-Alkyl, substituiert mit einer zyklischen Gruppe, die ausgewählt ist aus C₃₋₆-Cycloalkyl, Heterocyclyl, Phenyl und Heteroaryl, wobei die zyklische Gruppe mit null bis 3 Substituenten substituiert ist, die unabhängig ausgewählt sind aus F, Cl, Br, -OH, -CN, C₁₋₃-Alkyl, C₁₋₂-Fluoralkyl, C₁₋₃-Alkoxy, C₁₋₂-Fluoralkoxy, -OCH₂CH=CH₂, -OCH₂C≡CH, -NR_{c}R_{c}, -NRₐS(O)₂(C₁₋₃-Alkyl), -NRₐC(O)(C₁₋₃-Alkyl), -NRaC(O)O(C₁₋₄-Alkyl) und C₃₋₄-Cycloalkyl;
oder R₄ₐ und R_{4b} zusammen mit dem Kohlenstoffatom, an das sie angehängt sind, ein C₃₋₆-Cycloalkyl oder ein 3- to 6-gliedriges Heterocyclyl, jedes substituiert mit null bis 3 R_{f}, bilden,
jedes R_{f} unabhängig F, Cl, Br, -OH, -CN, C₁₋₄-Alkyl, C₁₋₂-Fluoralkyl, C₁₋₃-Alkoxy, C₁₋₂-Fluoralkoxy, -OCH₂CH=CH₂, -OCH₂C≡CH, -NR_{c}R_{c} oder eine zyklische Gruppe ist, die ausgewählt ist aus C₃₋₆-Cycloalkyl, 3- bis 6-gliedrigem Heterocyclyl, Phenyl, monozyklischem Heteroaryl und bizyklischem Heteroaryl, ist, wobei jede zyklische Gruppe mit null bis 3 Substituenten substituiert ist, die unabhängig ausgewählt sind aus F, Cl, Br, -OH, -CN, C₁₋₄-Alkyl, C₁₋₂-Fluoralkyl, C₁₋₃-Alkoxy, C₁₋₂-Fluoralkoxy und -NR_{c}R_{c};
R_{4c} C₁₋₄-Alkyl oder C₃₋₆-Cycloalkyl, jedes substituiert mit null bis 4 Substituenten, unabhängig ausgewählt aus F, Cl, -OH, C₁₋₂-Alkoxy, C₁₋₂-Fluoralkoxy und -CN, ist;
jedes R₅ unabhängig F, -CN, -OH, C₁₋₅-Alkyl, substituiert mit null bis 4 R_{g}, C₁₋₂-Alkoxy, substituiert mit null bis 3 R_{g}, C₂₋₃-Alkenyl, substituiert mit null bis 4 R_{g}, C₂₋₃-Alkynyl, substituiert mit null bis 4 R_{g}, C₃₋₄-Cycloalkyl, substituiert mit null bis 4 R_{g}, Phenyl, substituiert mit null bis 3 R_{g}, Oxadiazolyl, substituiert mit null bis 3 R_{g}, Pyridinyl, substituiert mit null bis 3 R_{g}, -(CH₂)₁₋₂(Heterocyclyl, substituiert mit null bis 4 R_{g}), -(CH₂)₁₋₂NRcC(O)(C₁₋₄-Alkyl), -(CH₂)₁₋₂NR_{c}C(O)O(C₁₋₄-Alkyl), -(CH₂)₁₋₂NR_{c}S(O)₂(C₁₋₄-Alkyl), -C(O)(C₁₋₄-Alkyl), -C(O)OH, -C(O)O(C₁₋₄-Alkyl), -C(O)O(C₃₋₄-Cycloalkyl), -C(O)NRₐRₐ oder -C(O)NRₐ(C₃₋₄-Cycloalkyl) oder zwei R₅, angehängt an das gleiche Kohlenstoffatom, =O bilden;
jedes R₆ H, F oder -CH₃ ist und
m null, 1, 2 oder 3 ist.

3. Verbindung nach Anspruch 1 oder Salz davon, wobei:
R₁ F, -CN oder -OCH₃ ist;
R₂ H, -CH₃, -CH₂CN, -CH₂CH₂F oder -CH₂CH=CH₂ ist;
R₃ H, -CN, -CH₂OH, -C(O)OCH₂CH₃, -NO₂ oder Pyridinyl ist;
R₄ -CH₂R₄ₐ oder -CHR₄ₐR_{4b} ist;
R₄ₐ Phenyl, Naphthalenyl oder Indolyl, jedes substituiert mit null bis 2 Substituenten, die unabhängig ausgewählt sind aus F, -CH₃, -CH₂CH₃ und -OCH₃, ist;
R_{4b} Phenyl oder Fluorphenyl ist;
jedes R₅ -CH₃ oder two R₅, angehängt an das gleiche Kohlenstoffatom, =O bilden; und
m null, 1 oder 2 ist.

4. Verbindung nach Anspruch 1 oder Salz davon, mit der Struktur: 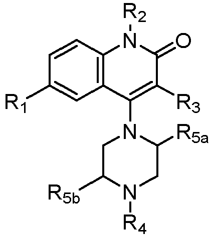 wobei:
R₅ₐ und R_{5b} unabhängig aus R₅ ausgewählt sind.

5. Verbindung nach Anspruch 1 oder Salz davon, mit folgender Struktur: 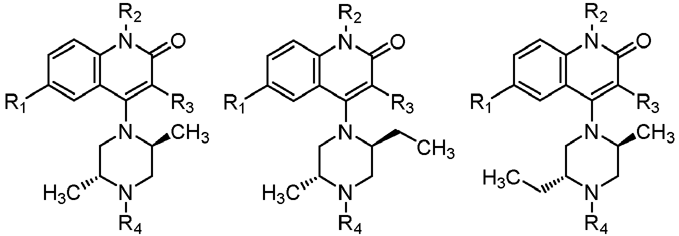 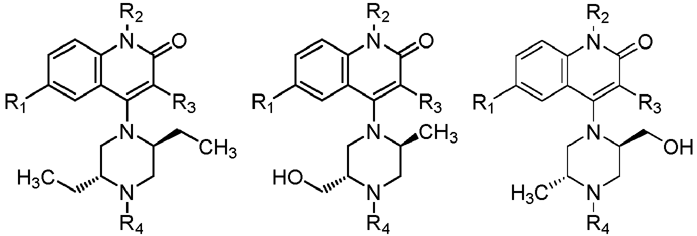 oder

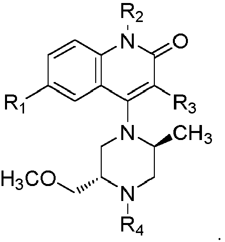

6. Verbindung nach Anspruch 1 oder Salz davon, wobei R₄ -CH₂R₄ₐ oder -CH₂CH₂R₄ₐ ist.

7. Verbindung nach Anspruch 1 oder Salz davon, wobei R₄ -CH₂R₄ₐ ist.

8. Verbindung nach Anspruch 1 oder Salz davon, wobei R₄ -CHR₄ₐR_{4b} oder -CR₄ₐR4bR_{4c} ist.

9. Verbindung nach Anspruch 1 oder Salz davon, wobei R₄ -CHR₄ₐR_{4b} ist.

10. Verbindung nach Anspruch 1 oder Salz davon, wobei R₄ -CH₂R₄ₐ oder -CHR₄ₐR_{4b} ist.

11. Verbindung nach Anspruch 1 oder Salz davon, wobei die Verbindung Folgendes ist:
6-Fluor-4- { 4- [(3 -fluorphenyl)methyl]piperazin-1 -yl} -1 -methyl-3 -nitro-1,2-dihydrochinolin-2-on (1);
6-Fluor-4- { 4- [(3 -fluor-5 -methylphenyl)methyl]piperazin-1 -yl} -1 -methyl-3 - (pyridin-4-yl)-1,2-dihydrochinolin-2-on (2);
4-(4-((1H-Indol-4-yl)methyl)piperazin-1-yl)-6-fluor-1-methyl-3 -nitrochinolin-2(1H)-on (3);
4-{4-[(1-Ethyl-1H-indol-4-yl)methyl]piperazin-1-yl}-6-fluor-1-methyl-3-nitro-1,2-dihydrochinolin-2-on (4);
4-(4-Benzyl-3 -oxopiperazin-1-yl)-6-fluor-1-methyl-3 -nitro-1,2-dihydrochinolin-2-on (5);
4-[(2S,5R)-4-[bis(4-Fluorphenyl)methyl]-2,5-dimethylpiperazin-1-yl]-1-methyl-2-oxo-1,2-dihydrochinolin-6-carbonitril (7) oder
6-Methoxy-1 -methyl-4- { 4- [(naphthalen-1 -yl)methyl]piperazin-1 -yl} -3 -nitro-1,2-dihydrochinolin-2-on (8).

12. Pharmazeutische Zusammensetzung, die eine Verbindung nach einem der Ansprüche 1 bis 11 oder ein pharmazeutisch unbedenkliches Salz davon und einen pharmazeutisch unbedenklichen Träger umfasst.

13. Verbindung nach einem der Ansprüche 1 bis 11 oder pharmazeutisch unbedenkliches Salz davon, zur Verwendung in einer Therapie.

14. Verbindung nach einem der Ansprüche 1 bis 11 oder pharmazeutisch unbedenkliches Salz davon, zur Verwendung bei der Behandlung von Krebs oder Virusinfektionen.

15. Verbindung oder pharmazeutisch unbedenkliches Salz davon zur Verwendung nach Anspruch 14, wobei der Krebs aus Darmkrebs, Bauchspeicheldrüsenkrebs, Brustkrebs, Prostatakrebs, Lungenkrebs, Eierstockkrebs, Gebärmutterhalskrebs, Nierenkrebs, Krebs an Kopf und Hals, Lymphom, Leukämie und Melanom ausgewählt ist.

## Revendications

1. Composé de formule (I) : 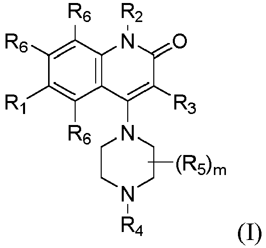 ou sel de celui-ci, dans laquelle :
R₁ est F, Cl, Br, -CN, un alkyle en C₁₋₃ substitué par zéro à 4 R₁ₐ, un cycloalkyle en C₃₋₄ substitué par zéro à 4 R₁ₐ, un alcoxy en C₁₋₃ substitué par zéro à 4 R₁ₐ, -C(O)NRₐRₐ, -NRₐRₐ, -S(O)ₙRₑ ou -P(O)RₑRₑ ;
chaque R₁ₐ est indépendamment F, Cl, -CN, -OH, -OCH₃ ou -NRₐRₐ ;
chaque Rₐ est indépendamment H ou un alkyle en C₁₋₃ ;
chaque Rₑ est indépendamment un cycloalkyle en C₃₋₄ ou un alkyle en C₁₋₃ substitué par zéro à 4 R₁ₐ ;
R₂ est H, un alkyle en C₁₋₃ substitué par zéro à 4 R₂ₐ, un alcényle en C₂₋₃ substitué par zéro à 4 R₂ₐ ou un cycloalkyle en C₃₋₄ substitué par zéro à 4 R₂ₐ ;
chaque R₂ₐ est indépendamment F, Cl, -CN, -OH, -O(alkyle en C₁₋₂), un cycloalkyle en C₃₋₄, un alcényle en C₃₋₄ ou un alkynyle en C₃₋₄ ;
R₃ est H, F, Cl, Br, -CN, un alkyle en C₁₋₃, un fluoroalkyle en C₁₋₂, un cycloalkyle en C₃₋₄, un fluorocycloalkyle en C₃₋₄, -NO₂ ou un pyridinyle substitué par zéro à 2 R₃ₐ ;
chaque R₃ₐ est un halo, -CN, un alkyle en C₁₋₃ ou un alcoxy en C₁₋₃ ;
R₄ est -CH₂R₄ₐ, -CH₂CH₂R₄ₐ, -CH₂CHR₄ₐR_{4d}, -CHR₄ₐR_{4b} ou -CR₄ₐR_{4b}R_{4c} ;
R₄ₐ et R_{4b} sont indépendamment :
(i) un alkyle en C₁₋₆ substitué par zéro à 4 substituants indépendamment choisis parmi F, Cl, -CN, -OH, -OCH₃, -SCH₃, un fluoroalcoxy en C₁₋₃, -NRₐRₐ, -S(O)₂Rₑ ou -NRₐS(O)2Rₑ ;
(ii) un cycloalkyle en C₃₋₆, un hétérocyclyle, un phényle ou un hétéroaryle, chacun étant substitué par zéro à 4 substituants indépendamment choisis parmi F, Cl, Br, -CN, -OH, un alkyle en C₁₋₆, un fluoroalkyle en C₁₋₃, un hydroxyalkyle en C₁₋₄, -(CH₂)₁₋₂O(alkyle en C₁₋₃), un alcoxy en C₁₋₄, -O(hydroxyalkyle en C₁₋₄), -O(CH)₁₋₃O(alkyle en C₁₋₃), un fluoroalcoxy en C₁₋₃, -O(CH)₁₋₃NR_{c}R_{c}, -OCH₂CH=CH₂, -OCH₂C≡CH, -C(O)(alkyle en C₁₋₄), -C(O)OH, -C(O)O(alkyle en C₁₋₄), -NR_{c}R_{c}, -NRₐS(O)₂(alkyle en C₁₋₃), -NRₐC(O)(alkyle en C₁₋₃), -NRₐC(O)O(alkyle en C₁₋₄), -P(O)(alkyle en C₁₋₃)₂, -S(O)₂(alkyle en C₁₋₃), -O(CH₂)₁₋₂(cycloalkyle en C₃₋₆), -O(CH₂)₁₋₂(morpholinyle), un cyclopropyle, un cyanocyclopropyle, un méthylazétidinyle, un acétylazétidinyle, un (*tert-*butoxycarbonyl)azétidinyle, un triazolyle, un tétrahydropyranyle, un morpholinyle, un thiophényle, un méthylpipéridinyle et R_{d} ; ou
(iii) un alkyle en C₁₋₄ substitué par un groupe cyclique choisi parmi un cycloalkyle en C₃₋₆, un hétérocyclyle, un aryle et un hétéroaryle, ledit groupe cyclique étant substitué par zéro à 3 substituants indépendamment choisis parmi F, Cl, Br, -OH, -CN, un alkyle en C₁₋₆, un fluoroalkyle en C₁₋₃, un alcoxy en C₁₋₃, un fluoroalcoxy en C₁₋₃, -OCH₂CH=CH₂, -OCH₂C≡CH, -NR_{c}R_{c}, -NRₐS(O)₂(alkyle en C₁₋₃), -NRₐC(O)(alkyle en C₁₋₃), -NRₐC(O)O(alkyle en C₁₋₄) et un cycloalkyle en C₃₋₆ ;
ou R₄ₐ et R_{4b}, ensemble avec l'atome de carbone auquel ils sont liés, forment un cycloalkyle en C₃₋₆ ou un hétérocyclyle à 3 à 6 chaînons, chacun étant substitué par zéro à 3 R_{f};
chaque Rf est indépendamment F, Cl, Br, -OH, -CN, un alkyle en C₁₋₆, un fluoroalkyle en C₁₋₃, un alcoxy en C₁₋₃, un fluoroalcoxy en C₁₋₃, -OCH₂CH=CH₂, -OCH₂C≡CH, -NR_{c}R_{c} ou un groupe cyclique choisi parmi un cycloalkyle en C₃₋₆, un hétérocyclyle à 3 à 6 chaînons, un phényle, un hétéroaryle monocyclique et un hétéroaryle bicyclique, chaque groupe cyclique étant substitué par zéro à 3 substituants indépendamment choisis parmi F, Cl, Br, -OH, -CN, un alkyle en C₁₋₆, un fluoroalkyle en C₁₋₃, un alcoxy en C₁₋₃, un fluoroalcoxy en C₁₋₃ et -NR_{c}R_{c} ;
R_{4c} est un alkyle en C₁₋₆ ou un cycloalkyle en C₃₋₆, chacun étant substitué par zéro à 4 substituants indépendamment choisis parmi F, Cl, -OH, un alcoxy en C₁₋₂, un fluoroalcoxy en C₁₋₂ et -CN ;
R_{4d} est -OCH₃ ;
chaque R_{c} est indépendamment H ou un alkyle en C₁₋₂ ;
R_{d} est un phényle substitué par zéro à 1 substituant choisi parmi F, Cl, -CN, -CH₃ et -OCH₃ ;
chaque R₅ est indépendamment F, Cl, -CN, -OH, un alkyle en C₁₋₆ substitué par zéro à 4 R_{g}, un alcoxy en C₁₋₃ substitué par zéro à 4 R_{g}, un alcényle en C₂₋₄ substitué par zéro à 4 R_{g}, un alkynyle en C₂₋₄ substitué par zéro à 4 R_{g}, un cycloalkyle en C₃₋₄ substitué par zéro à 4 R_{g}, un phényle substitué par zéro à 4 R_{g}, un oxadiazolyle substitué par zéro à 3 R_{g}, un pyridinyle substitué par zéro à 4 R_{g}, -(CH₂)₁₋₂(hétérocyclyle substitué par zéro à 4 R_{g}), -(CH₂)₁₋₂NR_{c}C(O)(alkyle en C₁₋₄), -(CH₂)₁₋₂NR_{c}C(O)O(alkyle en C₁₋₄), -(CH₂)₁₋₂NR_{c}S(O)₂(alkyle en C₁₋₄), -C(O)(alkyle en C₁₋₄), -C(O)OH, -C(O)O(alkyle en C₁₋₄), -C(O)O(cycloalkyle en C₃₋₄), -C(O)NRₐRₐ ou -C(O)NRₐ(cycloalkyle en C₃₋₄), ou deux R₅, liés au même atome de carbone, forment =O ;
chaque R_{g} est indépendamment F, Cl, -CN, -OH, un alcoxy en C₁₋₃, un fluoroalcoxy en C₁₋₃, -O(CH₂)₁₋₂O(alkyle en C₁₋₂), un cycloalkyle en C₃₋₅ ou -NR_{c}R_{c} ;
chaque R₆ est H, F, Cl, -CN, -CH₃, -CH₂F, -CHF₂, -CF₃ ou -OCH₃ ;
m est zéro, 1, 2 ou 3 ; et
n est zéro, 1 ou 2.

2. Composé selon la revendication 1 ou sel de celui-ci, dans lequel :
R₁ est F, Cl, Br, -CN, un alkyle en C₁₋₃ substitué par zéro à 4 R₁ₐ, un cyclopropyle substitué par zéro à 3 R₁ₐ, un alcoxy en C₁₋₃ substitué par zéro à 3 R₁ₐ, -C(O)NRₐRₐ, -NRₐRₐ, -S(O)ₙCH₃ ou -P(O)(CH₃)₂ ;
chaque R₁ₐ est indépendamment F, Cl ou -CN ;
chaque Rₐ est indépendamment H ou un alkyle en C₁₋₃ ;
R₂ est H, un alkyle en C₁₋₂ substitué par zéro à 2 R₂ₐ ou un alcényle en C₂₋₃ substitué par zéro à 2 R₂ₐ ;
chaque R₂ₐ est indépendamment F, Cl, -CN, -OH, -O(alkyle en C₁₋₂), un cyclopropyle, un alcényle en C₃₋₄ ou un alkynyle en C₃₋₄ ;
R₃ est H, F, Cl, Br, -CN, un alkyle en C₁₋₂, un fluoroalkyle en C₁₋₂, un cycloalkyle en C₃₋₄, -NO₂ ou un pyridinyle substitué par zéro à 2 R₃ₐ ;
R₄ₐ et R_{4b} sont indépendamment :
(i) un alkyle en C₁₋₄ substitué par zéro à 4 substituants indépendamment choisis parmi F, Cl, -CN, -OH, -OCH₃, -SCH₃, un fluoroalcoxy en C₁₋₃ et -NRₐRₐ ;
(ii) un cycloalkyle en C₃₋₆, un hétérocyclyle, un phényle ou un hétéroaryle, chacun étant substitué par zéro à 4 substituants indépendamment choisis parmi F, Cl, Br, -CN, -OH, un alkyle en C₁₋₆, un fluoroalkyle en C₁₋₃, -CH₂OH, -(CH₂)₁₋₂O(alkyle en C₁₋₂), un alcoxy en C₁₋₄, -O(hydroxyalkyle en C₁₋₄), -O(CH)₁₋₂O(alkyle en C₁₋₂), un fluoroalcoxy en C₁₋₃, -O(CH)₁₋₂NR_{c}R_{c}, -OCH₂CH=CH₂, -OCH₂C≡CH, -C(O)(alkyle en C₁₋₄), -C(O)OH, -C(O)O(alkyle en C₁₋₄), -NR_{c}R_{c}, -NRₐS(O)₂(alkyle en C₁₋₃), -NRₐC(O)(alkyle en C₁₋₃), -NRₐC(O)O(alkyle en C₁₋₄), -P(O)(alkyle en C₁₋₂)₂, -S(O)₂(alkyle en C₁₋₃), -O(CH₂)₁₋₂(cycloalkyle en C₃₋₄), -O(CH₂)₁₋₂(morpholinyle), un cyclopropyle, un cyanocyclopropyle, un méthylazétidinyle, un acétylazétidinyle, un (*tert-*butoxycarbonyl)azétidinyle, un triazolyle, un tétrahydropyranyle, un morpholinyle, un thiophényle, un méthylpipéridinyle et R_{d} ; ou
(iii) un alkyle en C₁₋₃ substitué par un groupe cyclique choisi parmi un cycloalkyle en C₃₋₆, un hétérocyclyle, un phényle et un hétéroaryle, ledit groupe cyclique étant substitué par zéro à 3 substituants indépendamment choisis parmi F, Cl, Br, -OH, -CN, un alkyle en C₁₋₃, un fluoroalkyle en C₁₋₂, un alcoxy en C₁₋₃, un fluoroalcoxy en C₁₋₂, -OCH₂CH=CH₂, -OCH₂C≡CH, -NR_{c}R_{c}, -NRₐS(O)₂(alkyle en C₁₋₃), -NRₐC(O)(alkyle en C₁₋₃), -NRₐC(O)O(alkyle en C₁₋₄) et un cycloalkyle en C₃₋₄ ;
ou R₄ₐ et R_{4b}, ensemble avec l'atome de carbone auquel ils sont liés, forment un cycloalkyle en C₃₋₆ ou un hétérocyclyle à 3 à 6 chaînons, chacun étant substitué par zéro à 3 R_{f};
chaque Rf est indépendamment F, Cl, Br, -OH, -CN, un alkyle en C₁₋₄, un fluoroalkyle en C₁₋₂, un alcoxy en C₁₋₃, un fluoroalcoxy en C₁₋₂, -OCH₂CH=CH₂, -OCH₂C≡CH, -NR_{c}R_{c} ou un groupe cyclique choisi parmi un cycloalkyle en C₃₋₆, un hétérocyclyle à 3 à 6 chaînons, un phényle, un hétéroaryle monocyclique et un hétéroaryle bicyclique, chaque groupe cyclique étant substitué par zéro à 3 substituants indépendamment choisis parmi F, Cl, Br, -OH, -CN, un alkyle en C₁₋₄, un fluoroalkyle en C₁₋₂, un alcoxy en C₁₋₃, un fluoroalcoxy en C₁₋₂ et -NR_{c}R_{c} ;
R_{4c} est un alkyle en C₁₋₄, un cycloalkyle en C₃₋₆, chacun substitué par zéro à 4 substituants indépendamment choisis parmi F, Cl, -OH, un alcoxy en C₁₋₂, un fluoroalcoxy en C₁₋₂ et -CN ;
chaque R₅ est indépendamment F, -CN, -OH, un alkyle en C₁₋₅ substitué par zéro à 4 R_{g}, un alcoxy en C₁₋₂ substitué par zéro à 3 R_{g}, un alcényle en C₂₋₃ substitué par zéro à 4 R_{g}, un alkynyle en C₂₋₃ substitué par zéro à 4 R_{g}, un cycloalkyle en C₃₋₄ substitué par zéro à 4 R_{g}, un phényle substitué par zéro à 3 R_{g}, un oxadiazolyle substitué par zéro à 3 R_{g}, un pyridinyle substitué par zéro à 3 R_{g}, -(CH₂)₁₋₂(hétérocyclyle substitué par zéro à 4 R_{g}), -(CH₂)₁₋₂NR_{c}C(O)(alkyle en C₁₋₄), -(CH₂)₁₋₂NR_{c}C(O)O(alkyle en C₁₋₄), -(CH₂)₁₋₂NR_{c}S(O)₂(alkyle en C₁₋₄), -C(O)(alkyle en C₁₋₄), -C(O)OH, -C(O)O(alkyle en C₁₋₄), -C(O)O(cycloalkyle en C₃₋₄), -C(O)NRₐRₐ ou -C(O)NRₐ(cycloalkyle en C₃₋₄), ou deux R₅, liés au même atome de carbone, forment =O ;
chaque R₆ est H, F ou-CH₃ ; et
m est zéro, 1, 2 ou 3.

3. Composé selon la revendication 1 ou sel de celui-ci, dans lequel :
R₁ est F, -CN ou -OCH₃ ;
R₂ est H, -CH₃, -CH₂CN, -CH₂CH₂F ou -CH₂CH=CH₂ ;
R₃ est H, -CN, -CH₂OH, -C(O)OCH₂CH₃, -NO₂ ou un pyridinyle ;
R₄ est -CH₂R₄ₐ ou -CHR₄ₐR_{4b} ;
R₄ₐ est un phényle, un naphtalényle ou un indolyle, chacun étant substitué par zéro à 2 substituants indépendamment choisis parmi F, -CH₃, -CH₂CH₃ et -OCH₃ ;
R_{4b} est un phényle ou un fluorophényle ;
chaque R₅ est -CH₃, ou deux R₅, liés au même atome de carbone, forment =O ; et
m est zéro, 1 ou 2.

4. Composé selon la revendication 1 ou sel de celui-ci, ayant la structure : 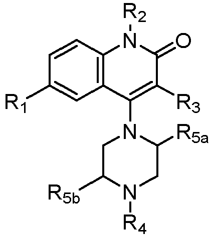 dans laquelle :
R₅ₐ et R_{5b} sont indépendamment choisis parmi R₅.

5. Composé selon la revendication 1 ou sel de celui-ci, ayant la structure : 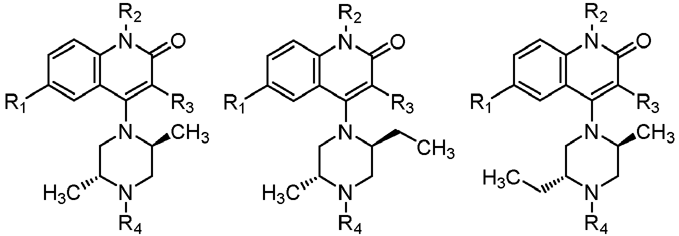 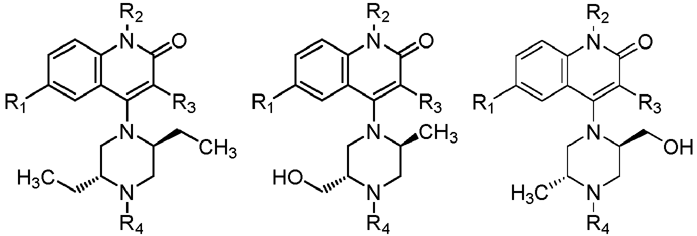 ou

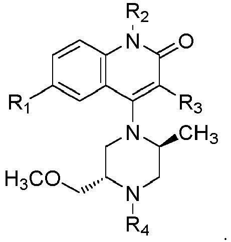

6. Composé selon la revendication 1 ou sel de celui-ci, dans lequel R₄ est -CH₂R₄ₐ ou -CH₂CH₂R₄ₐ.

7. Composé selon la revendication 1 ou sel de celui-ci, dans lequel R₄ est -CH₂R₄ₐ.

8. Composé selon la revendication 1 ou sel de celui-ci, dans lequel R₄ est -CHR₄ₐR_{4b} ou -CR₄ₐR_{4b}R_{4c}.

9. Composé selon la revendication 1 ou sel de celui-ci, dans lequel R₄ est -CHR₄ₐR_{4b}.

10. Composé selon la revendication 1 ou sel de celui-ci, dans lequel R₄ est -CH₂R₄ₐ ou -CHR₄ₐR_{4b}.

11. Composé selon la revendication 1 ou sel de celui-ci, dans lequel ledit composé est :
6-fluoro-4-{4-[(3-fluorophényl)méthyl]pipérazin-1-yl}-1-méthyl-3-nitro-1,2-dihydroquinoléin-2-one (1) ;
6-fluoro-4-{4-[(3-fluoro-5-méthylphényl)méthyl]pipérazin-l-yl}-1-méthyl-3-(pyridin-4-yl)-1,2-dihydroquinoléin-2-one (2) ;
4-(4-((1H-indol-4-yl)méthyl)pipérazin-1-yl)-6-fluoro-1-méthyl-3-nitroquinoléin-2(1H)-one (3) ;
4-{4-[(1-éthyl-1H-indol-4-yl)méthyl]pipérazin-1-yl}-6-fluoro-1-méthyl-3-nitro-1,2-dihydroquinoléin-2-one (4) ;
4-(4-benzyl-3-oxopipérazin-1-yl)-6-fluoro-1-méthyl-3-nitro-1,2-dihydroquinoléin-2-one (5) ;
4-[(2S,5R)-4-[bis(4-fluorophényl)méthyl]-2,5-diméthylpipérazin-1-yl]-1-méthyl-2-oxo-1,2-dihydroquinoléine-6-carbonitrile (7) ; ou
6-méthoxy-1-méthyl-4-{4-[(naphtalén-1-yl)méthyl]pipérazin-1-yl}-3-nitro-1,2-dihydroquinoléin-2-one (8).

12. Composition pharmaceutique comprenant un composé selon l'une quelconque des revendications 1 à 11 ou un sel pharmaceutiquement acceptable de celui-ci, et un véhicule pharmaceutiquement acceptable.

13. Composé selon l'une quelconque des revendications 1 à 11, ou sel pharmaceutiquement acceptable de celui-ci, pour utilisation en thérapie.

14. Composé selon l'une quelconque des revendications 1 à 11, ou sel pharmaceutiquement acceptable de celui-ci, pour utilisation dans le traitement d'un cancer ou d'infections virales.

15. Composé ou sel pharmaceutiquement acceptable de celui-ci pour utilisation selon la revendication 14, où ledit cancer est choisi parmi un cancer du côlon, un cancer du pancréas, un cancer du sein, un cancer de la prostate, un cancer du poumon, un cancer de l'ovaire, un cancer du col de l'utérus, un cancer du rein, un cancer de la tête et du cou, un lymphome, une leucémie et un mélanome.
